# EUROPEAN PATENT APPLICATION

(11) **EP 4 083 062 A1**
(43) Date of publication of application: **02.11.2022**
(21) Application number: 22166188.7
(22) Date of filing: 31.10.2014
(51) Int. Cl.: C07K 14/705, A61P 35/00, A61K 35/28, C12N 15/86, A61K 35/17, C07K 14/73, A61K 39/00, C07K 14/725

(54) **MODIFIED HEMATOPOIETIC STEM/PROGENITOR AND NON-T EFFECTOR CELLS, AND USES THEREOF**

(30) Priority: 31.10.2013 US 201361898387 P
(62) Divisional of application: 14859129.0
(71) Applicant: Fred Hutchinson Cancer Center, Seattle, WA 98109 (US); Seattle Children's Hospital dba Seattle Children's Research Institute, Seattle, WA 98101 (US)
(72) Inventor: DELANEY, Colleen, Seattle Washington 98112, (US); JENSEN, Michael, Bainbridge Island Washington 98110, (US); GARDNER, Rebecca, Shoreline Washington 98117, (US)
(74) Representative: Forresters IP LLP

(57) **Abstract**

Hematopoeitic stem/progenitor cells (HSPC) and/or non-T effector cells are genetically modified to express (i) an extracellular component including a ligand binding domain that binds a cellular marker preferentially expressed on an unwanted cell; and (ii) an intracellular component comprising an effector domain. Among other uses, the modified cells can be administered to patients to target unwanted cancer cells without the need for immunological matching before administration.

## Description

### CROSS REFERENCE TO RELATED APPLICATIONS

This application claims priority to U.S. Provisional Patent Application No. 61/898,387 filed on October 31, 2013, the entire contents of which are incorporated by reference herein.

### FIELD OF THE DISCLOSURE

Hematopoeitic stem/progenitor cells (HSPC) and/or non-T effector cells are genetically modified to express (i) an extracellular component including a ligand binding domain that binds a cellular marker preferentially expressed on an unwanted cell; and (ii) an intracellular component comprising an effector domain. Among other uses, the modified cells can be administered to patients to target unwanted cancer cells without the need for immunological matching before administration.

### BACKGROUND OF THE DISCLOSURE

Significant progress has been made in genetically engineering T cells of the immune system to target and kill unwanted cell types, such as cancer cells. For example, T cells have been genetically engineered to express molecules having extracellular components that bind particular target antigens and intracellular components that direct actions of the T cell when the extracellular component has bound the target antigen. As an example, the extracellular component can be designed to bind target antigens found on cancer cells and, when bound, the intracellular component directs the T cell to destroy the bound cancer cell. Examples of such molecules include genetically engineered T cell receptors (TCR) and chimeric antigen receptors (CAR).

While genetically engineered T cells provide a significant advance in the ability to target and destroy unwanted cell types, they require immunological matching with each particular subject before they can be used in a treatment setting. Once a donor match is found (or T cells are obtained from a subject needing treatment), the cells must be modified and expanded before they can be used in the subject. This time-intensive and expensive process can cause, in some instances, lethal delays in treatment.

### SUMMARY OF THE DISCLOSURE

**The** current disclosure provides genetically modified stem cells that can be administered as therapeutics without the need for immunological matching to particular subjects. Thus, these modified stem cells may be provided as "off-the-shelf" treatments removing delays and expense in treatment associated with donor identification and subsequent cell modification and expansion. The modified stem cells can be administered alone or in combination with various other treatments to obtain numerous treatment objectives. In particular embodiments, the modified stem cells are differentiated into modified non-T effector cells before administration.

More particularly, hematopoietic stem/progenitor cells (HSPC) are genetically modified to express molecules having an extracellular component that binds particular cellular markers preferentially found on unwanted cell types and an intracellular component that directs actions of the genetically modified cell when the extracellular component has bound the cellular marker. As an example, the extracellular component can be designed to bind cellular markers preferentially found on cancer cells and, when bound, the intracellular component directs the genetically modified cell to destroy the bound cancer cell. Examples of such molecules include genetically engineered T cell receptors (TCR), chimeric antigen receptors (CAR), and other molecules disclosed herein. In particular embodiments, the modified HSPC can be differentiated into non-T effector cells before administration.

### BRIEF DESCRIPTION OF THE FIGURES

FIG. 1. Nucleotide sequence of anti-CD19 short spacer chimeric receptor, GMCSFRss-CD19scFv-IgG4hinge-CD28tm-41 BB-Zeta-T2A-EGFRt.
FIG 2. Amino acid sequence of GMCSFRss-CD19scFv-IgG4hinge-CD28tm-41BB-Zeta-T2A-EGFRt.

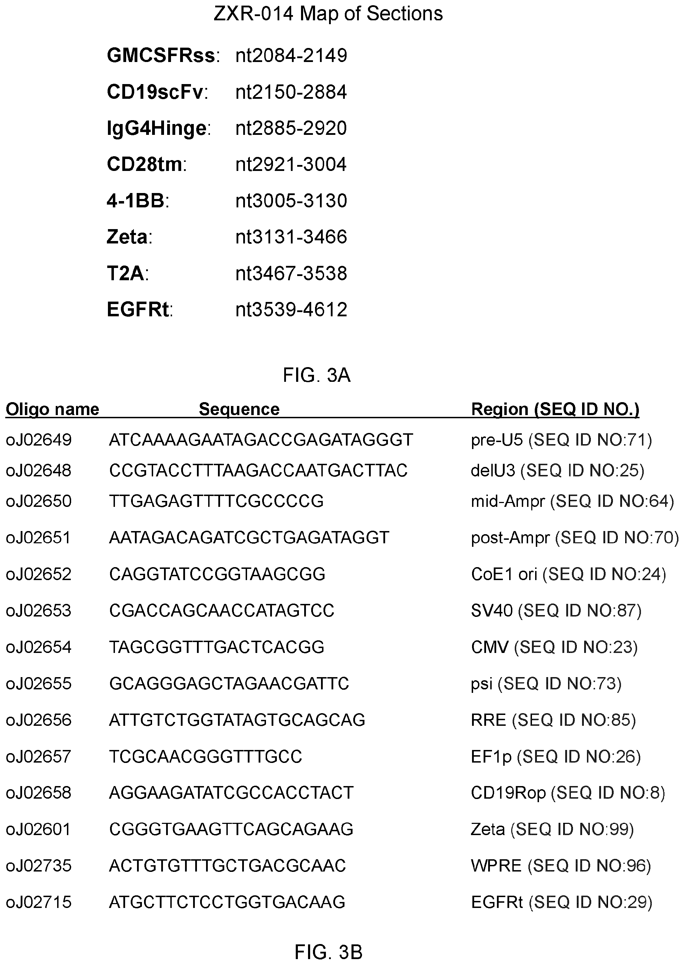

FIG. 3A and 3B. FIG. 3A shows a map of the sections of ZXR-014 nucleotide and amino acid sequences. FIG. 3B shows exemplary primer sequences.

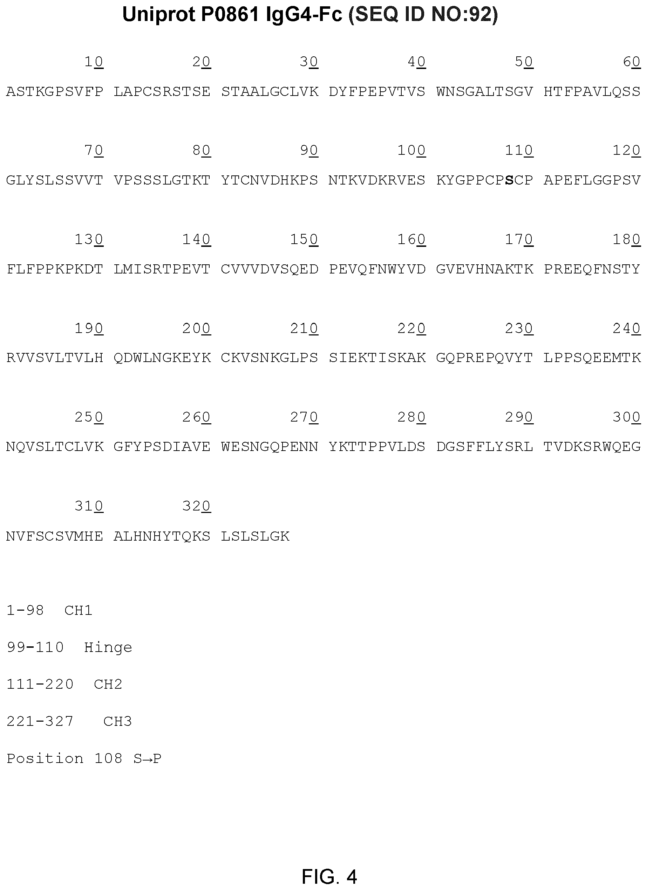

FIG. 4. Amino acid sequence and map of sections of Uniprot P0861 IgG4-Fc.
FIG. 5. Amino acid sequence and map of sections of Uniprot P10747 CD28.
FIG. 6. Amino acid sequence and map of sections of Uniprot Q07011 4-1BB.
FIG. 7. Amino acid sequence and map of sections of Uniprot P20963 human CD3ζ isoform 3.

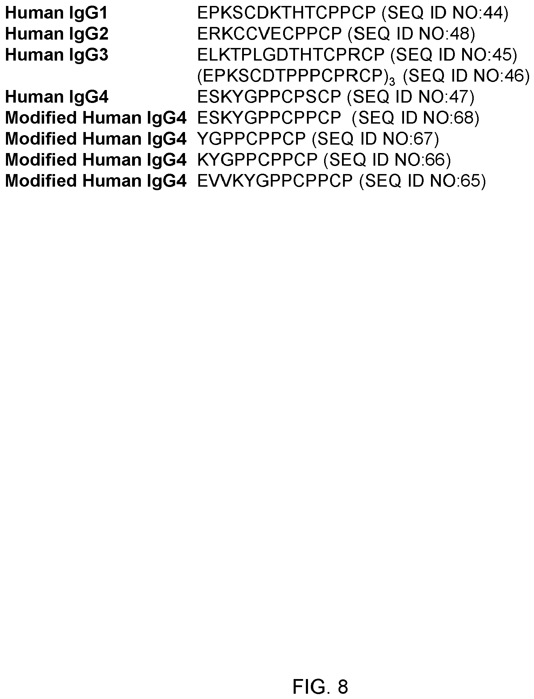

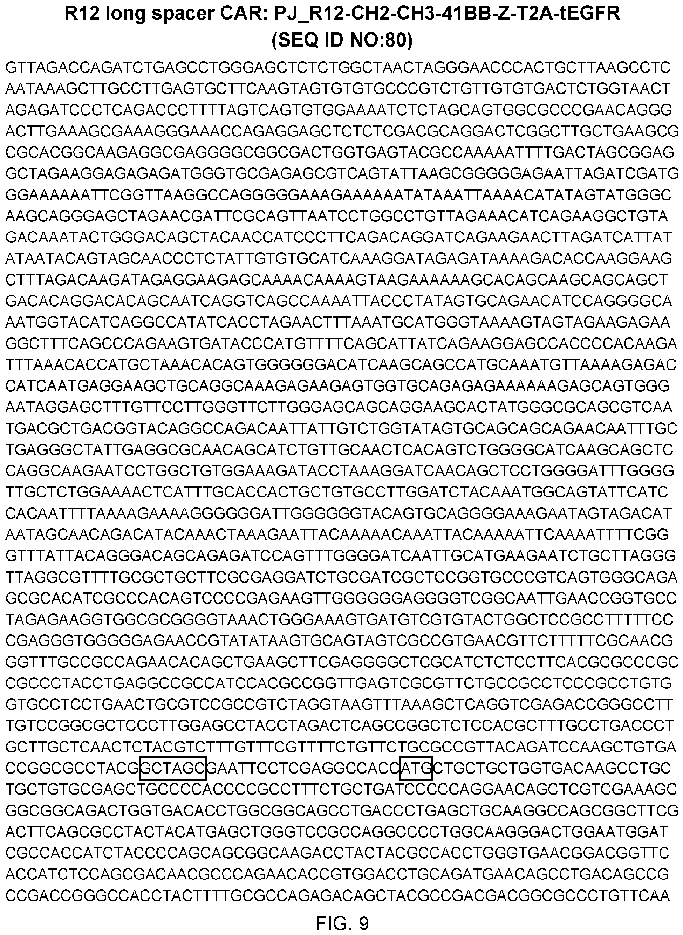

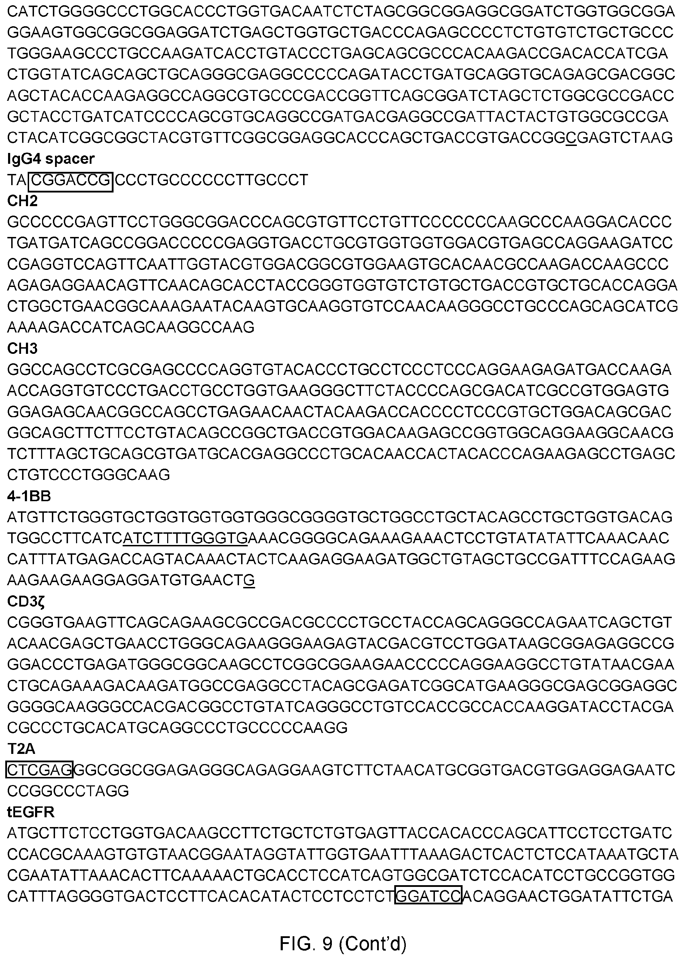

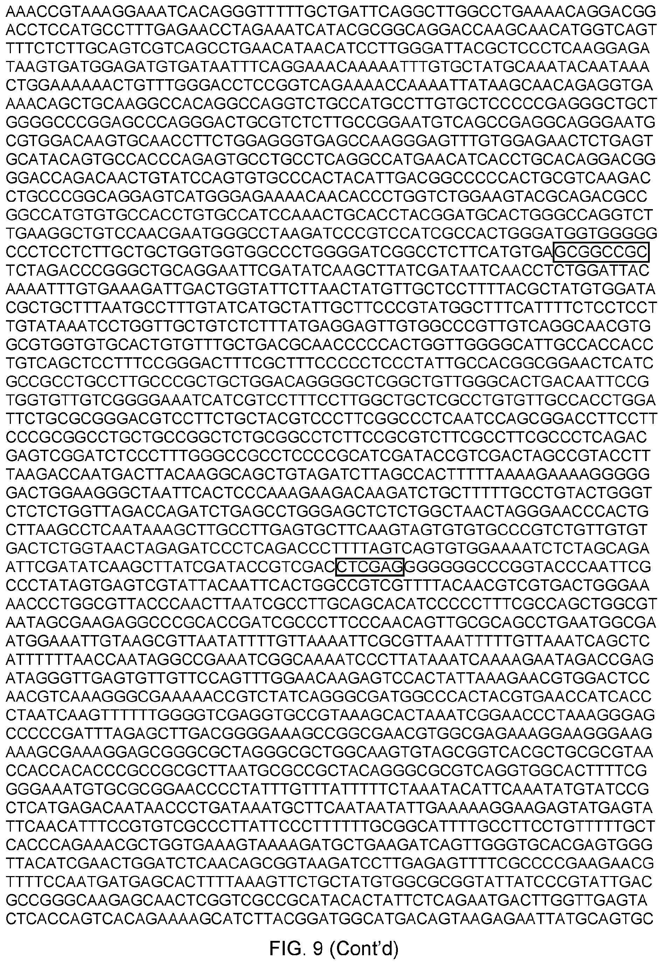

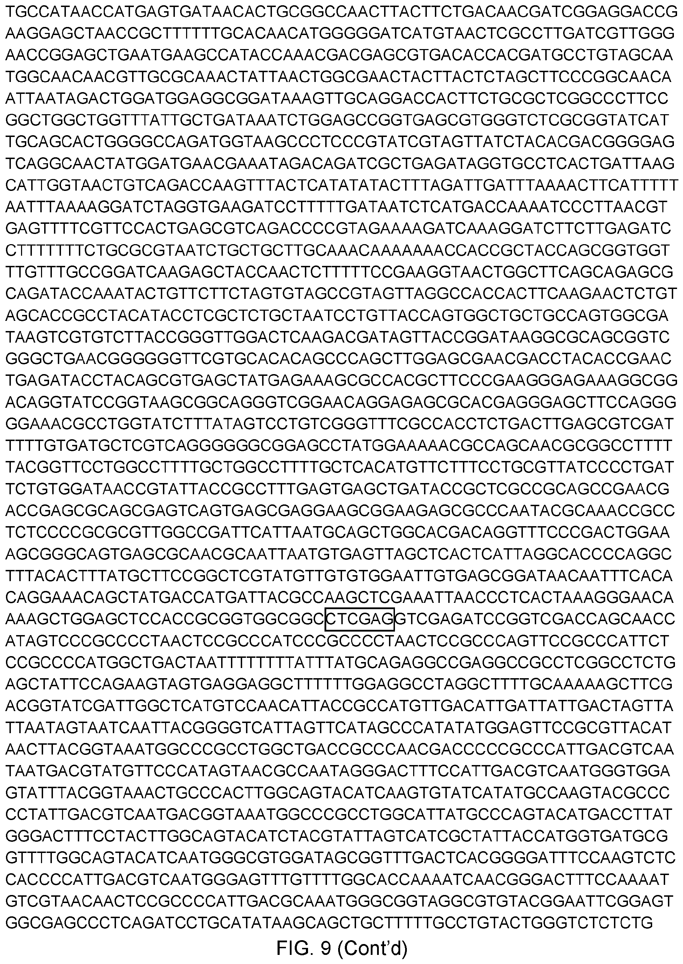

FIG. 8. Exemplary hinge region sequences.
FIG**.** 9. Sequence of R12 long spacer CAR: PJ_R12-CH2-CH3-41BB-Z-T2A-tEGFR.

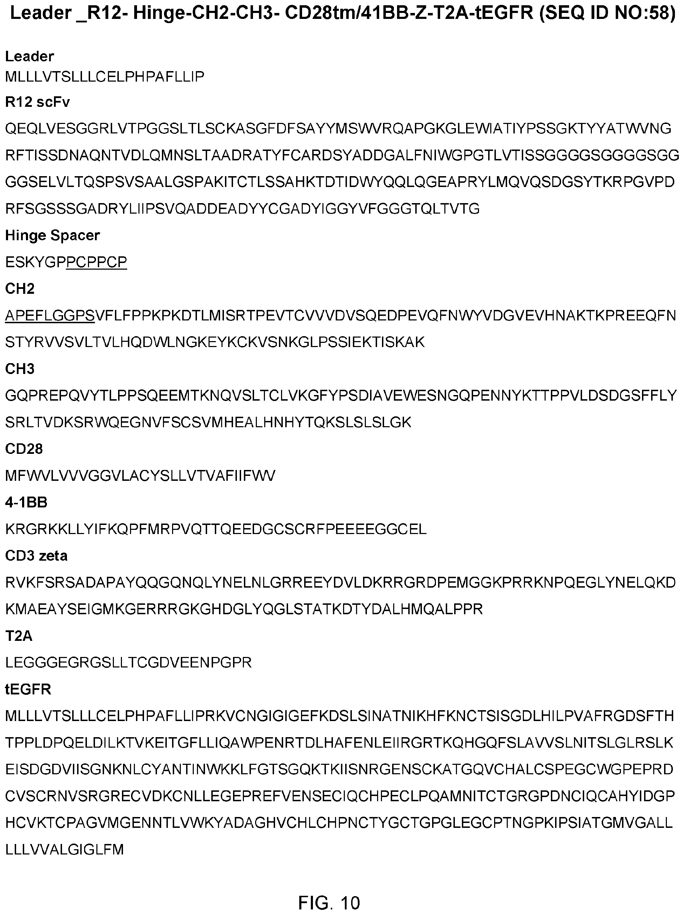

FIG. 10. Sequence of Leader_R12-Hinge-CH2-CH3-CD28tm/41BB-Z-T2A-tEGFR.

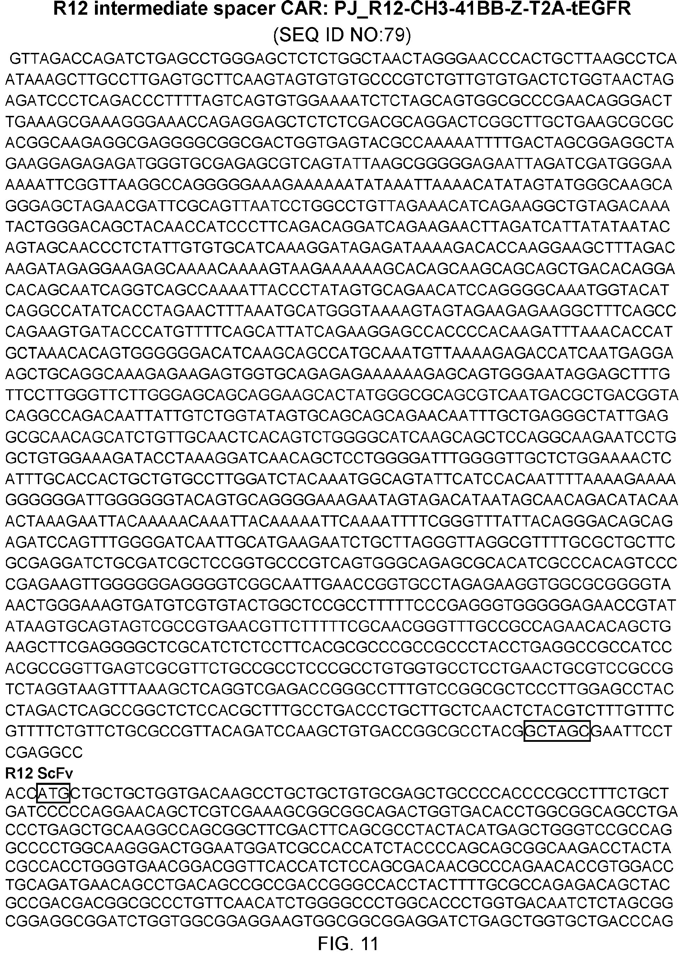

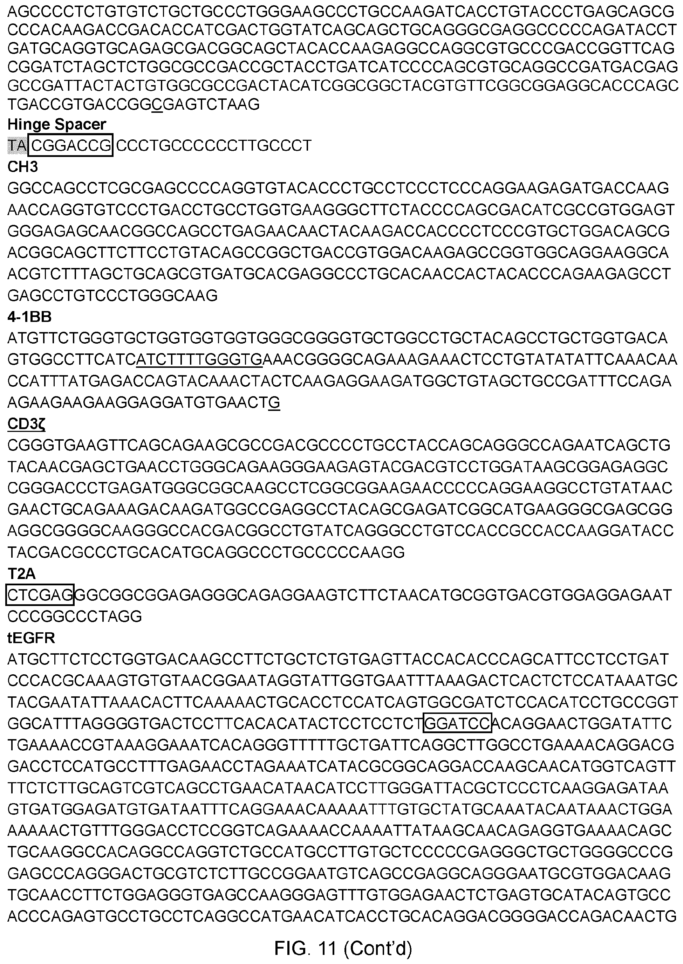

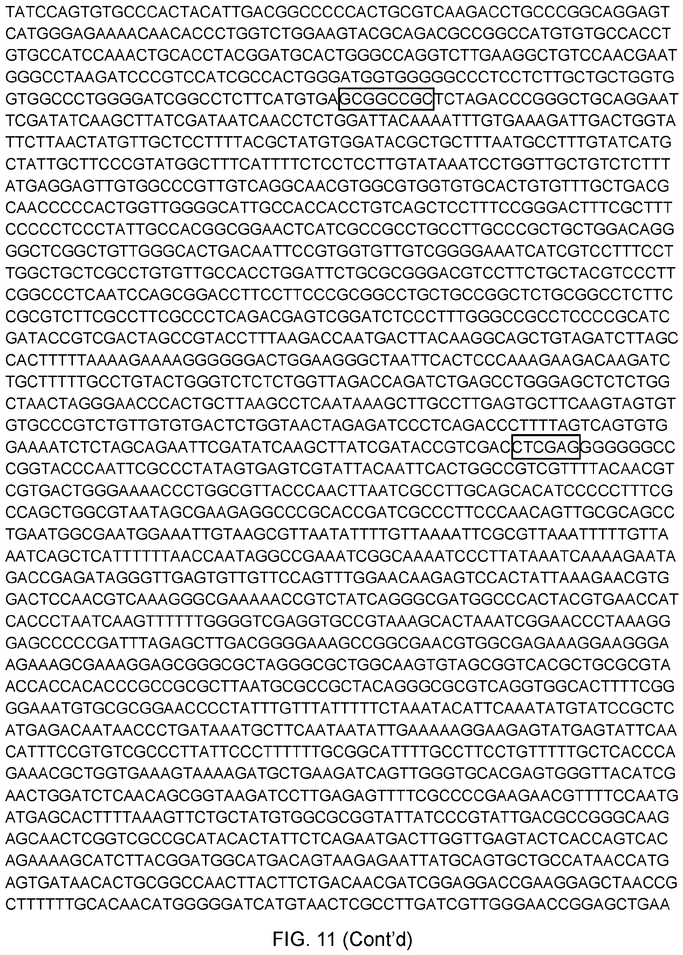

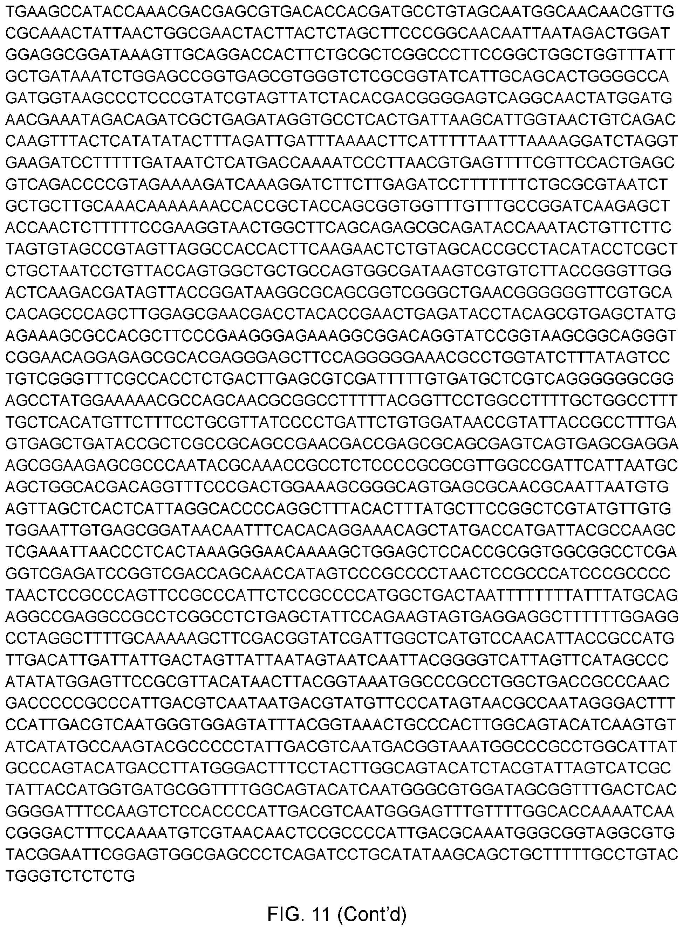

FIG. 11. Sequence of R12 intermediate spacer CAR: PJ_R12-CH3-41BB-Z-T2A-tEGFR.
FIG. 12. Sequence of Leader _R12-Hinge-CH3-CD28tm/41BB-Z-T2A-tEGFR.
FIG. 13. Sequence of R12 short spacer CAR: PJ_R12-Hinge-41 BB-Z-T2A-tEGFR.

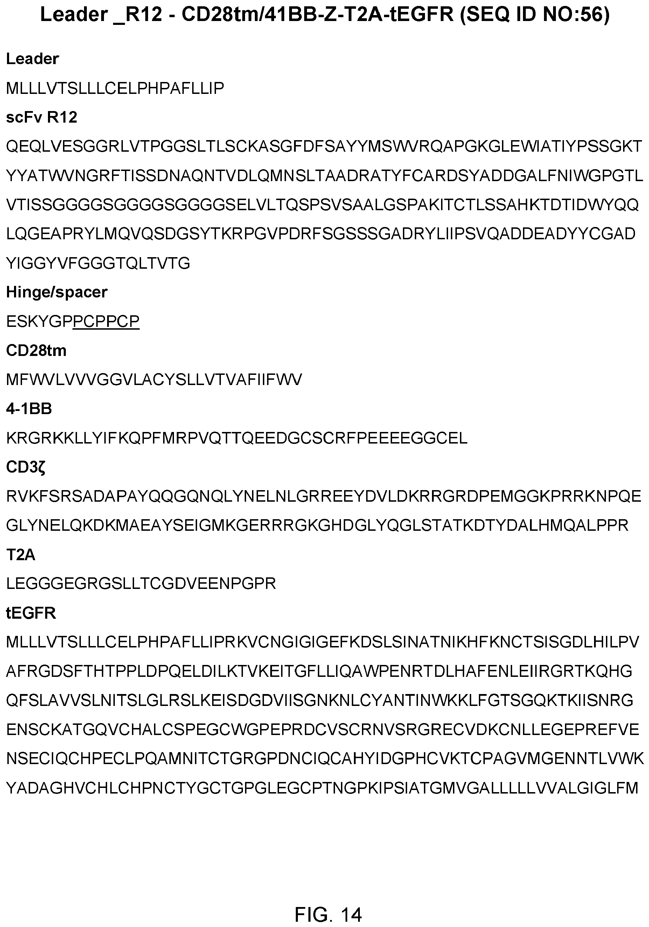

FIG. 14. Sequence of Leader_R12-CD28tm/41BB-Z-T2A-tEGFR.

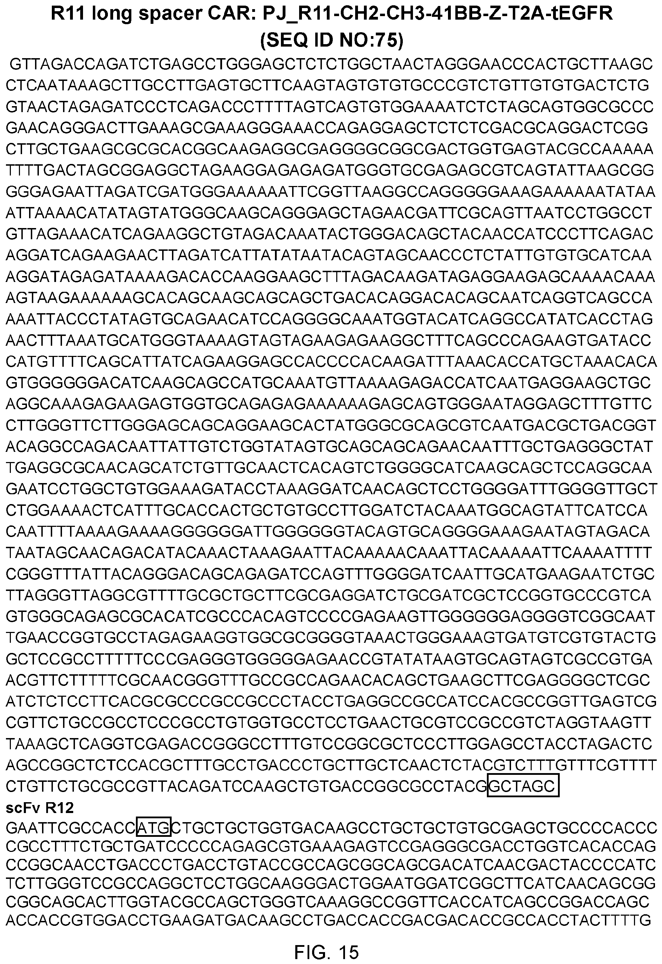

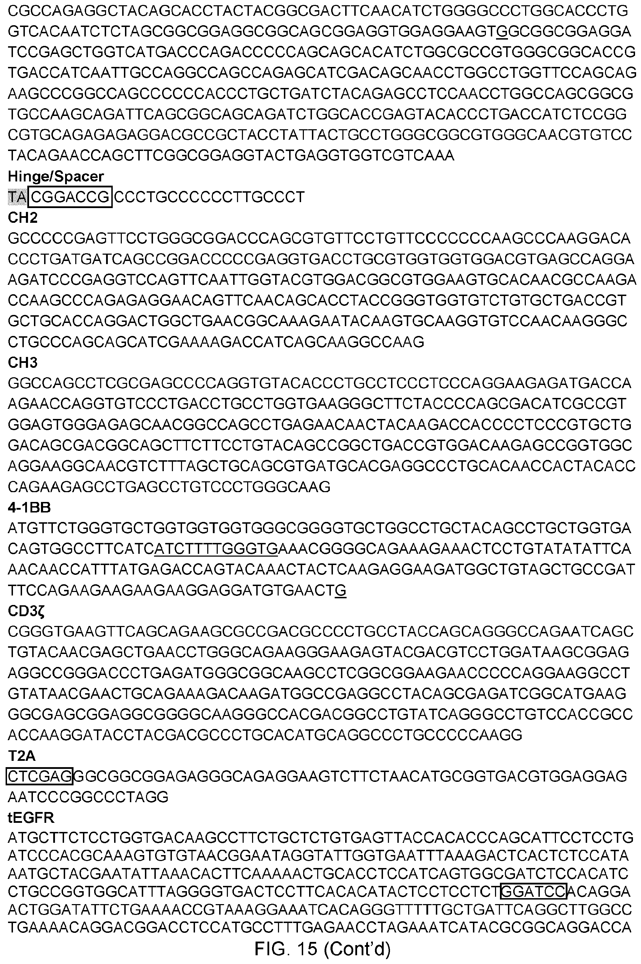

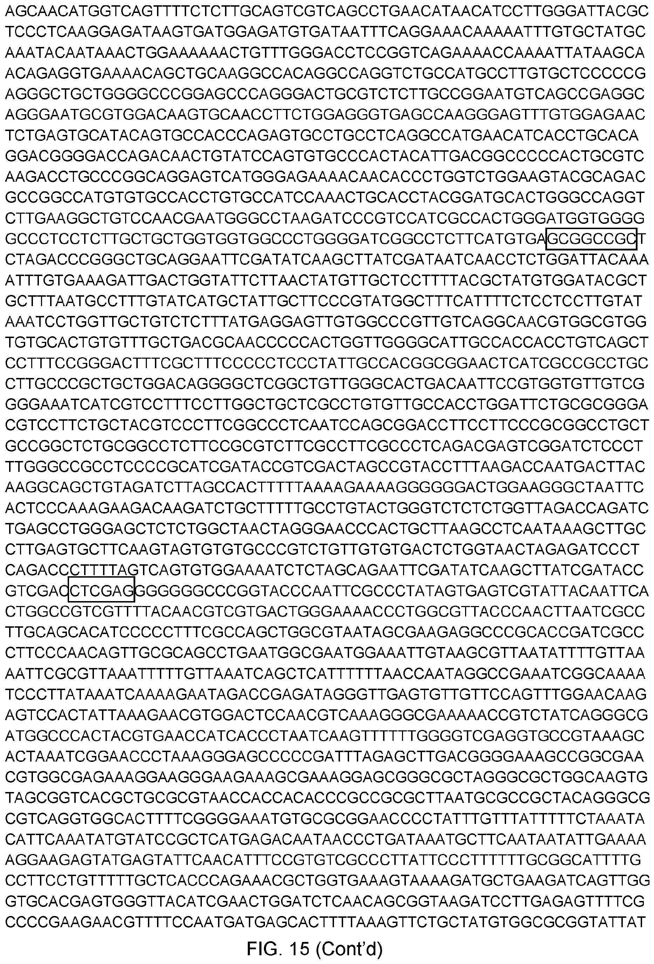

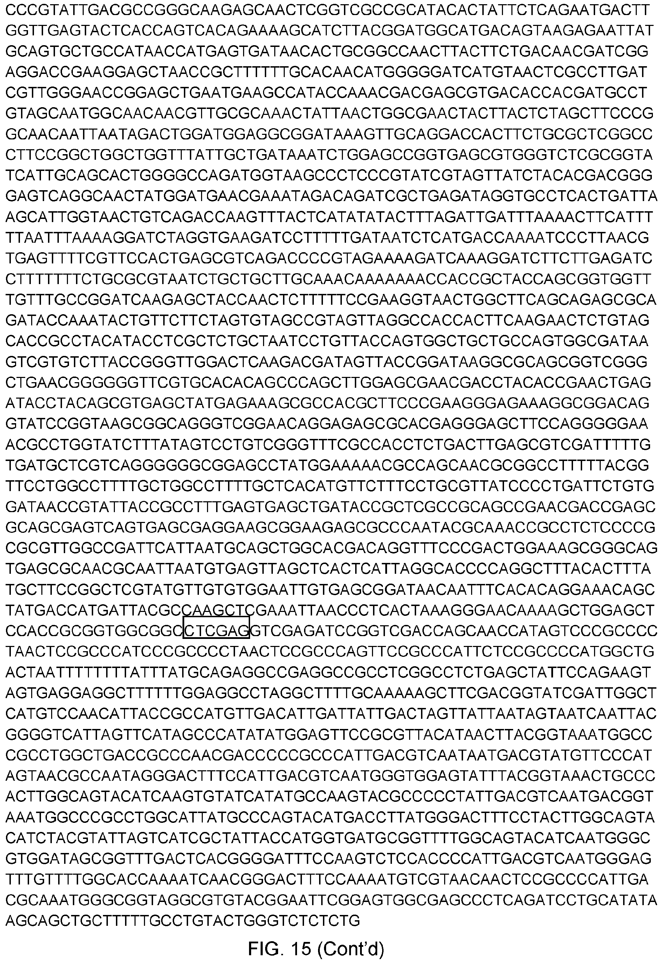

FIG. 15. Sequence of R11 long spacer CAR: PJ_R11-CH2-CH3-41BB-Z-T2A-tEGFR.

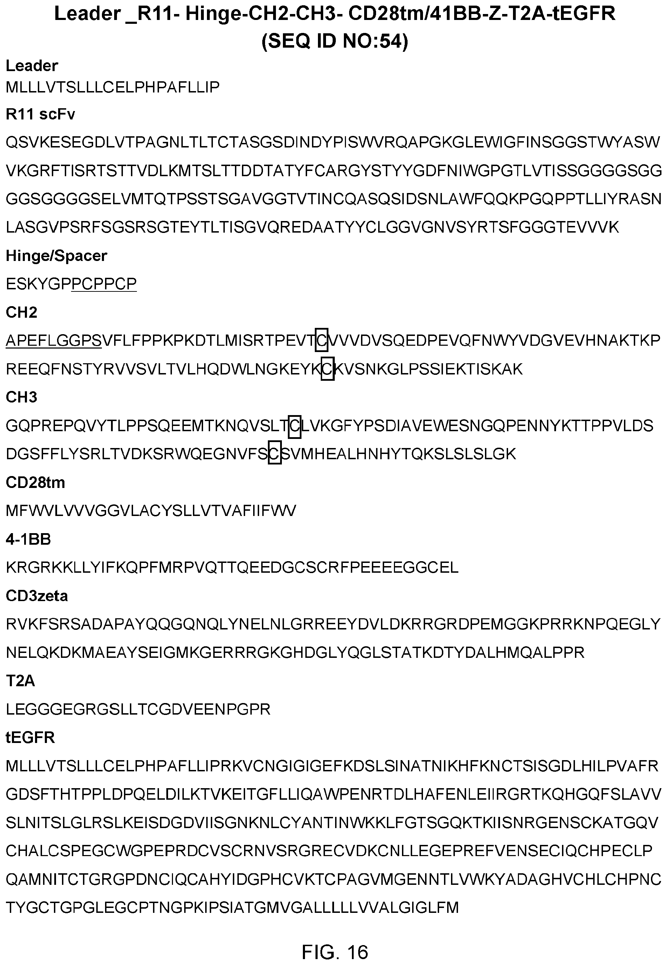

FIG. 16. Sequence of Leader_R11-Hinge-CH2-CH3-CD28tm/41BB-Z-T2A-tEGFR.

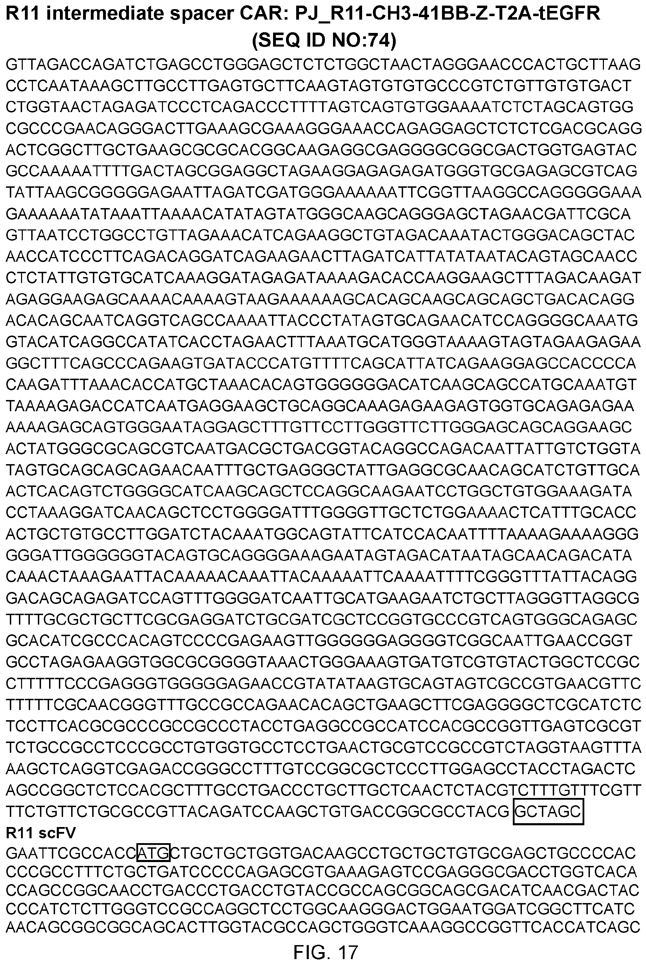

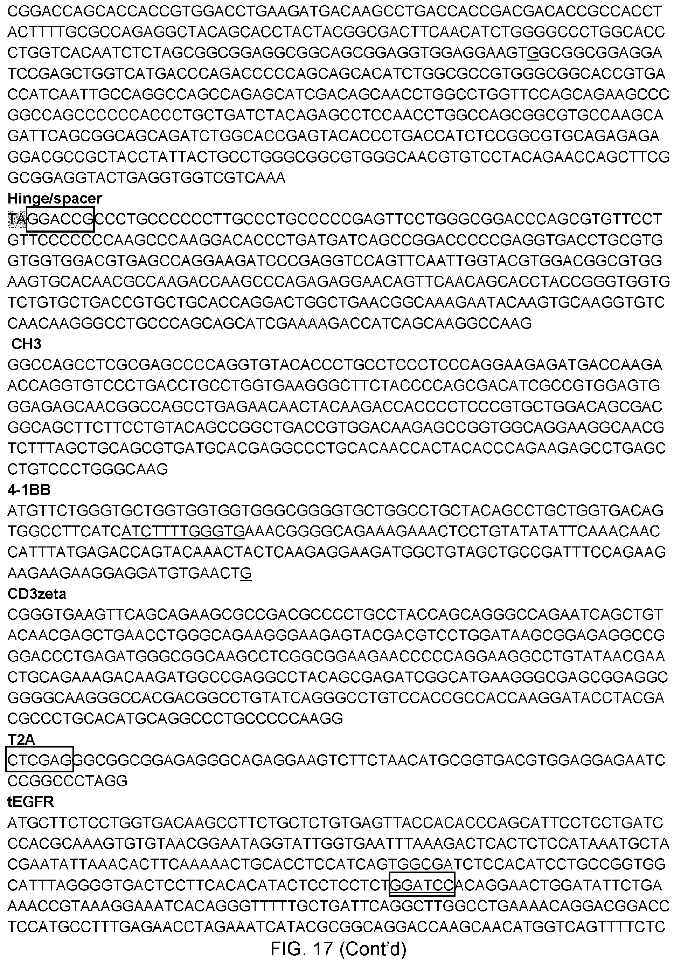

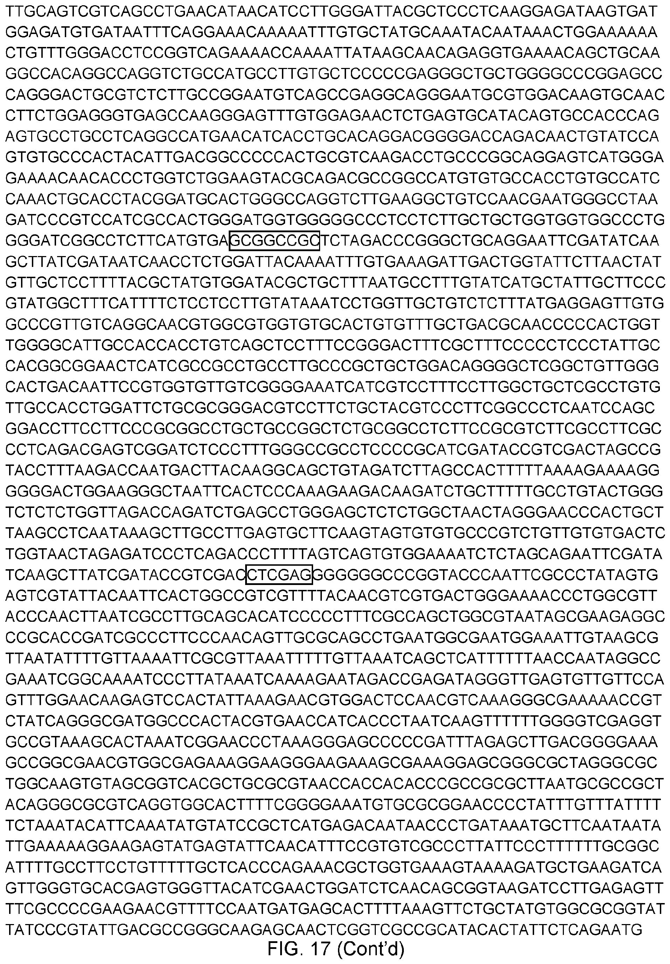

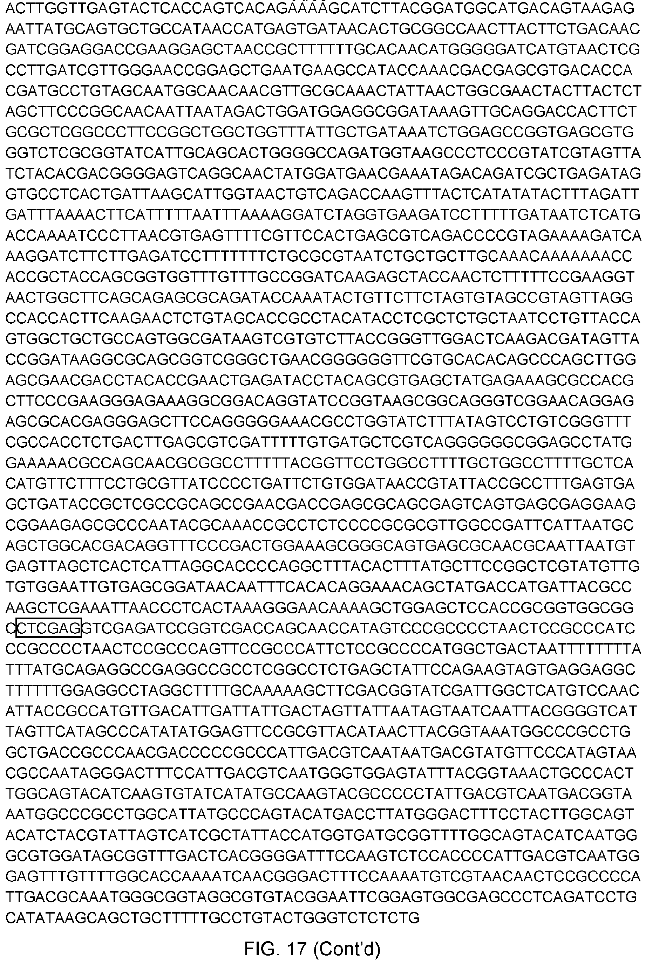

FIG. 17. Sequence of R11 intermediate spacer CAR: PJ_R11-CH3-41BB-Z-T2A-tEGFR.

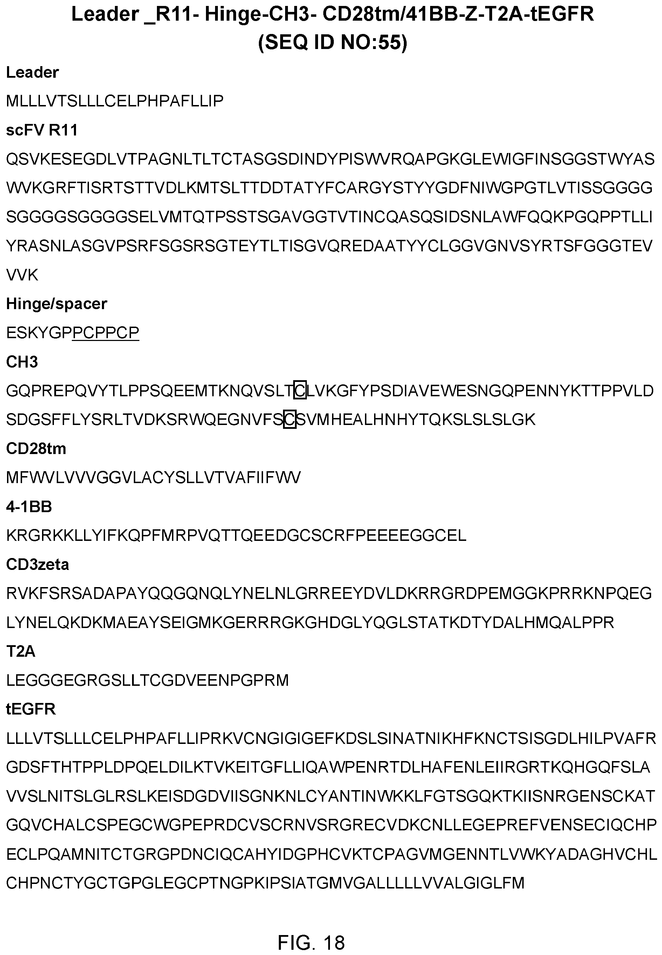

FIG. 18. Sequence of Leader_R11-Hinge-CH3-CD28tm/41BB-Z-T2A-tEGFR.

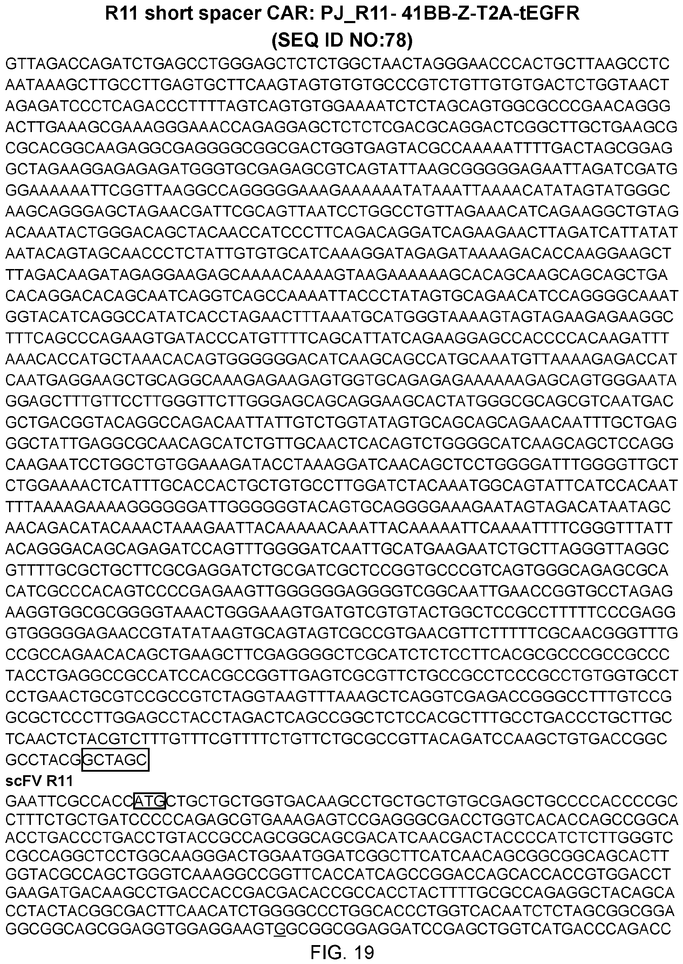

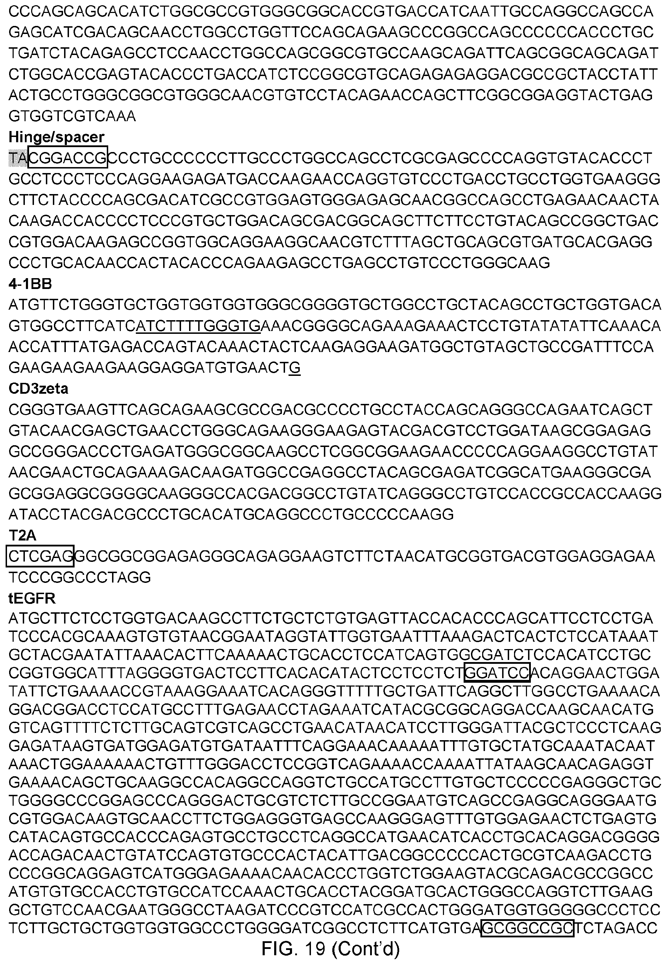

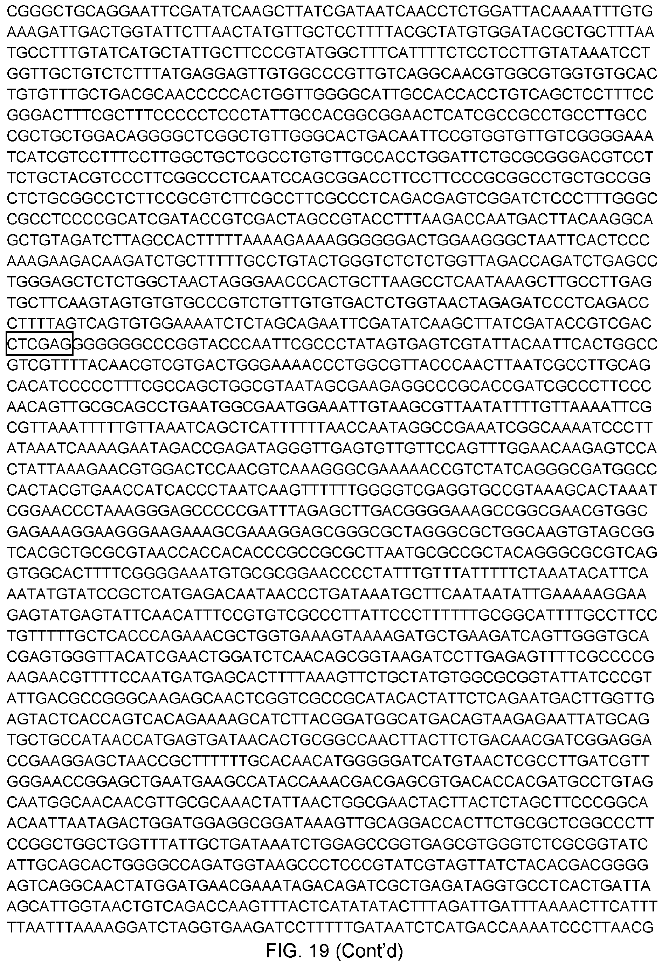

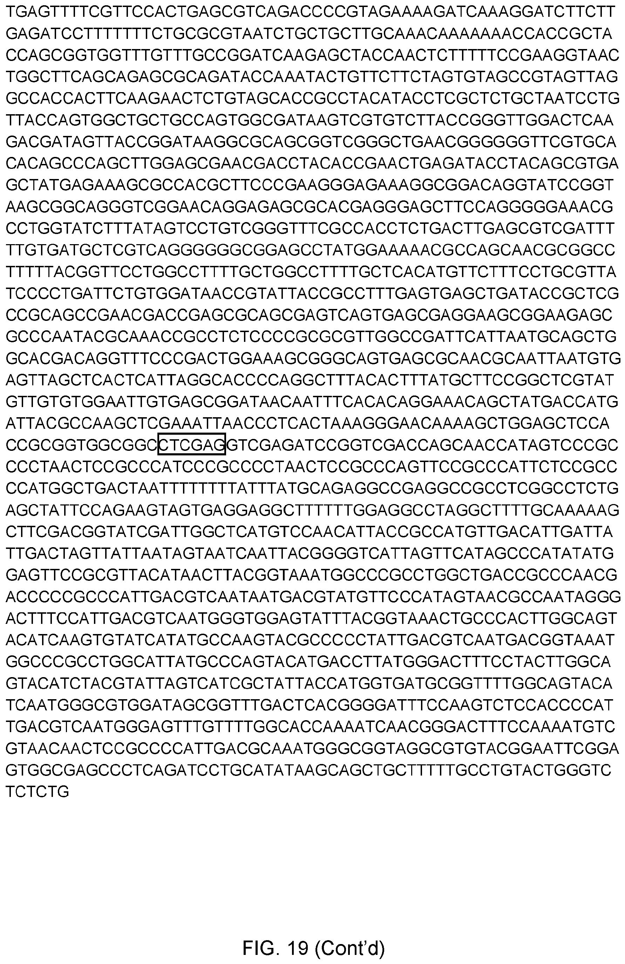

FIG. 19. Sequence of R11 short spacer CAR: PJ_R11-41BB-Z-T2A-tEGFR.

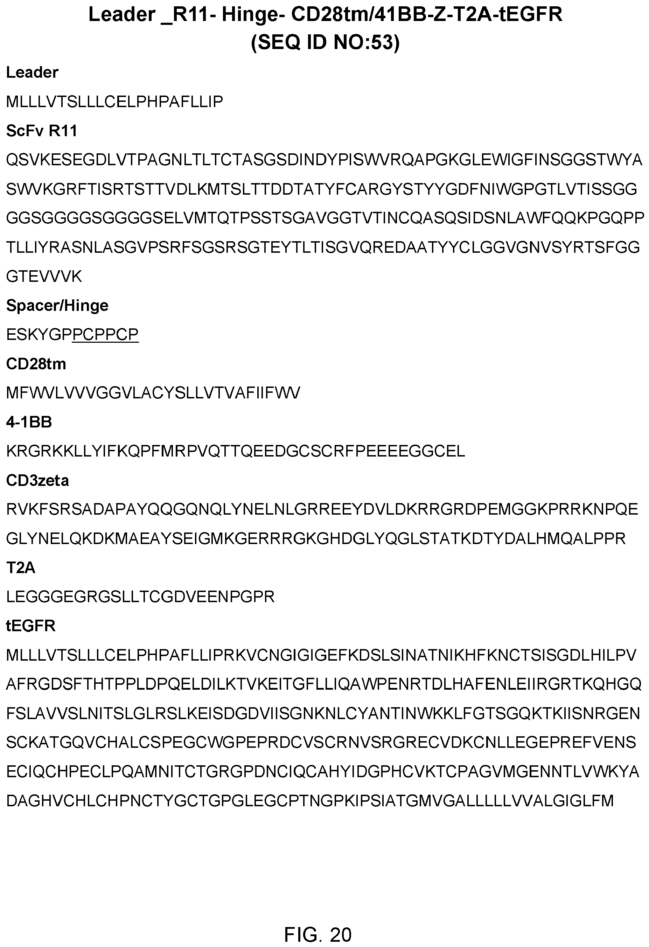

FIG. 20. Sequence of Leader_R11-Hinge-CD28tm/41BB-Z-T2A-tEGFR.

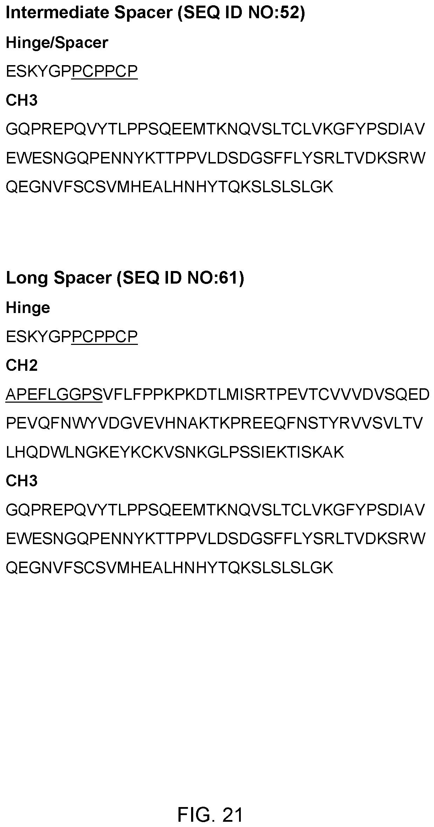

FIG. 21. Exemplary spacer sequences.

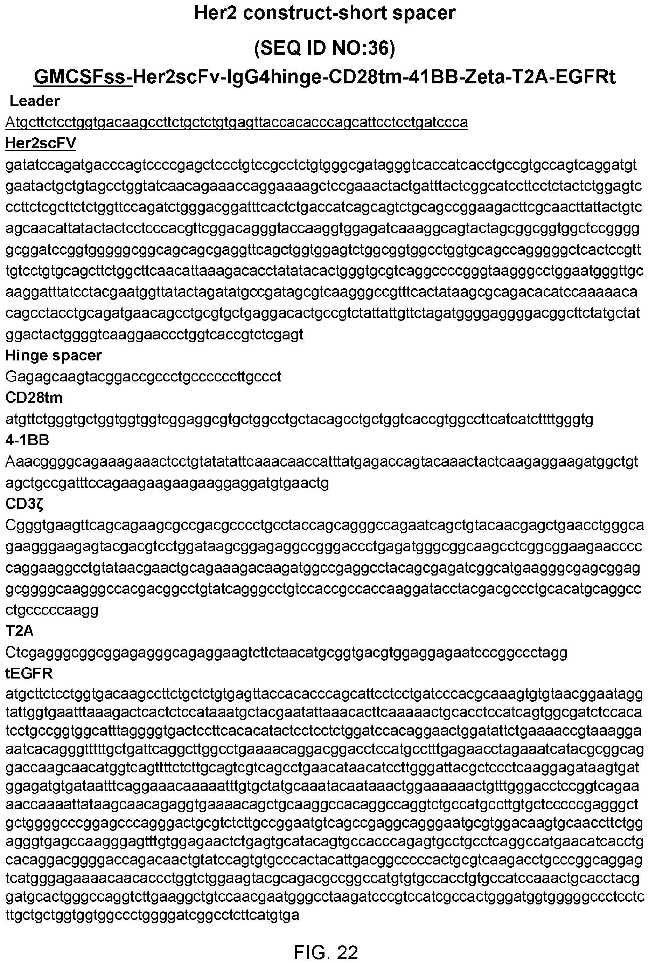

FIG. 22. Sequence of Her2 short-spacer construct, GMCSFss-Her2scFv-IgG4hinge-CD28tm-41BB-Zeta-T2A-EGFRt.

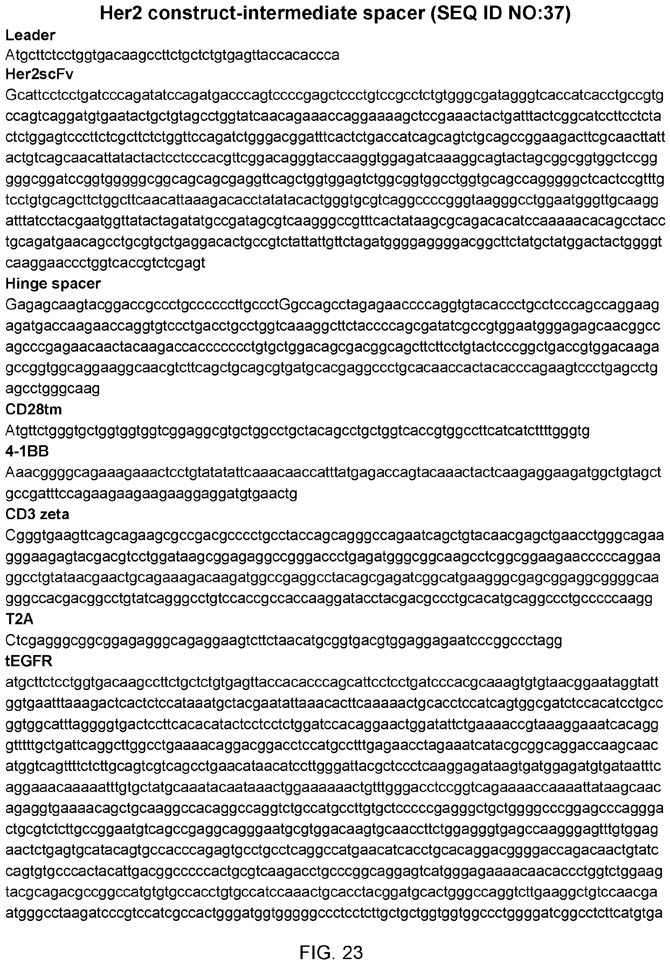

FIG. 23. Sequence of intermediate spacer Her2 construct.

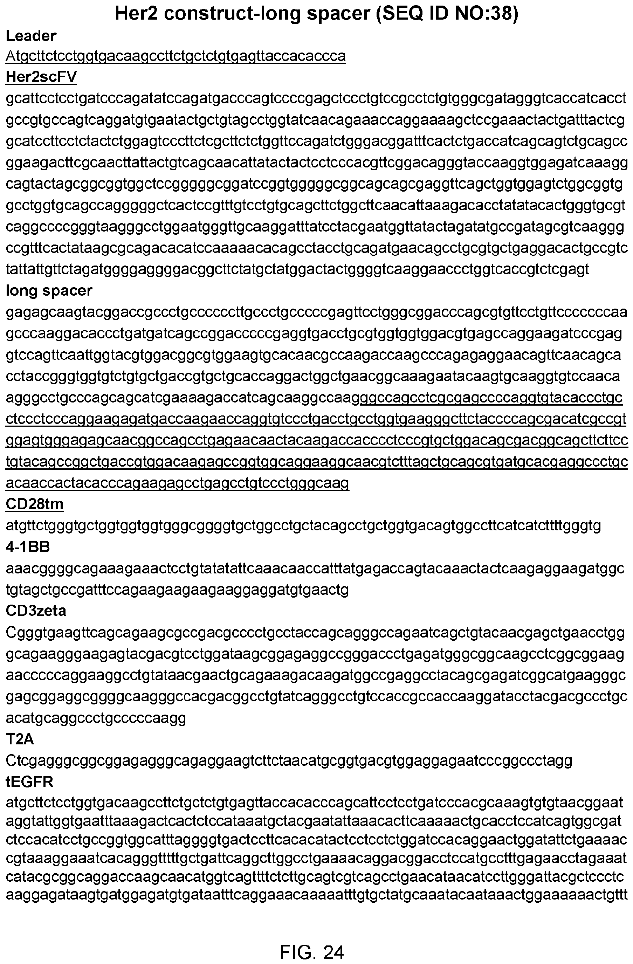

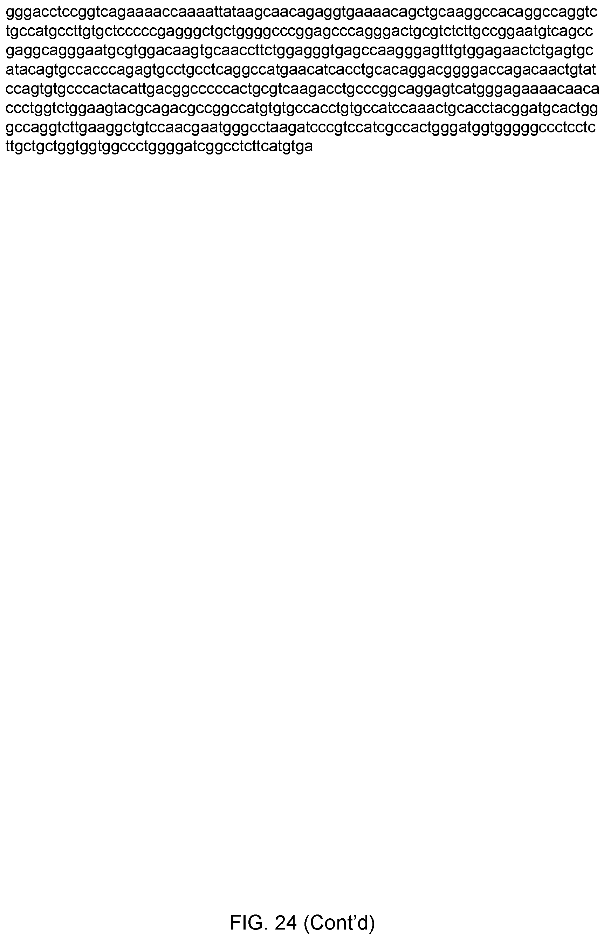

FIG. 24. Sequence of long spacer Her2 construct.

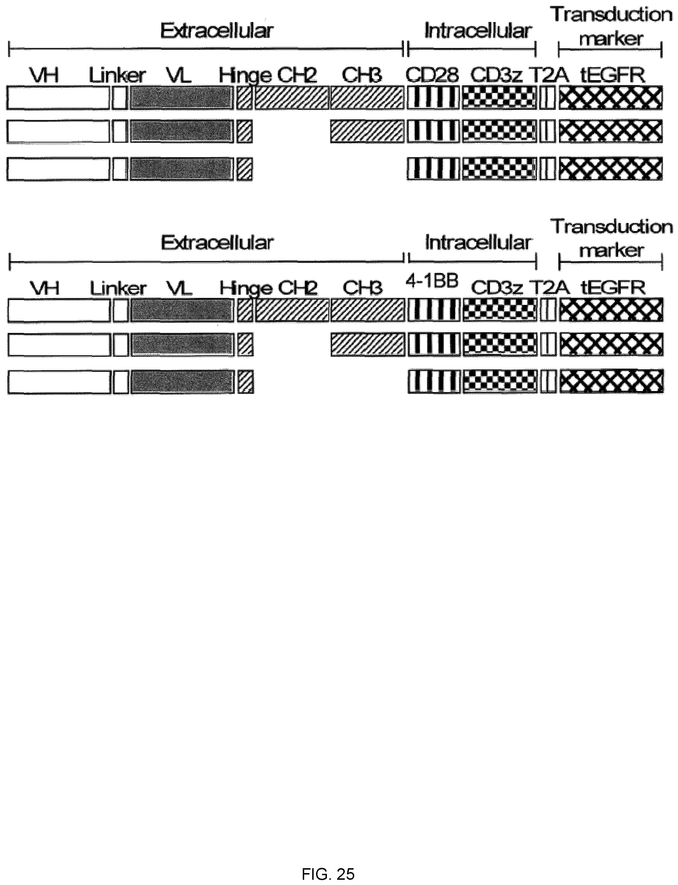

FIG. 25. Library of spacer sequences. A plasmid library was constructed which contains codon optimized DNA sequences that encode extracellular components including portions of the IgG4 hinge, the IgG4 hinge linked to CH2 and CH3 domains, or the IgG4 hinge linked to the CH3 domain. Any scFV sequence (VH and VL) can be cloned 5' to the sequences encoded in this library of variable spacer domains. The spacer domains are in turn linked to CD28 transmembrane and intracellular signaling domains and to CD3 ζ. A T2A sequence in the vector separates the chimeric receptor from a selectable marker encoding a truncated human epidermal growth factor receptor (EGFR).

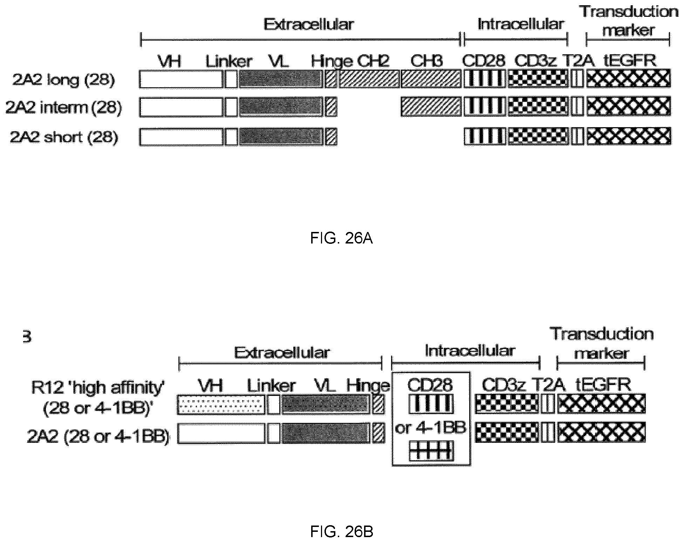

FIG. 26A and 26B. Design of ROR1 chimeric receptors with modified spacer length and derived from the 2A2 and R12 scFV with different affinity. (FIG. 26A) Design of lentiviral transgene inserts encoding a panel of ROR1 chimeric receptors containing the 2A2 scFV, an IgG4-Fc derived spacer of 'Hinge-CH2-CH3' (long spacer, 229 AA), 'Hinge-CH3' (intermediate, 119 AA), or 'Hinge' only (short, 12 AA), and a signaling module with CD3ζ and CD28. Each chimeric receptor cassette contains a truncated EGFR marker encoded downstream of a T2A element. (FIG. 26B) Lentiviral transgene inserts encoding ROR1-specific chimeric receptors derived from the R12 and 2A2 scFV with short IgG4-Fc 'Hinge' spacer (12 AA), and a signaling module containing CD28 or 4-1BB and CD3ζ respectively (total: 4 constructs).

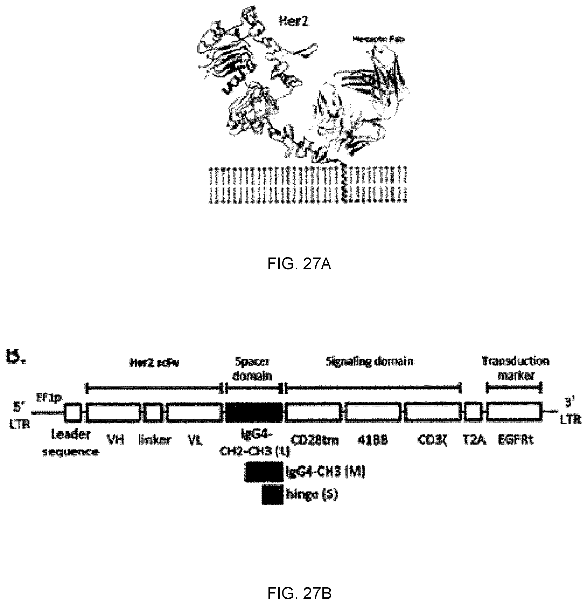

FIG. 27A and 27B. FIG. 27A) Depiction of Herceptin Fab epitope location on tumor cell membrane proximal epitope on human HER2, FIG. 27B) Structural formats of Herceptin scFv CAR spacer length variants as -T2A- linked proteins with the carboxyl EGFRt marker transmembrane protein.

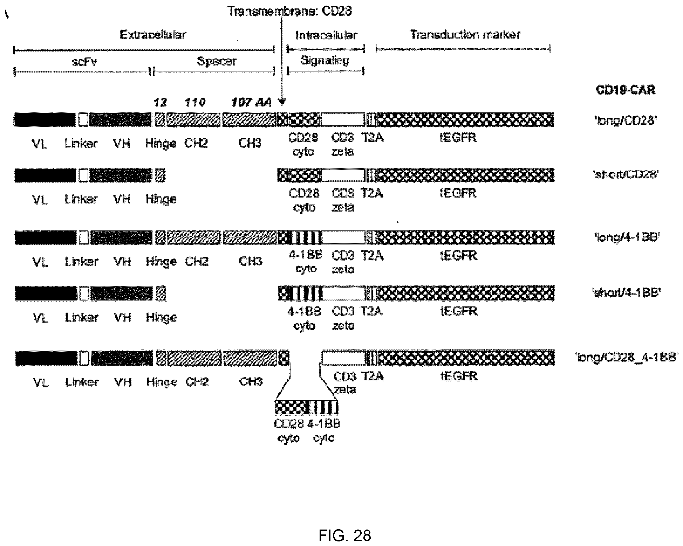

FIG. 28. CD19-chimeric receptor vectors. Design of lentiviral transgene inserts encoding a panel of CD19-specific chimeric receptors that differ in extracellular spacer length and intracellular co-stimulation. Each chimeric receptor encoded the CD19-specific single chain variable fragment derived from the FMC63 mAb in a VL-VH orientation, an IgG4-derived spacer domain of Hinge-CH2-CH3 (long spacer, 229 AA) or Hinge only (short spacer, 12 AA), and a signaling module containing CD3ζ with CD28 or 4-1BB alone or in tandem. Each chimeric receptor cassette contains a truncated EGFR marker encoded downstream of a cleavable 2A element.
FIG. 29A and 29B. Exemplary SIN lentiviral plasmids. FIG. 29A shows a SIN CD19 specific scFvFc-CD3ζCD28 CAR and huEGFRt lentiviral plasmid. FIG. 29B shows SIN CD19-specific scFv-4-1BBCD3ζ CAR and huEGFRt lentiviral plasmid.
FIG. 30A and 30B. EGFR expression as a marker of transduction efficiency/gene expression stability by percent (FIG. 30A) and absolute number (FIG. 30B). HSPC were cultured on Delta as previously described. On day +3, the cells were transduced using scFvFc-CD3ζCD28 CAR and huEGFRt vector at an MOI of 3 in the presence of protamine sulfate and underwent spinfection. Transgene expression was measured over the course of the culture by flow using Erbitux, which binds to the EGFRt tag. Designated cultures had irradiated LCL added at a 1:1 ratio on day +7.
FIG. 31. CD34+ CB cells cultured on Notch ligand underwent transduction with lentivirus on day + 3 with a MOI of 3 using scFvFc-CD3ζCD28 CAR and huEGFRt vector. LCL was added to indicated cultures on day 7 at a 1:1 ratio (transduced (■), transduced with LCL (X), non-transduced (largely unseen, behind ■ line), non-transduced with LCL (▲)). CD34 fold expansion was enhanced with addition of LCL through an overall TNC fold expansion.
FIG. 32. Day 14 MOI 3 using scFv-4-1 BB/CD3ζ CAR and huEGFRt vector for transduction with and without LCL. The addition of LCL at day +7 did not appear to drive proliferation of CAR expressing HSPC or their progeny as noted by similar population distributions among the culture with and without LCL.

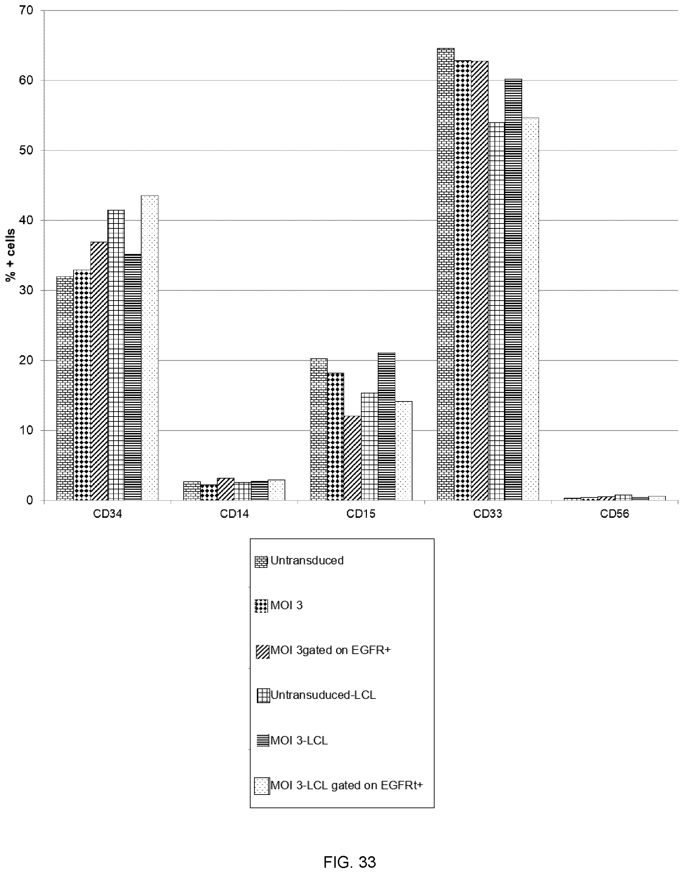

FIG. 33. End of culture phenotype. HSPC were cultured on Delta as previously described. Designated cultures were transduced on day + 3 at an MOI of 3 with lentivirus to express a scFv-4-1BB/CD3ζ CAR and huEGFRt. Additionally, designated cultures were given irradiated LCL at a 1:1 ratio on day +7. Cultures were analyzed by flow cytometry on day 14. There were no significant differences detected between the transduced and untransduced cultures. Likewise, there were no differences detected between the total population of cells and the EGFRt+ cells suggesting that the CAR construct is equally distributed among the subgroups.
FIG. 34. Functional analysis of scFvFc-CD3ζCD28 CAR and huEGFRt vector. At the end of 14 days of culture on Delta, cells were taken off Delta, placed in RPMI media supplemented with IL-2 and IL-15 for an additional week to derive an NK population.
FIG. 35. A chromium release assay with target cell of K562 (x and ● ) or LCL (▲ and ◆) using NK effector cells derived from CD34+ CB cells expanded on Notch ligand and transduced to express a CD19 specific scFvFc-CD3ζCD28 CAR and huEGFRt (● and ◆) or non-transduced (▲ and x). Mature NK cells were derived by an additional week in culture with RPMI, IL-2 and IL-15.
FIG. 36. Mice receiving transduced cells using scFv-4-1BB/CD3ζ CAR and huEGFRt vector had impaired engraftment of CD19, thereby demonstrating anti-CD19 effects, which was dependent upon expression of the transgene.
FIG. 37. NOG mice receiving cells from cultures that were transduced with lentivirus encoding for scFv-4-1BB/003ζ CAR and huEGFRt and show significant EGFRt expression and reduced CD19 engraftment.

### DETAILED DESCRIPTION

Significant progress has been made in genetically engineering T cells of the immune system to target and kill unwanted cell types, such as cancer cells. For example, T cells have been genetically engineered to express molecules having an extracellular component that binds particular target antigens and an intracellular component that directs actions of the T cell when the extracellular component has bound the target antigen. As an example, the extracellular component can be designed to bind target antigens preferentially found on cancer cells and, when bound, the intracellular component directs the T cell to destroy the bound cancer cell. Examples of such molecules include genetically engineered T cell receptors (TCR) and chimeric antigen receptors (CAR).

While genetically engineered T cells provide a significant advance in the ability to target and destroy unwanted cell types, they require immunological matching with each particular subject before they can be used in a treatment setting. Once a donor match is found (or T cells are obtained from a subject in need of treatment), the cells must be modified and expanded before they can be used in the subject. This time-intensive and expensive process can cause, in some instances, lethal delays in treatment.

The current disclosure provides genetically modified stem cells that can be administered as therapeutics without the need for immunological matching to particular subjects. Thus, these modified stem cells may be provided as "off-the-shelf" treatments eliminating delays and expenses in treatment associated with donor identification and subsequent cell modification and expansion. The modified stem cells can be administered alone or in combination with various other treatments to obtain numerous treatment objectives. In particular embodiments, the modified stem cells can be differentiated into non-T effector cells before administration.

More particularly, hematopoietic stem/progenitor cells (HSPC) are genetically modified to express molecules having an extracellular component that binds particular cellular markers and an intracellular component that directs actions of the genetically modified cell when the extracellular component has bound the cellular marker. As an example, the extracellular component can be designed to bind cellular markers preferentially found on cancer cells and, when bound, the intracellular component directs the genetically modified cell to destroy the bound cancer cell. Examples of such molecules include genetically engineered T cell receptors (TCR), chimeric antigen receptors (CAR), and other molecules disclosed herein. The HSPC can be differentiated into non-T effector cells before administration.

**As** an exemplary use of a particular embodiment, cord blood transplant (CBT) is a standard of care for relapsed pediatric acute lymphoblastic leukemia (ALL) when a suitably matched donor cannot be identified. This is particularly important for patients of minority or mixed ethnicity background (and 30% of Caucasians) who are very unlikely to find a suitable donor.

The ability of CBT to eradicate ALL and provide a durable remission is due in part to a graft-versus-leukemia (GVL) effect. Still, however, the rate of relapse for ALL post CBT is around 40% (Smith et al., Biol Blood Marrow Transplant, 2009. 15(9): p. 1086-93; Tomblyn et al., J Clin Oncol, 2009. 27(22): p. 3634-41) with overall survival related to both relapse and treatment related mortality, including graft-versus-host disease (GVHD). Compositions and formulations disclosed herein can enhance the GVL effect, without increasing rates of GVHD. This strategy is clinically feasible using ex vivo expansion of cord blood (CB) HSPC through activation of the endogenous Notch signaling pathway using a Notch ligand, resulting in a greater than 100 fold increase of CD34+ cells. Clinically, the expanded HSPC can be infused along with an unmanipulated unit, leading to a transient engraftment of the expanded HSPC, with progeny derived from the expanded unit, while long-term engraftment is ultimately derived from the unmanipulated unit.

Notch ligand expanded CB HSPC are amenable to genetic modification using vectors that express a CD19-specific CAR. By taking advantage of the Notch ligand CB expansion system, GVL can be engineered into CBT by the genetic modification of expanded HSPC to express a CD19 CAR, whereby the engrafted myeloid and lymphoid effector cells recognize and lyse residual leukemia cells.

**The** claimed invention is now described more generally.

Hematopoietic Stem/Progenitor Cells or HSPC refer to hematopoietic stem cells and/or hematopoietic progenitor cells. HSPC can self-renew or can differentiate into (i) myeloid progenitor cells which ultimately give rise to monocytes and macrophages, neutrophils, basophils, eosinophils, erythrocytes, megakaryocytes/platelets, or dendritic cells; or (ii) lymphoid progenitor cells which ultimately give rise to T-cells, B-cells, and lymphocyte-like cells called natural killer cells (NK-cells). For a general discussion of hematopoiesis and HSPC differentiation, see Chapter 17, Differentiated Cells and the Maintenance of Tissues, Alberts et al., 1989, Molecular Biology of the Cell, 2nd Ed., Garland Publishing, New York, NY; Chapter 2 of Regenerative Medicine, Department of Health and Human Services, August 5, 2006, and Chapter 5 of Hematopoietic Stem Cells, 2009, Stem Cell Information, Department of Health and Human Services.

HSPC can be positive for a specific marker expressed in increased levels on HSPC relative to other types of hematopoietic cells. For example, such markers include CD34, CD43, CD45RO, CD45RA, CD59, CD90, CD109, CD117, CD133, CD166, HLA DR, or a combination thereof. Also, the HSPC can be negative for an expressed marker relative to other types of hematopoietic cells. For example, such markers include Lin, CD38, or a combination thereof. Preferably, the HSPC are CD34+ cells.

Sources of HSPC include umbilical cord blood, placental blood, and peripheral blood (see U.S. Patent Nos. 5,004,681; 7,399,633; and 7,147,626; Craddock et al., 1997, Blood 90(12):4779-4788; Jin et al., 2008, Journal of Translational Medicine 6:39; Pelus, 2008, Curr. Opin. Hematol. 15(4):285-292; Papayannopoulou et al., 1998, Blood 91(7):2231-2239; Tricot et al., 2008, Haematologica 93(11):1739-1742; and Weaver et al., 2001, Bone Marrow Transplantation 27(2):S23-S29). Methods regarding collection, anti-coagulation and processing, etc. of blood samples are well known in the art. See, for example, Alsever et al., 1941, N.Y. St. J. Med. 41:126; De Gowin, et al., 1940, J. Am. Med. Ass. 114:850; Smith, et al., 1959, J. Thorac. Cardiovasc. Surg. 38:573; Rous and Turner, 1916, J. Exp. Med. 23:219; and Hum, 1968, Storage of Blood, Academic Press, New York, pp. 26-160. Sources of HSPC also include bone marrow (see Kodo et al., 1984, J. Clin Invest. 73:1377-1384), embryonic cells, aortal-gonadal-mesonephros derived cells, lymph, liver, thymus, and spleen from age-appropriate donors. All collected samples of HSPC can be screened for undesirable components and discarded, treated, or used according to accepted current standards at the time.

HSPC can collected and isolated from a sample using any appropriate technique. Appropriate collection and isolation procedures include magnetic separation; fluorescence activated cell sorting (FACS; Williams et al., 1985, J. Immunol. 135:1004; Lu et al., 1986, Blood 68(1):126-133); affinity chromatography; cytotoxic agents joined to a monoclonal antibody or used in conjunction with a monoclonal antibody, e.g., complement and cytotoxins; "panning" with antibody attached to a solid matrix (Broxmeyer et al., 1984, J. Clin. Invest. 73:939-953); selective agglutination using a lectin such as soybean (Reisner et al., 1980, Proc. Natl. Acad. Sci. U.S.A. 77:1164); etc.

In particular embodiments, a HSPC sample (for example, a fresh cord blood unit) can be processed to select/enrich for CD34+ cells using anti-CD34 antibodies directly or indirectly conjugated to magnetic particles in connection with a magnetic cell separator, for example, the CliniMACS^{®} Cell Separation System (Miltenyi Biotec, Bergisch Gladbach, Germany). See also, sec. 5.4.1.1 of U.S. Patent No. 7,399,633 which describes enrichment of CD34+ HSPC from 1-2% of a normal bone marrow cell population to 50-80% of the population.

Similarly, HSPC expressing CD43, CD45RO, CD45RA, CD59, CD90, CD109, CD117, CD133, CD166, HLA DR, or a combination thereof, can be enriched for using antibodies against these antigens. U.S. Pat. No. 5,877,299 describes additional appropriate hematopoietic antigens that can be used to isolate, collect, and enrich HSPC cells from samples.

Following isolation and/or enrichment, HSPC can be expanded in orderto increase the number of HSPC. Isolation and/or expansion methods are described in, for example, U.S. Patent Nos. 7,399,633 and 5,004,681; U.S. Patent Publication No. 2010/0183564; International Patent Publication Nos. (WO) WO2006/047569; WO2007/095594; WO 2011/127470; and WO 2011/127472; Vamum-Finney et al., 1993, Blood 101:1784-1789; Delaney et al., 2005, Blood 106:2693-2699; Ohishi et al., 2002, J. Clin. Invest. 110:1165-1174; Delaney et al., 2010, Nature Med. 16(2): 232-236; and Chapter 2 of Regenerative Medicine, Department of Health and Human Services, August 2006, and the references cited therein. Each of the referenced methods of collection, isolation, and expansion can be used in particular embodiments of the disclosure.

Preferred methods of expanding HSPC include expansion of HSPC with a Notch agonist. For information regarding expansion of HSPC using Notch agonists, see sec. 5.1 and 5.3 of U.S. Patent No. 7,399,633; U.S. Patent Nos. 5,780,300; 5,648,464; 5,849,869; and 5,856,441; WO 1992/119734; Schlondorfiand Blobel, 1999, J. Cell Sci. 112:3603-3617; Olkkonen and Stenmark, 1997, Int. Rev. Cytol. 176:1-85; Kopan et al., 2009, Cell 137:216-233; Rebay et al., 1991, Cell 67:687-699 and Jarriault et al., 1998, Mol. Cell. Biol. 18:7423-7431. In particular embodiments, the Notch agonist is immobilized during expansion.

Notch agonists include any compound that binds to or otherwise interacts with Notch proteins or other proteins in the Notch pathway such that Notch pathway activity is promoted. Exemplary Notch agonists are the extracellular binding ligands Delta and Serrate (e.g., Jagged), RBP Jxl Suppressor of Hairless, Deltex, Fringe, or fragments thereof which promote Notch pathway activation. Nucleic acid and amino acid sequences of Delta family members and Serrate family members have been isolated from several species and are described in, for example, WO 1993/12141; WO 1996/27610; WO 1997/01571; and Gray et al., 1999, Am. J. Path. 154:785-794.

In particular embodiments, the Notch agonist is Delta1^{ext-IgG}. In particular embodiments, Delta1^{ext-IgG} is applied to a solid phase at a concentration between 0.2 and 20 µg/ml, between 1.25 and 10 µg/ml, or between 2 and 6 µg/ml.

In particular embodiments, during expansion, HSPC are cultured in the presence of a Notch agonist and an aryl hydrocarbon receptor antagonist. The Notch agonist can be immobilized and the aryl hydrocarbon receptor antagonist can be in a fluid contacting the cells.

As is understood by one of ordinary skill in the art, additional culture conditions can include expansion in the presence of one more growth factors, such as: angiopoietin-like proteins (Angptls, e.g., Angptl2, Angptl3, Angptl7, Angpt15, and Mfap4); erythropoietin; fibroblast growth factor-1 (FGF-1); Flt-3 ligand (Flt-3L); granulocyte colony stimulating factor (G-CSF); granulocyte-macrophage colony stimulating factor (GM-CSF); insulin growth factor-2 (IFG-2); interleukin-3 (IL-3); interleukin-6 (IL-6); interleukin-7 (IL-7); interleukin-11 (IL-11); stem cell factor (SCF; also known as the c-kit ligand or mast cell growth factor); thrombopoietin (TPO); and analogs thereof (wherein the analogs include any structural variants of the growth factors having the biological activity of the naturally occurring growth factor; see, e.g., WO 2007/1145227 and U.S. Patent Publication No. 2010/0183564).

In particular embodiments, the amount or concentration of growth factors suitable for expanding HSPC is the amount or concentration effective to promote proliferation of HSPC, but substantially no differentiation of the HSPC. Cell populations are also preferably expanded until a sufficient number of cells are obtained to provide for at least one infusion into a human subject, typically around 10⁴ cells/kg to 10⁹ cells/kg.

**The** amount or concentration of growth factors suitable for expanding HSPC depends on the activity of the growth factor preparation, and the species correspondence between the growth factors and HSPC, etc. Generally, when the growth factor(s) and HSPC are of the same species, the total amount of growth factor in the culture medium ranges from 1 ng/ml to 5 µg/ml, from 5 ng/ml to 1 µg/ml, or from 5 ng/ml to 250 ng/ml. In additional embodiments, the amount of growth factors can be in the range of 5-1000 or 50-100 ng/ml.

In particular embodiments, the foregoing growth factors are present in the culture condition for expanding HSPC at the following concentrations: 25-300 ng/ml SCF, 25-300 ng/ml Flt-3L, 25-100 ng/ml TPO, 25-100 ng/ml IL-6 and 10 ng/ml IL-3. In more specific embodiments, 50, 100, or 200 ng/ml SCF; 50, 100, or 200 ng/ml of Flt-3L; 50 or 100 ng/ml TPO; 50 or 100 ng/ml IL-6; and 10 ng/ml IL-3 can be used.

In particular embodiments, HSPC can be expanded by exposing the HSPC to an immobilized Notch agonist, and 50 ng/ml or 100 ng/ml SCF; to an immobilized Notch agonist, and 50 ng/ml or 100 ng/ml of each of Flt-3L, IL-6, TPO, and SCF; or an immobilized Notch agonist, and 50 ng/ml or 100 ng/ml of each of Flt-3L, IL-6, TPO, and SCF, and 10 ng/ml of IL-11 or IL-3.

HSPC can be expanded in a tissue culture dish onto which an extracellular matrix protein such as fibronectin (FN), or a fragment thereof (e.g., CH-296 (Dao et. al., 1998, Blood 92(12):4612-21)) or RetroNectin^{®} (a recombinant human fibronectin fragment; (Clontech Laboratories, Inc., Madison, WI) is bound.

In a specific embodiment, methods of expanding HSPC include culturing isolated HSPC ex vivo on a solid phase coated with immobilized Delta1^{ext-IgG} and CH-296, and four or more growth factors selected from IL-6, TPO, Flt-3L, CSF, and IL-3; thereby producing an expanded HSPC sample.

In particular embodiments for expanding HSPC, the cells are cultured on a plastic tissue culture dish containing immobilized Delta ligand and fibronectin and 25 ng/ml or 100 ng/ml (or any range in between these values), and preferably 50 ng/ml, of each of SCF and TPO. In particular embodiments for expanding HSPC, the cells are cultured on a plastic tissue culture dish containing immobilized Delta ligand and fibronectin in the presence of and 25 ng/ml or 100 ng/ml (or any range in between these values), and preferably 50 ng/ml of each of SCF and Flt-3L. In particular embodiments for expanding HSPC, the cells are cultured on a plastic tissue culture dish containing immobilized Delta ligand and fibronectin and 25 ng/ml or 100 ng/ml (or any range in between these values), and preferably 50 ng/ml of each of SCF, Flt-3L and TPO. In particular embodiments for expanding HSPC, the cells are cultured on a plastic tissue culture dish containing immobilized Delta ligand and fibronectin and 25 ng/ml or 100 ng/ml (or any range in between these values), and preferably 50 ng/ml, of each of SCF, Flt-3L, TPO, and IL-6. In particular embodiments, the HSPC are cultured further in the presence of 5 to 15 ng/ml, and preferably 10 ng/ml of IL-3. In particular embodiments, the HSPC are cultured further in the presence of 5 to 15 ng/ml, and preferably 10 ng/ml, GM-CSF. In particular embodiments, the one or more growth factors used is not GM-SCF or IL-7. In particular alternative embodiments, fibronectin is excluded from the tissue culture dishes or is replaced by another extracellular matrix protein. Further methods and details regarding expansion of HSPC are found in WO 2013/086436.

In particular embodiments, the percentage of CD34+ cells in the expanded HSPC sample, obtained using the described methods is higher than the percentage of CD34+ cells in the isolated HSPC prior to expansion. For additional information regarding appropriate culturing conditions, see U.S. Patent No. 7,399,633; U.S. Patent Publication No. 2010/0183564; and Freshney Culture of Animal Cells, Wiley-Liss, Inc., New York, NY (1994)).

Modified HSPC. In particular embodiments, HSPC are modified to express molecules having an extracellular component and an intracellular component. The extracellular and intracellular components can be linked directly or through a spacer region, a transmembrane domain, a tag sequence, and/or a linker sequence.

Extracellular Components. Extracellular components include at least one ligand binding domain (hereafter binding domain). The binding domain is designed to target the modified cell to a particularly unwanted cell type by binding a cellular marker that is preferentially found on the unwanted cell type.

Cellular Markers. In particular embodiments, cellular markers are preferentially expressed by unwanted cells, such as unwanted cancer cells. "Preferentially expressed" means that a cellular marker is found at higher levels on an unwanted cell type as compared to other non-targeted cells. The difference in expression level is significant enough that, within sound medical judgment, administration of a cell that will target and kill the unwanted cell based on the presence of the marker outweighs the risk of collateral killing of other non-targeted cells that may also express the marker to a lesser degree. In some instances, a cellular marker is only expressed by the unwanted cell type. In other instances, the cellular marker is expressed on the unwanted cell type at least 25%, 35%, 45%, 55%, 65%, 75%, 85%, 95%, 96%, 97%, 98%, 99%, or 100% more than on non-targeted cells. Exemplary unwanted cancer cells include cancer cells from adrenal cancers, bladder cancers, blood cancers, bone cancers, brain cancers, breast cancers, carcinoma, cervical cancers, colon cancers, colorectal cancers, corpus uterine cancers, ear, nose and throat (ENT) cancers, endometrial cancers, esophageal cancers, gastrointestinal cancers, head and neck cancers, Hodgkin's disease, intestinal cancers, kidney cancers, larynx cancers, leukemias, liver cancers, lymph node cancers, lymphomas, lung cancers, melanomas, mesothelioma, myelomas, nasopharynx cancers, neuroblastomas, non-Hodgkin's lymphoma, oral cancers, ovarian cancers, pancreatic cancers, penile cancers, pharynx cancers, prostate cancers, rectal cancers, sarcoma, seminomas, skin cancers, stomach cancers, teratomas, testicular cancers, thyroid cancers, uterine cancers, vaginal cancers, vascular tumors, and metastases thereof.

The particular following cancers can be targeted by including within an extracellular component a binding domain that binds the associated cellular marker(s):

| Targeted Cancer | Cellular Marker(s) |
|---|---|
| Leukemia/Lymphoma | CD19, CD20, CD22, ROR1, CD33, WT-1 |
| Multiple Myeloma | B-cell maturation antigen (BCMA) |
| Prostate Cancer | PSMA, WT1, Prostate Stem Cell antigen (PSCA), SV40 T |
| Breast Cancer | HER2, ERBB2, ROR1 |
| Stem Cell Cancer | CD133 |
| Ovarian Cancer | L1-CAM, extracellular domain of MUC16 (MUC-CD), folate binding protein (folate receptor), Lewis Y, ROR1, mesothelin, WT-1 |
| Mesothelioma | mesothelin |
| Renal Cell Carcinoma | carboxy-anhydrase-IX (CAIX); |
| Melanoma | GD2 |
| Pancreatic Cancer | mesothelin, CEA, CD24, ROR1 |
| Lung Cancer | ROR1 |

Without limiting the foregoing, cellular markers also include A33; BAGE; Bcl-2; β-catenin; B7H4; BTLA; CA125; CA19-9; CD5; CD19; CD20; CD21; CD22; CD33; CD37; CD44v6; CD45; CD123; CEA; CEACAM6; c-Met; CS-1; cyclin B1; DAGE; EBNA; EGFR; ephrinB2; ErbB2; ErbB3; ErbB4; EphA2; estrogen receptor; FAP; ferritin; α-fetoprotein (AFP); FLT1; FLT4; folate-binding protein; Frizzled; GAGE; G250; GD-2; GHRHR; GHR; GM2; gp75; gp100 (Pmel 17); gp130; HLA; HER-2/neu; HPV E6; HPV E7; hTERT; HVEM; IGF1R; IL6R; KDR; Ki-67; LIFRβ; LRP; LRP5; LTβR; mesothelin; OSMRβ; p53; PD1; PD-L1; PD-L2; PRAME; progesterone receptor; PSA; PSMA; PTCH1; MAGE; MART; mesothelin; MUC; MUC1; MUM-1-B; myc; NYESO-1; RANK; ras; Robo1; RORI; survivin; TCRα; TCRβ; tenascin; TGFBR1; TGFBR2;TLR7; TLR9; TNFR1; TNFR2; TNFRSF4; TWEAK-R; TSTA tyrosinase; VEGF; and WT1.

Particular cancer cell cellular markers include:

| Cancer Antigen | Sequence | SEQ ID NO. |
|---|---|---|
| PSMA | MWNLLHETDSAVATARRPRWLCAGALVLAGGFFLL GFLFGWFIKSSNEATNITPKHNMKAFLDELKAENIKK FLYNFTQIPHLAGTEQNFQLAKQIQSQWKEFGLDSV ELAHYDVLLSYPNKTHPNYISIINEDGNEIFNTSLFEP PPPGYENVSDIVPPFSAFSPQGMPEGDLVYVNYAR TEDFFKLERDMKINCSGKIVIARYGKVFRGNKVKNA QLAGAKGVILYSDPADYFAPGVKSYPDGWNLPGG GVQRGNILNLNGAGDPLTPGYPANEYAYRRGIAEA VGLPSIPVHPIGYYDAQKLLEKMGGSAPPDSSWRG SLKVPYNVGPGFTGNFSTQKVKMHIHSTNEVTRIYN VIGTLRGAVEPDRYVILGGHRDSWVFGGIDPQSGA AVVHEIVRSFGTLKKEGWRPRRTILFASWDAEEFGL LGSTEWAEENSRLLQERGVAYINADSSIEGNYTLRV DCTPLMYSLVHNLTKELKSPDEGFEGKSLYESWTK KSPSPEFSGMPRISKLGSGNDFEVFFQRLGIASGRA RYTKNWETNKFSGYPLYHSVYETYELVEKFYDPMF KYHLTVAQVRGGMVFELANSIVLPFDCRDYAVVLR KYADKIYSISMKHPQEMKTYSVSFDSLFSAVKNFTEI ASKFSERLQDFDKSNPIVLRMMNDQLMFLERAFIDP LGLPDRPFYRHVIYAPSSHNKYAGESFPGIYDALFDI ESKVDPSKAWGEVKRQIYVAAFTVQAAAETLSEVA | 69 |
| PSCA | MKAVLLALLMAGLALQPGTALLCYSCKAQVSNEDC LQVENCTQLGEQCWTARIRAVGLLTVISKGCSLNCV DDSQDYYVGKKNITCCDTDLCNASGAHALQPAAAIL ALLPALGLLLWGPGQL | 72 |
| Mesothelin | | 63 |
| | | |
| CD19 | | 7 |
| CD20 | MTTPRNSVNGTFPAEPMKGPIAMQSGPKPLFRRM SSLVGPTQSFFMRESKTLGAVQIMNGLFHIALGGLL MIPAGIYAPICVTVWYPLWGGIMYIISGSLLAATEKN SRKCLVKGKMIMNSLSLFAAISGMILSIMDILNIKISHF LKMESLNFIRAHTPYINIYNCEPANPSEKNSPSTQYC YSIQSLFLGILSVMLIFAFFQELVIAGIVENEWKRTCS RPKSNIVLLSAEEKKEQTIEIKEEVVGLTETSSQPKN EEDIEIIPIQEEEEEEETETNFPEPPQDQESSPIENDSS P | 11 |
| ROR1 | MHRPRRRGTRPPLLALLAALLLAARGAAAQETELSV SAELVPTSSWNISSELNKDSYLTLDEPMNNITTSLG QTAELHCKVSGNPPPTIRWFKNDAPVVQEPRRLSF RSTIYGSRLRIRNLDTTDTGYFQCVATNGKEVVSST GVLFVKFGPPPTASPGYSDEYEEDGFCQPYRGIAC ARFIGNRTVYMESLHMQGEIENQITAAFTMIGTSSH LSDKCSQFAIPSLCHYAFPYCDETSSVPKPRDLCRD ECEILENVLCQTEYIFARSNPMILMRLKLPNCEDLPQ PESPEAANCIRIGIPMADPINKNHKCYNSTGVDYRG TVSVTKSGRQCQPWNSQYPHTHTFTALRFPELNG GHSYCRNPGNQKEAPWCFTLDENFKSDLCDIPACD SKDSKEKNKMEILYILVPSVAIPLAIALLFFFICVCRNN QKSSSAPVQRQPKHVRGQNVEMSMLNAYKPKSKA KELPLSAVRFMEELGECAFGKIYKGHLYLPGMDHA QLVAIKTLKDYNNPQQWTEFQQEASLMAELHHPNI VCLLGAVTQEQPVCMLFEYINQGDLHEFLIMRSPHS DVGCSSDEDGTVKSSLDHGDFLHIAIQIAAGMEYLS SHFFVHKDLAARNILIGEQLHVKISDLGLSREIYSAD | 84 |
| | YYRVQSKSLLPIRWMPPEAIMYGKFSSDSDIWSFG VVLWEIFSFGLQPYYGFSNQEVIEMVRKRQLLPCSE DCPPRMYSLMTECWNEIPSRRPRFKDIHVRLRSWE GLSSHTSSTTPSGGNATTQTTSLSASPVSNLSNPR YPNYMFPSQGITPQGQIAGFIGPPIPQNQRFIPINGY PIPPGYAAFPAAHYQPTGPPRVIQHCPPPKSRSPSS ASGSTSTGHVTSLPSSGSNQEANIPLLPHMSIPNHP GGMGITVFGNKSQKPYKIDSKQASLLGDANIHGHTE SMISAEL | |
| WT1 | MGHHHHHHHHHHSSGHIEGRHMRRVPGVAPTLVR SASETSEKRPFMCAYPGCNKRYFKLSHLQMHSRK HTGEKPYQCDFKDCERRFFRSDQLKRHQRRHTGV KPFQCKTCQRKFSRSDHLKTHTRTHTGEKPFSCR WPSCQKKFARSDELVRHHNMHQRNMTKLQLAL | 97 |

Unwanted cells and cellular markers are not restricted to cancer cells and cancer cellular markers but can also include for example, virally-infected cells, such as those expressing hepatitis B surface antigen.

Binding Domains. Binding domains include any substance that binds to a cellular marker to form a complex. Examples of binding domains include cellular marker ligands, receptor ligands, antibodies, peptides, peptide aptamers, receptors (e.g., T cell receptors), or combinations thereof.

Antibodies are one example of binding domains and include whole antibodies or binding fragments of an antibody, e.g., Fv, Fab, Fab', F(ab')2, Fc, and single chain (sc) forms and fragments thereof that bind specifically to a cellular marker. Additional examples include scFv-based grababodies and soluble VH domain antibodies. These antibodies form binding regions using only heavy chain variable regions. See, for example, Jespers et al., Nat. Biotechnol. 22:1161, 2004; Cortez-Retamozo et al., Cancer Res. 64:2853, 2004; Baral et al., Nature Med. 12:580, 2006; and Barthelemy et al., J. Biol. Chem. 283:3639, 2008).

Antibodies or antigen binding fragments can include all or a portion of polyclonal antibodies, monoclonal antibodies, human antibodies, humanized antibodies, synthetic antibodies, chimeric antibodies, bispecific antibodies, mini bodies, and linear antibodies.

Antibodies from human origin or humanized antibodies have lowered or no immunogenicity in humans and have a lower number of non-immunogenic epitopes compared to non-human antibodies. Antibodies and their fragments will generally be selected to have a reduced level or no antigenicity in human subjects.

Antibodies that specifically bind a particular cellular marker can be prepared using methods of obtaining monoclonal antibodies, methods of phage display, methods to generate human or humanized antibodies, or methods using a transgenic animal or plant engineered to produce antibodies as is known to those of ordinary skill in the art (see, for example, U.S. Patent Nos. 6,291,161 and 6,291,158). Phage display libraries of partially or fully synthetic antibodies are available and can be screened for an antibody or fragment thereof that can bind to a cellular marker. For example, binding domains may be identified by screening a Fab phage library for Fab fragments that specifically bind to a cellular marker of interest (see Hoet et al., Nat. Biotechnol. 23:344, 2005). Phage display libraries of human antibodies are also available. Additionally, traditional strategies for hybridoma development using a cellular marker of interest as an immunogen in convenient systems (e.g., mice, HuMAb mouse^{®} (GenPharm Int'1. Inc., Mountain View, CA), TC mouse^{®} (Kirin Pharma Co. Ltd., Tokyo, JP), KM-mouse^{®} (Medarex, Inc., Princeton, NJ), llamas, chicken, rats, hamsters, rabbits, etc.) can be used to develop binding domains. In particular embodiments, antibodies specifically bind to a cellular marker preferentially expressed by a particular unwanted cell type and do not cross react with nonspecific components or unrelated targets. Once identified, the amino acid sequence of the antibody and gene sequence encoding the antibody can be isolated and/or determined.

**An** alternative source of binding domains includes sequences that encode random peptide libraries or sequences that encode an engineered diversity of amino acids in loop regions of alternative non-antibody scaffolds, such as scTCR (see, e.g., Lake et al., Int. Immunol.11:745, 1999; Maynard et al., J. Immunol. Methods 306:51, 2005; U.S. Patent No. 8,361,794), fibrinogen domains (see, e.g., Weisel et al., Science 230:1388, 1985), Kunitz domains (see, e.g., U.S. Patent No. 6,423,498), designed ankyrin repeat proteins (DARPins; Binz et al., J. Mol. Biol. 332:489, 2003 and Binz et al., Nat. Biotechnol. 22:575, 2004), fibronectin binding domains (adnectins or monobodies; Richards et al., J. Mol. Biol. 326:1475, 2003; Parker et al., Protein Eng. Des. Selec. 18:435, 2005 and Hackel et al. (2008) J. Mol. Biol. 381:1238-1252), cysteine-knot miniproteins (Vita et al., 1995, Proc. Nat'I. Acad. Sci. (USA) 92:6404-6408; Martin et al., 2002, Nat. Biotechnol. 21:71, 2002 and Huang et al. (2005) Structure 13:755, 2005), tetratricopeptide repeat domains (Main et al., Structure 11:497, 2003 and Cortajarena et al., ACS Chem. Biol. 3:161, 2008), leucine-rich repeat domains (Stumpp et al., J. Mol. Biol. 332:471,2003), lipocalin domains (see, e.g., WO 2006/095164, Beste et al., Proc. Nat'I. Acad. Sci. (USA) 96:1898, 1999 and Schönfeld et al., Proc. Nat'I. Acad. Sci. (USA) 106:8198, 2009), V-like domains (see, e.g., U.S. Patent Application Publication No. 2007/0065431), C-type lectin domains (Zelensky and Gready, FEBS J. 272:6179, 2005; Beavil et al., Proc. Nat'I. Acad. Sci. (USA) 89:753, 1992 and Sato et al., Proc. Nat'I. Acad. Sci. (USA) 100:7779, 2003), mAb2 or Fcab^{™} (see, e.g., WO 2007/098934 and WO 2006/072620), armadillo repeat proteins (see, e.g., Madhurantakam et al., Protein Sci. 21: 1015, 2012; WO 2009/040338), affilin (Ebersbach et al., J. Mol. Biol. 372: 172, 2007), affibody, avimers, knottins, fynomers, atrimers, cytotoxic T-lymphocyte associated protein-4 (Weidle et al., Cancer Gen. Proteo. 10:155, 2013), or the like (Nord et al., Protein Eng. 8:601, 1995; Nord et al., Nat. Biotechnol. 15:772, 1997; Nord et al., Euro. J. Biochem. 268:4269, 2001; Binz et al., Nat. Biotechnol. 23:1257, 2005; Boersma and Plückthun, Curr. Opin. Biotechnol. 22:849, 2011).

In particular embodiments, a binding domain is a single chain T cell receptor (scTCR) including Vα/β and Cα/β chains (e.g., Vα-Cα, Vβ-Cβ, Va-V(B) or including a Vα-Cα, Vβ-Cβ, Vα-Vβ pair specific for a cellular marker of interest (e.g., peptide-MHC complex).

Peptide aptamers include a peptide loop (which is specific for a cellular marker) attached at both ends to a protein scaffold. This double structural constraint increases the binding affinity of peptide aptamers to levels comparable to antibodies. The variable loop length is typically 8 to 20 amino acids and the scaffold can be any protein that is stable, soluble, small, and non-toxic. Peptide aptamer selection can be made using different systems, such as the yeast two-hybrid system (e.g., Gal4 yeast-two-hybrid system), or the LexA interaction trap system.

In particular embodiments, the binding domain can be an antibody that binds the cellular marker CD19. In particular embodiments, a binding domain is a single chain Fv fragment (scFv) that includes VH and VL regions specific for CD19. In particular embodiments, the VH and VL regions are human. Exemplary VH and VL regions include the segments of the anti-CD19 specific monoclonal antibody FMC63. In particular embodiments, the scFV is human or humanized and includes a variable light chain including a CDRL1 sequence of RASQDISKYLN (SEQ ID NO. 108), a CDRL2 sequence of SRLHSGV (SEQ ID NO. 111), and a CDRL3 sequence of GNTLPYTFG (SEQ ID NO. 104). In other embodiments, the scFV is a human or humanized ScFv including a variable heavy chain including a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).

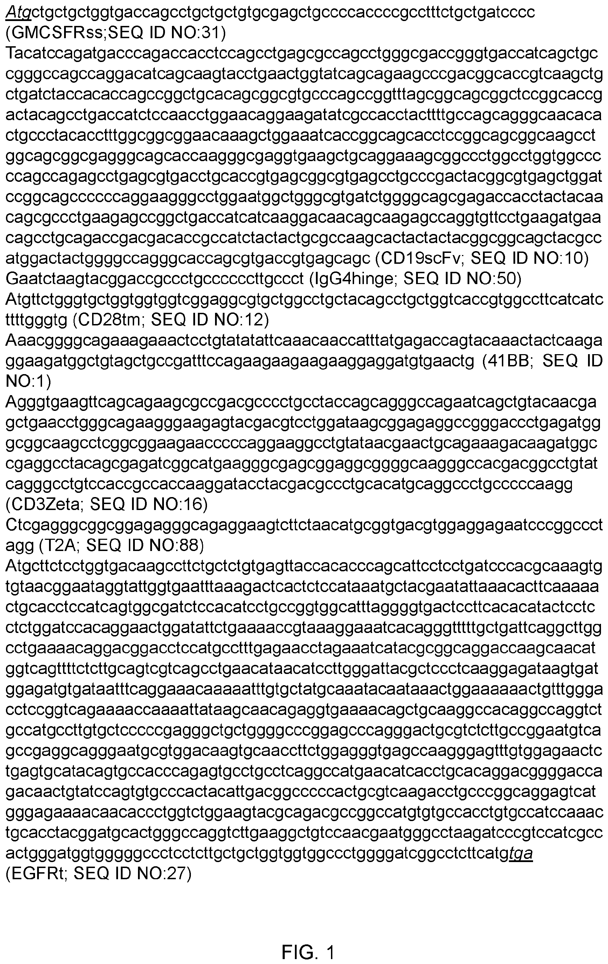

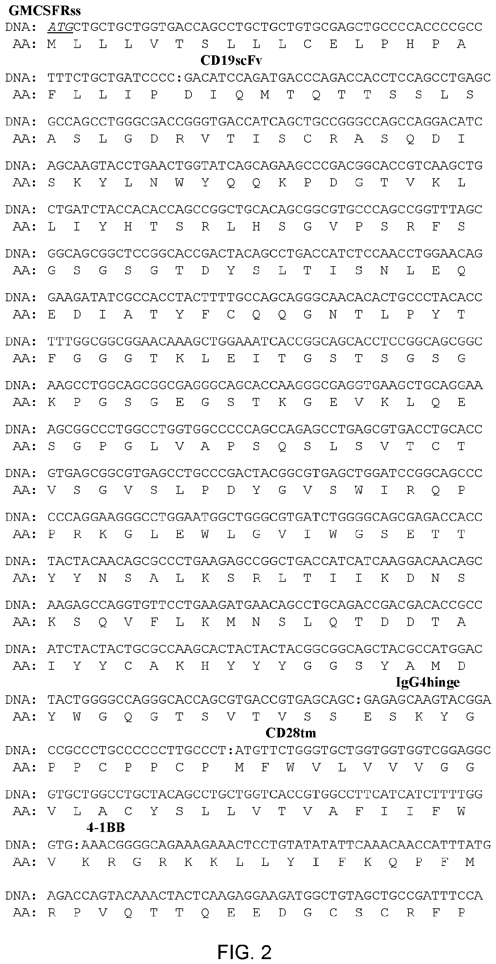

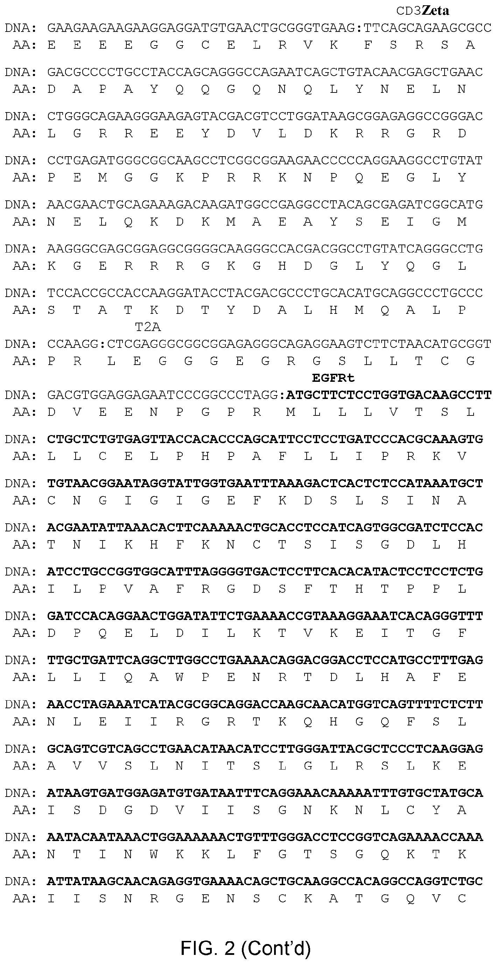

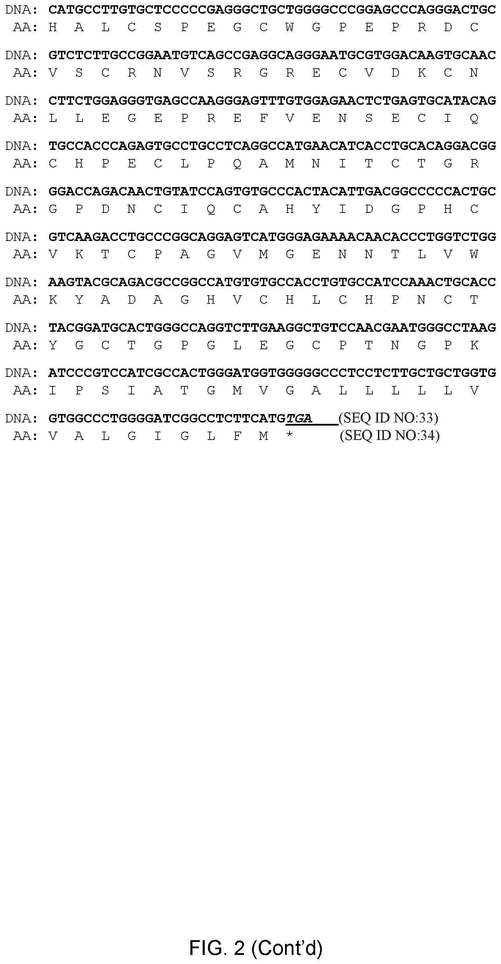

A gene sequence encoding a binding domain is shown in FIG. 1 as the scFv from an antibody that specifically binds CD19, such as FMC63. A gene sequence encoding a flexible linker including the amino acids GSTSGSGKPGSGEGSTKG (SEQ ID NO:30) separates the VH and VL chains in the scFV. The amino acid sequence of the scFv including the linker is shown in FIG. 2 (SEQ ID NO:34). Other CD19-targeting antibodies such as SJ25C1 (Bejcek et al. Cancer Res 2005, PMID 7538901) and HD37 (Pezutto et al. JI 1987, PMID 2437199) are known. SEQ ID NO.10 provides the anti-CD19 scFv (VH-VL) DNA sequence and SEQ ID NO.9 provides the anti-CD19 scFv (VH-VL) amino acid sequence.

In particular embodiments, the binding domain binds the cellular marker ROR1. In particular embodiments, the scFV is a human or humanized scFv including a variable light chain including a CDRL1 sequence of ASGFDFSAYYM (SEQ ID NO. 101), a CDRL2 sequence of TIYPSSG (SEQ ID NO. 112), and a CDRL3 sequence of ADRATYFCA (SEQ ID NO. 100). In particular embodiments, the scFV is a human or humanized scFv including a variable heavy chain including a CDRH1 sequence of DTIDWY (SEQ ID NO. 102), a CDRH2 sequence of VQSDGSYTKRPGVPDR (SEQ ID NO. 113), and a CDRH3 sequence of YIGGYVFG (SEQ ID NO. 117).

In particular embodiments, the binding domain binds the cellular marker ROR1. In particular embodiments, the scFV is a human or humanized scFv including a variable light chain including a CDRL1 sequence of SGSDINDYPIS (SEQ ID NO. 109), a CDRL2 sequence of INSGGST (SEQ ID NO. 105), and a CDRL3 sequence of YFCARGYS (SEQ ID NO. 116). In particular embodiments, the scFV is a human or humanized ScFv including a variable heavy chain including a CDRH1 sequence of SNLAW (SEQ ID NO. 110), a CDRH2 sequence of RASNLASGVPSRFSGS (SEQ ID NO. 107), and a CDRH3 sequence of NVSYRTSF (SEQ ID NO. 106). A number of additional antibodies specific for ROR1 are known to those of skill in the art.

In particular embodiments, the binding domain binds the cellular marker Her2. A number of antibodies specific for Her2 are known to those of skill in the art and can be readily characterized for sequence, epitope binding, and affinity. In particular embodiments, the binding domain includes a scFV sequence from the Herceptin antibody. In particular embodiments, the binding domain includes a human or humanized ScFv including a variable light chain including a CDRL1 sequence, a CDRL2 sequence and a CDRL3 sequence of the Herceptin antibody. In particular embodiments, the scFV is a human or humanized ScFv including a variable heavy chain including a CDRH1 sequence, a CDRH2 sequence, and a CDRH3 sequence of the Herceptin antibody. The CDR sequences can readily be determined from the amino acid sequence of Herceptin. An exemplary gene sequence encoding a Her2 ligand binding domain is found in SEQ ID NOs: 39 and 40.

In particular embodiments, CDR regions are found within antibody regions as numbered by Kabat as follows: for the light chain: CDRL1 are amino acids 24-34;CDRL2 are amino acids 50-56; CDRL3 are amino acids 89-97 and for the heavy chain: CDRH1 are amino acids 31-35; CDRH2 are amino acids 50-65; and CDRH3 are amino acids 95-102.

Other antibodies are well-known and commercially available. For example, anti-PSMA and anti-PSCA antibodies are available from Abcam plc (ab66912 and ab15168, respectively). Mesothelin and WT1 antibodies are available from Santa Cruz Biotechnology, Inc. Anti-CD20 antibodies, such as rituximab (trade names Rituxan, MabThera and Zytux), have been developed by IDEC Pharmaceuticals.

Intracellular Components. Intracellular components of expressed molecules can include effector domains. Effector domains are capable of transmitting functional signals to a cell. In particular embodiments, an effector domain will directly or indirectly promote a cellular response by associating with one or more other proteins that directly promote a cellular response. Effector domains can provide for activation of at least one function of a modified cell upon binding to the cellular marker expressed on an unwanted cell. Activation of the modified cell can include one or more of differentiation, proliferation and/or activation or other effector functions.

An effector domain can include one, two, three or more receptor signaling domains, intracellular signaling domains (e.g., cytoplasmic signaling sequences), costimulatory domains, or combinations thereof. Exemplary effector domains include signaling and stimulatory domains selected from: 4-1BB, CARD11, CD3 gamma, CD3 delta, CD3 epsilon, CD3ζ, CD27, CD28, CD79A, CD79B, DAP10, FcRa, FcRβ, FcRy, Fyn, HVEM, ICOS, LAG3, LAT, Lck, LRP, NKG2D, NOTCH1, pTα, PTCH2, OX40, ROR2, Ryk, SLAMF1, Slp76, TCRα, TCRβ, TRIM, Wnt, Zap70, or any combination thereof.

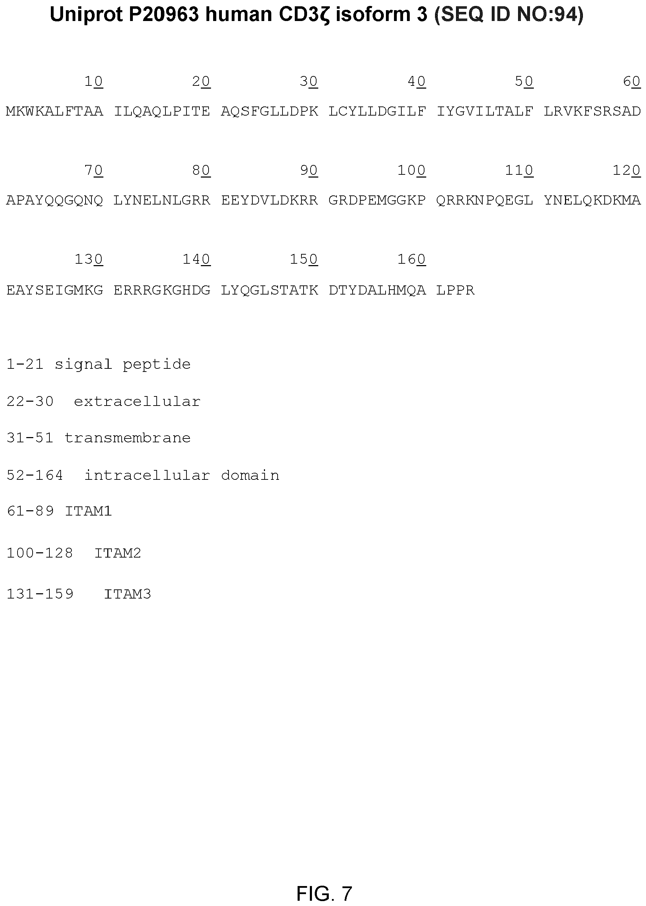

Primary cytoplasmic signaling sequences that act in a stimulatory manner may contain signaling motifs which are known as receptor tyrosine-based activation motifs or iTAMs. Examples of iTAM containing primary cytoplasmic signaling sequences include those derived from CD3γ, CD3δ, CD3ε, CD3ζ, CD5, CD22, CD66d, CD79a, CD79b, and FeR gamma. In particular embodiments, variants of CD3ζ retain at least one, two, three, or all ITAM regions as shown in FIG. 7.

In particular embodiments, an effector domain includes a cytoplasmic portion that associates with a cytoplasmic signaling protein, wherein the cytoplasmic signaling protein is a lymphocyte receptor or signaling domain thereof, a protein including a plurality of ITAMs, a costimulatory domain, or any combination thereof.

Examples of intracellular signaling domains include the cytoplasmic sequences of the CD3ζ chain, and/or co- receptors that act in concert to initiate signal transduction following binding domain engagement.

In particular embodiments, an intracellular signaling domain of a molecule expressed by a modified cell can be designed to include an intracellular signaling domain combined with any other desired cytoplasmic domain(s). For example, the intracellular signaling domain of a molecule can include an intracellular signaling domain and a costimulatory domain, such as a costimulatory signaling region.

The costimulatory signaling region refers to a portion of the molecule including the intracellular domain of a costimulatory domain. A costimulatory domain is a cell surface molecule other than the expressed cellular marker binding domain that can be required for a lymphocyte response to cellular marker binding. Examples of such molecules include CD27, CD28, 4-1BB (CD 137), OX40, CD30, CD40, lymphocyte function-associated antigen-1 (LFA-1), CD2, CD7, LIGHT, NKG2C, B7-H3, and a ligand that specifically binds with CD83.

In particular embodiments, the amino acid sequence of the intracellular signaling domain including a variant of CD3ζ and a portion of the 4-1BB intracellular signaling domain as provided in FIG. 2. A representative gene sequence is provided in FIG. 1 (SEQ ID NO:16; SEQ ID NO:1).

In particular embodiments, the intracellular signaling domain includes (i) all or a portion of the signaling domain of CD3ζ, (ii) all or a portion of the signaling domain of CD28, (iii) all or a portion of the signaling domain of 4-1BB, or (iv) all or a portion of the signaling domain of CD3ζ, CD28 and/or 4-1BB.

The intracellular signaling domain sequences of the expressed molecule can be linked to each other in a random or specified order. Optionally, a short oligo- or protein linker, preferably between 2 and 10 amino acids in length may form the linkage.

Spacer Regions. In particular embodiments, a spacer region is found between the binding domain and intracellular component of an expressed molecule. In particular embodiments, the spacer region is part of the extracellular component of an expressed molecule.

The length of a spacer region can be customized for individual cellular markers on unwanted cells to optimize unwanted cell recognition and destruction. In particular embodiments, a spacer region length can be selected based upon the location of a cellular marker epitope, affinity of a binding domain for the epitope, and/or the ability of the modified cells expressing the molecule to proliferate in vitro and/or in vivo in response to cellular marker recognition.

Typically a spacer region is found between the binding domain and a transmembrane domain of an expressed molecule. Spacer regions can provide for flexibility of the binding domain and allow for high expression levels in modified cells. In particular embodiments, a spacer region can have at least 10 to 250 amino acids, at least 10 to 200 amino acids, at least 10 to 150 amino acids, at least 10 to 100 amino acids, at least 10 to 50 amino acids, or at least 10 to 25 amino acids. In further embodiments, a spacer region has 250 amino acids or less; 200 amino acids or less, 150 amino acids or less; 100 amino acids or less; 50 amino acids or less; 40 amino acids or less; 30 amino acids or less; 20 amino acids or less; or 10 amino acids or less.

In particular embodiments, spacer regions can be derived from a hinge region of an immunoglobulin like molecule, for example all or a portion of the hinge region from a human IgG1, IgG2, IgG3, or IgG4. Hinge regions can be modified to avoid undesirable structural interactions such as dimerization. In particular embodiments, all or a portion of a hinge region can be combined with one or more domains of a constant region of an immunoglobulin. For example, a portion of a hinge region can be combined with all or a portion of a CH2 or CH3 domain. In particular embodiments, the spacer region does not include the 47-48 amino acid hinge region sequence from CD8α.

In particular embodiments, the spacer region is selected from the group including a hinge region sequence from IgG1, IgG2, IgG3, or IgG4 in combination with all or a portion of a CH2 region; all or a portion of a CH3 region; or all or a portion of a CH2 region and all or a portion of a CH3 region.

In particular embodiments, a short spacer region has 12 amino acids or less and includes all or a portion of a IgG4 hinge region sequence (e.g., the protein encoded by SEQ ID NO:50), an intermediate spacer region has 119 amino acids or less and includes all or a portion of a IgG4 hinge region sequence and a CH3 region (e.g., SEQ ID NO:52), and a long spacer has 229 amino acids or less and includes all or a portion of a IgG4 hinge region sequence, a CH2 region, and a CH3 region (e.g., SEQ ID NO:50).

In particular embodiments, when a binding domain binds to a portion of a cellular marker that is very proximal to the unwanted cell's membrane, a long spacer (e.g. 229 amino acids or less and greater than 119 amino acids) is selected. Very proximal to the unwanted cell's membrane means within the first 100 extracellular amino acids of a cellular marker.

In particular embodiments, when a binding domain binds to a portion of a cellular marker that is distal to the unwanted cell's membrane, an intermediate or short spacer is selected (e.g. 119 amino acids or less or 12 amino acids or less).

As is understood by one of ordinary skill in the art, whether a binding portion of a cellular marker is proximal or distal to a membrane can also be determined by modeling three dimensional structures or based on analysis of crystal structure.

In a particular embodiment, an expressed molecule includes a binding domain including a scFV that binds to a ROR1 epitope located in the membrane distal to the Ig/Frizzled domain and a spacer that is 15 amino acids or less. In particular embodiments, an expressed molecule includes a binding domain including an scFV that binds a ROR1 epitope located in the membrane proximal to the Kringle domain and a spacer that is longer than 15 amino acids. In particular embodiments an expressed molecule includes a binding domain including a scFV that binds CD19 and a spacer that is 15 amino acids or less.

In particular embodiments, when the binding domain includes (i) a variable light chain including a CDRL1 sequence of RASQDISKYLN (SEQ ID NO: 108), a CDRL2 sequence of SRLHSGV (SEQ ID NO: 111), and a CDRL3 sequence of GNTLPYTFG (SEQ ID NO: 104) and a variable heavy chain including a CDRH1 sequence of DYGVS (SEQ ID NO: 103), a CDRH2 sequence of VTWGSETTYYNSALKS (SEQ ID NO: 114), and a CDRH3 sequence of YAMDYWG (SEQ ID NO: 115), or (ii) a variable light chain including a CDRL1 sequence of ASGFDFSAYYM (SEQ ID NO: 101), a CDRL2 sequence of TIYPSSG (SEQ ID NO: 112), and a CDRL3 sequence of ADRATYFCA (SEQ ID NO: 100), and a variable heavy chain including a CDRH1 sequence of DTIDWY (SEQ ID NO: 102), a CDRH2 sequence of VQSDGSYTKRPGVPDR (SEQ ID NO: 113), and a CDRH3 sequence of YIGGYVFG (SEQ ID NO: 117), the spacer can be 12 amino acid or less and, in a more particular embodiment can include SEQ ID NO:47.

In particular embodiments, when the binding domain includes (i) a variable light chain including a CDRL1 sequence of SGSDINDYPIS (SEQ ID NO: 109), a CDRL2 sequence of INSGGST (SEQ ID NO: 105), and a CDRL3 sequence of YFCARGYS (SEQ ID NO: 116), and a variable heavy chain including a CDRH1 sequence of SNLAW (SEQ ID NO: 110), a CDRH2 sequence of RASNLASGVPSRFSGS (SEQ ID NO: 107), and a CDRH3 sequence of NVSYRTSF (SEQ ID NO: 106), or (ii) a variable light chain including a CDRL1 sequence, a CDRL2 sequence and a CDRL3 sequence of the Herceptin antibody and a variable heavy chain including a CDRH1 sequence, a CDRH2, and a CDRH3 sequence of the Herceptin antibody, the spacer can be 229 amino acid or less and, in a more particular embodiment can include SEQ ID NO:61.

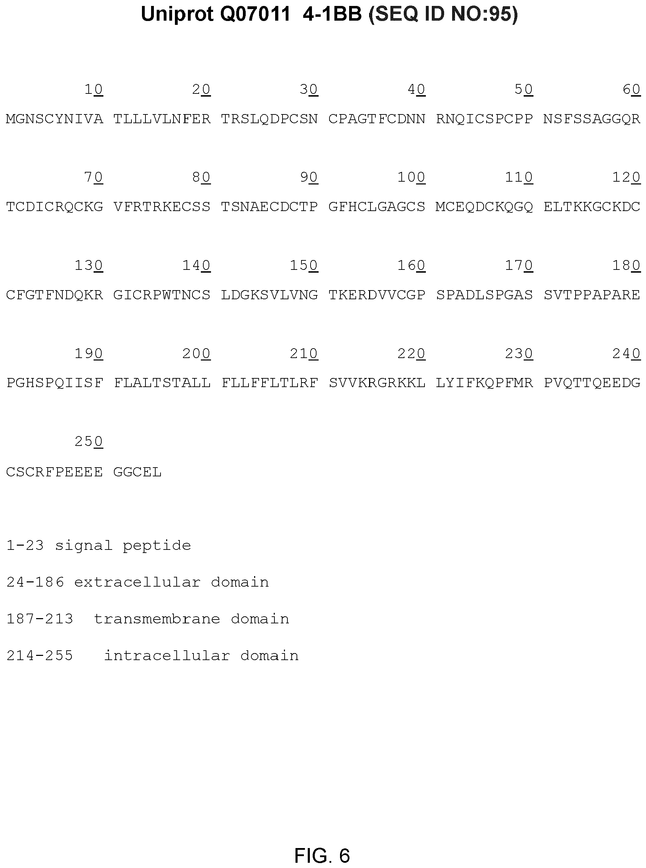

Transmembrane Domains. Expressed molecules disclosed herein can also include a transmembrane domain, at least a portion of which is located between the extracellular component and the intracellular component. The transmembrane domain can anchor the expressed molecule in the modified cell's membrane. The transmembrane domain can be derived either from a natural and/or a synthetic source. When the source is natural, the transmembrane domain can be derived from any membrane-bound or transmembrane protein. Transmembrane domains can include at least the transmembrane region(s) of the alpha, beta or zeta chain of a T-cell receptor, CD28, CD3, CD45, CD4, CD5, CD9, CD16, CD22; CD33, CD37, CD64, CD80, CD86, CD134, CD137 and CD154. Transmembrane domains can include those shown in FIG. 2 or FIG. 6.

In particular embodiments, the transmembrane domain includes the amino acid sequence of the CD28 transmembrane domain as shown in FIG. 2 or the amino acid sequence of the CD4 transmembrane domain. A representative gene sequence encoding the CD28 transmembrane domain is shown in FIG. 1 (SEQ ID NO:12). SEQ ID NO:118 is a representative gene sequence encoding the CD4 transmembrane domain.

Tag Sequences. In particular embodiments, the expressed molecule further includes a tag sequence. A tag sequence can provide for identification and/or selection of transduced cells. A number of different tag sequences can be employed. Positive selectable tag sequences may be encoded by a gene, which upon being introduced into the modified cell, expresses a dominant phenotype permitting positive selection of cells carrying the gene. Genes of this type are known in the art, and include, hygromycin-B phosphotransferase gene (hph) which confers resistance to hygromycin B, the amino glycoside phosphotransferase gene (neo or aph) from Tn5 which codes for resistance to the antibiotic 0418, the dihydrofolate reductase (DHFR) gene, the adenosine deaminase gene (ADA), and the multi-drug resistance (MDR) gene. In particular embodiments, the tag sequence is a truncated EGFR as shown in FIG. 2. An exemplary gene sequence encoding the truncated EGFR is shown in FIG. 1. (SEQ ID NO:9).

In particular embodiments, functional genes can be introduced into the modified HSPC to allow for negative selection in vivo. "Negative selection" means that an administered cell can be eliminated as a result of a change in the in vivo condition of a subject. The negative selectable phenotype can result from the insertion of a gene that confers sensitivity to an administered agent. Negative selectable genes are known in the art, and include: the Herpes simplex virus type I thymidine kinase (HSV-I TK) gene which confers ganciclovir sensitivity; the cellular hypoxanthine phosphribosyltransferase (HPRT) gene, the cellular adenine phosphoribosyltransferase (APRT) gene, and bacterial cytosine deaminase. For additional supporting disclosure regarding negative selection, see Lupton S.D. et. al., Mol. and Cell Biol., 11:6 (1991); Riddell et al., Human Gene Therapy 3:319-338 (1992); WO 1992/008796 and WO 1994/028143 and U.S. Patent No. 6,040,177 at columns 14-17).

The design of particular molecules to be expressed by the modified cells can be customized depending on the type of targeted cellular marker, the affinity of the binding domain for the cellular marker, the flexibility needed for the cellular marker binding domain, and/or the intracellular signaling domain. In particular embodiments, a number of constructs are tested in vitro and in in vivo models to determine the ability of modified cells to expand in culture and/or kill unwanted cells. In particular embodiments, a molecule is selected that provides for capability of at least 30% of modified-effectors (e.g., differentiated modified HSPC) to proliferate through at least two generations in vitro and/or within 72 hours after introduction in vivo. In particular embodiments, a molecule is not selected that results in greater than 50% of the cells undergoing activation induced cell death (AICD) within 72 hours in vivo in immunodeficient mice, and fails to reduce presence of tumor cells.

The following disclosure provides more particular examples of expressed molecules and associated vectors.

"Chimeric antigen receptor" or "CAR" refer to a synthetically designed receptor including a binding domain that binds to a cellular marker preferentially associated with an unwanted cell that is linked to an effector domain. The binding domain and effector domain can be linked via a spacer domain, transmembrane domain, tag sequence, and/or linker sequence.

In particular embodiments, ROR1-specific and CD19-specific CARs can be constructed using VL and VH chain segments of the 2A2, R12, and R11 mAhs (ROR1) and FMC63 mAb (CD19). Variable region sequences for R11 and R12 are provided in Yang et al, Plos One 6(6):e21018, June 15, 2011. Each scFV can be linked by a (G₄S)₃ (SEQ ID NO:60) protein to a spacer domain derived from IgG4-Fc (Uniprot Database: P0186I, SEQ ID NO:92) including either 'Hinge-CH2-CH3' (229 AA, SEQ ID NO:61), 'Hinge-CH3' (119 AA, SEQ ID NO: 52) or'Hinge' only (12 AA, SEQ. ID NO:47) sequences (FIG. 1). All spacers can contain a S→P substitution within the 'Hinge' domain located at position 108 of the native IgG4-Fc protein, and can be linked to the 27 AA transmembrane domain of human CD28 (Uniprot: P10747, SEQ ID NO:93) and to an effector domain signaling module including either (i) the 41 AA cytoplasmic domain of human CD28 with an LL→GG substitution located at positions 186-187 of the native CD28 protein (SEQ ID NO:93) or (ii) the 42 AA cytoplasmic domain of human 4-1BB (Uniprot: Q07011, SEQ ID NO: 95), each of which can be linked to the 112 AA cytoplasmic domain of isoform 3 of human CD3ζ (Uniprot: P20963, SEQ ID NO:94). The construct encodes a T2A ribosomal skip element (SEQ ID NO:88)) and a tEGFR sequence (SEQ ID NO:27) downstream of the chimeric receptor. Codon-optimized gene sequences encoding each transgene can be synthesized (Life Technologies) and cloned into the epHIV7 lentiviral vector using Nhel and Not1 restriction sites. The epHIV7 lentiviral vector can be derived from the pHIV7 vector by replacing the cytomegalovirus promoter of pHIV7 with an EF-1 promoter. ROR1-chimeric receptor, CD19-chimeric receptor ortEGFR-encoding lentiviruses can be produced in 293T cells using the packaging vectors pCHGP-2, pCMV-Rev2 and pCMV-G, and Calphos^{®} transfection reagent (Clontech).

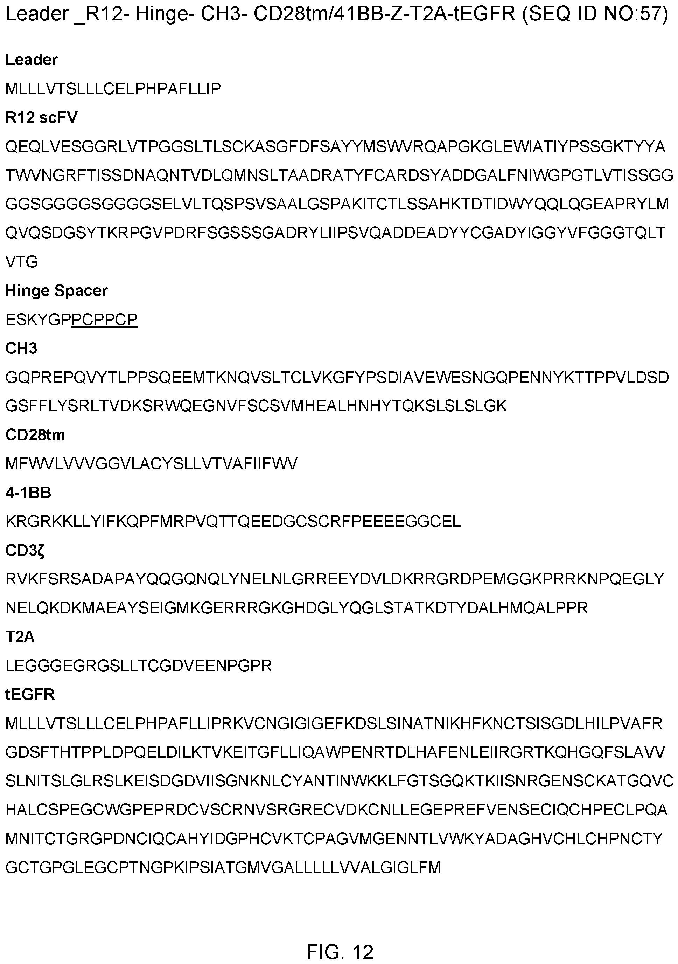

HER2-specific chimeric receptors can be constructed using VL and VH chain segments of a HER2-specific mAb that recognizes a membrane proximal epitope on HER2 (FIG. 12A), and the scFVs can be linked to IgG4 hinge/CH2/CH3, IgG4 hinge/CH3, and IgG4 hinge only extracellular spacer domains and to the CD28 transmembrane domain, 4-1BB and CD3ζ signaling domains (FIG. 12B).

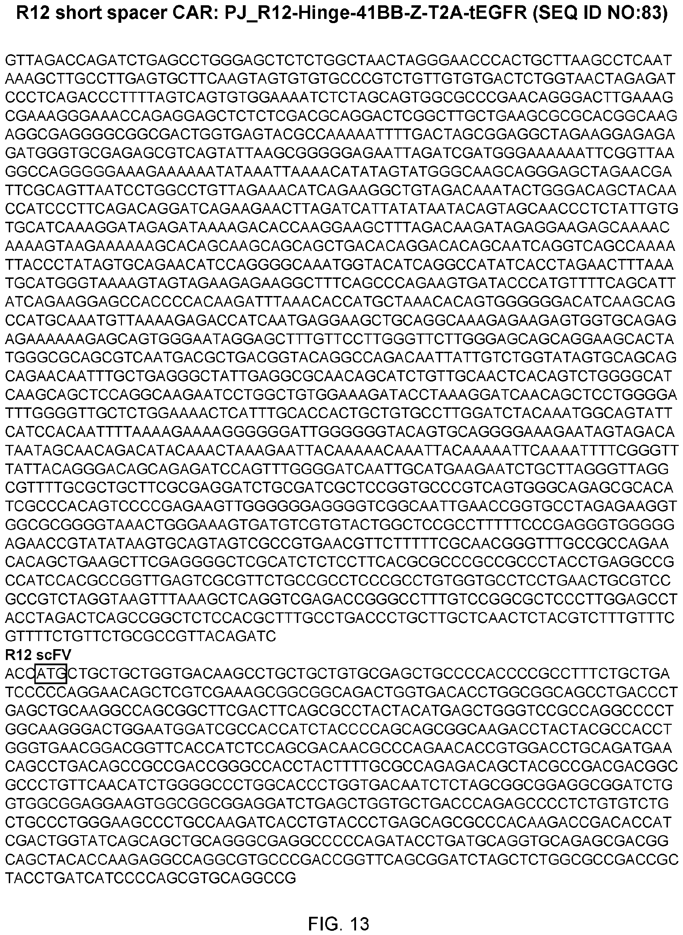

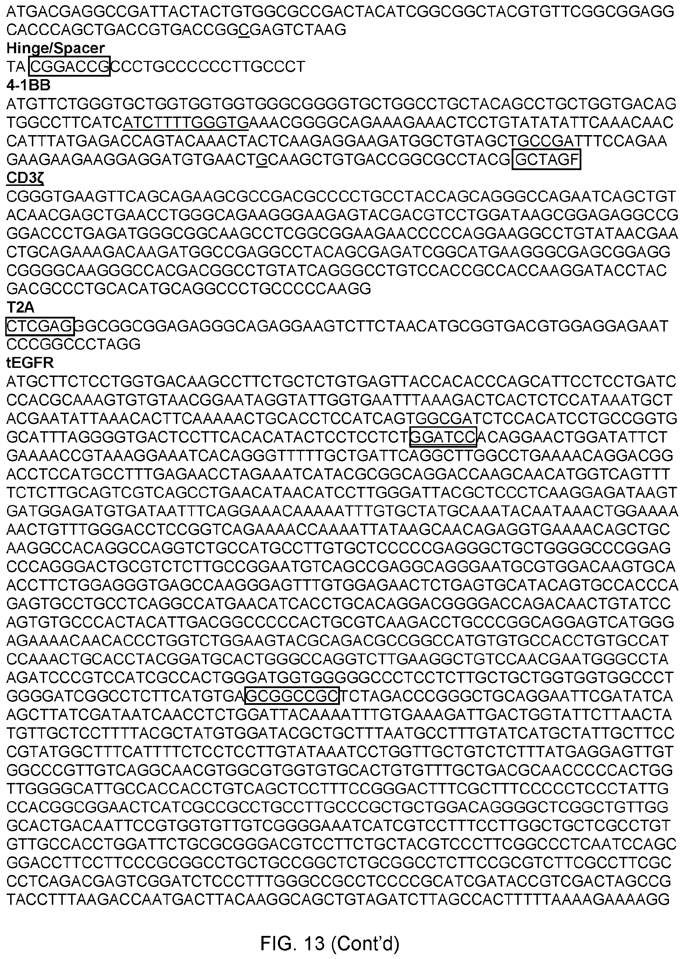

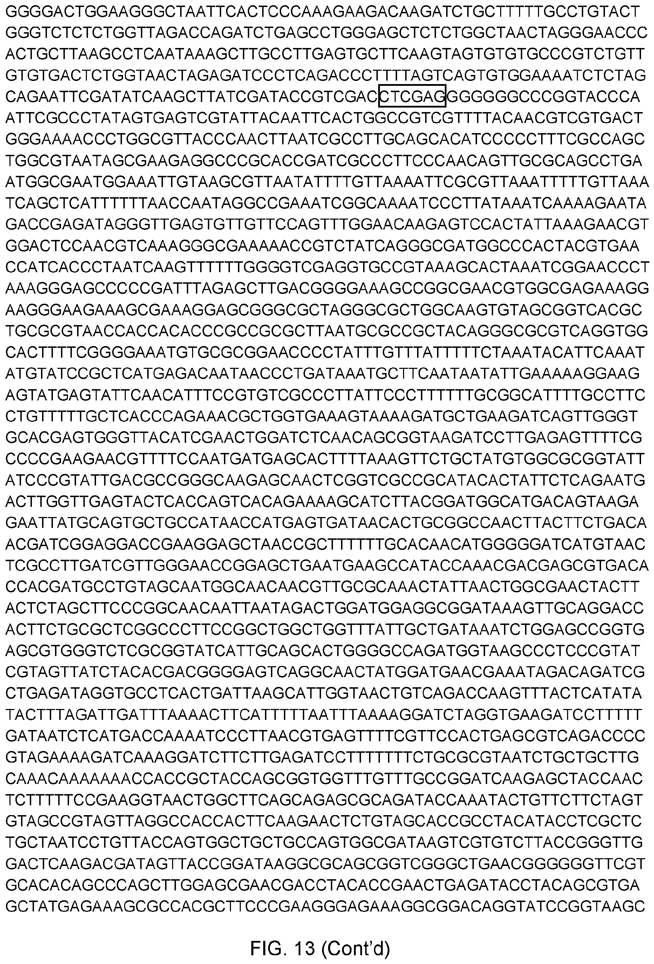

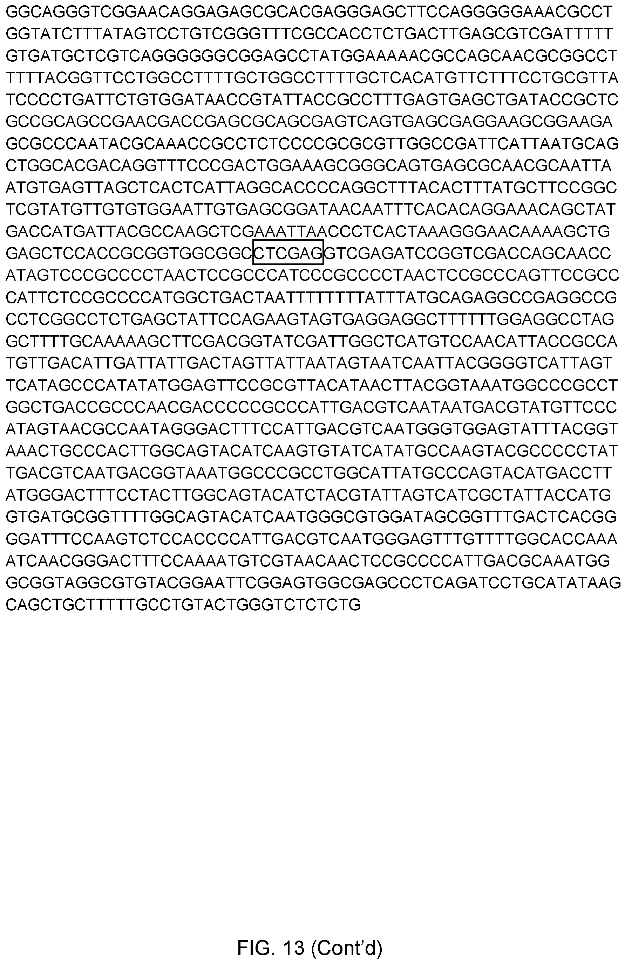

As indicated, each CD19 chimeric receptor can include a single chain variable fragment corresponding to the sequence of the CD19-specific mAb FMC63 (scFv: VL-VH), a spacer derived from IgG4-Fc including either the 'Hinge-CH2-CH3' domain (229 AA, long spacer) or the 'Hinge' domain only (12 AA, short spacer), and a signaling module of CD3ζ with membrane proximal CD28 or 4-1BB costimulatory domains, either alone or in tandem (FIG. 13A). The transgene cassette can include a truncated EGFR (tEGFR) downstream from the chimeric receptor gene and be separated by a cleavable T2A element, to serve as a tag sequence for transduction, selection and in vivo tracking for chimeric receptor-modified cells.

As is understood by one of ordinary skill in the art, modified HSPC can be made recombinant by the introduction of a recombinant gene sequence into the HSPC. A description of genetically engineered HSPC can be found in sec. 5.1 of U.S. Patent No. 7,399,633. A gene whose expression is desired in the modified cell is introduced into the HSPC such that it is expressible by the cells and/or their progeny.

Desired genes can be introduced into HSPC by any method known in the art, including transfection, electroporation, microinjection, lipofection, calcium phosphate mediated transfection, infection with a viral or bacteriophage vector containing the gene sequences, cell fusion, chromosome-mediated gene transfer, microcell-mediated gene transfer, sheroplast fusion, etc. Numerous techniques are known in the art for the introduction of foreign genes into cells (see e.g., Loeffler and Behr, 1993, Meth. Enzymol. 217:599-618; Cohen et al., 1993, Meth. Enzymol. 217:618-644; Cline, 1985, Pharmac. Ther. 29:69-92) and may be used, provided that the necessary developmental and physiological functions of the recipient cells are not disrupted. The technique should provide for the stable transfer of the gene to the cell, so that the gene is expressible by the cell and preferably heritable and expressible by its cell progeny. As indicated, in particular embodiments, the method of transfer includes the transfer of a selectable tag sequence to the cells. The cells are then placed under selection to isolate those cells that have taken up and are expressing the transferred gene.

The term "gene" refers to a nucleic acid sequence (used interchangeably with polynucleotide or nucleotide sequence) that encodes a molecule having an extracellular component and an intracellular component as described herein. This definition includes various sequence polymorphisms, mutations, and/or sequence variants wherein such alterations do not substantially affect the function of the encoded molecule. The term "gene" may include not only coding sequences but also regulatory regions such as promoters, enhancers, and termination regions. The term further can include all introns and other DNA sequences spliced from the mRNA transcript, along with variants resulting from alternative splice sites. Gene sequences encoding the molecule can be DNA or RNA that directs the expression of the molecule. These nucleic acid sequences may be a DNA strand sequence that is transcribed into RNA or an RNA sequence that is translated into protein. The nucleic acid sequences include both the full-length nucleic acid sequences as well as non-full-length sequences derived from the full-length protein. The sequences can also include degenerate codons of the native sequence or sequences that may be introduced to provide codon preference in a specific cell type. Portions of complete gene sequences are referenced throughout the disclosure as is understood by one of ordinary skill in the art.

A gene sequence encoding a binding domain, effector domain, spacer region, transmembrane domain, tag sequence, linker sequence, or any other protein or peptide sequence described herein can be readily prepared by synthetic or recombinant methods from the relevant amino acid sequence. In embodiments, the gene sequence encoding any of these sequences can also have one or more restriction enzyme sites at the 5' and/or 3' ends of the coding sequence in order to provide for easy excision and replacement of the gene sequence encoding the sequence with another gene sequence encoding a different sequence. In embodiments, the gene sequence encoding the sequences can be codon optimized for expression in mammalian cells.

"Encoding" refers to the property of specific sequences of nucleotides in a gene, such as a cDNA, or an mRNA, to serve as templates for synthesis of other macromolecules such as a defined sequences of amino acids. Thus, a gene codes for a protein if transcription and translation of mRNA corresponding to that gene produces the protein in a cell or other biological system. A "gene sequence encoding a protein" includes all nucleotide sequences that are degenerate versions of each other and that code for the same amino acid sequence or amino acid sequences of substantially similar form and function.

Polynucleotide gene sequences encoding more than one portion of an expressed molecule can be operably linked to each other and relevant regulatory sequences. For example, there can be a functional linkage between a regulatory sequence and a heterologous nucleic acid sequence resulting in expression of the latter. For another example, a first nucleic acid sequence can be operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary or helpful, join coding regions, into the same reading frame.

Retroviral vectors (see Miller et al., 1993, Meth. Enzymol. 217:581-599) can be used. In such embodiments, the gene to be expressed is cloned into the retroviral vector for its delivery into HSPC. In particular embodiments, a retroviral vector contains all of the cis-acting sequences necessary for the packaging and integration of the viral genome, i.e., (a) a long terminal repeat (LTR), or portions thereof, at each end of the vector; (b) primer binding sites for negative and positive strand DNA synthesis; and (c) a packaging signal, necessary for the incorporation of genomic RNA into virions. More detail about retroviral vectors can be found in Boesen et al., 1994, Biotherapy 6:291-302; Clowes et al., 1994, J. Clin. Invest. 93:644-651; Kiem et al., 1994, Blood 83:1467-1473; Salmons and Gunzberg, 1993, Human Gene Therapy 4:129-141; and Grossman and Wilson, 1993, Curr. Opin. in Genetics and Devel. 3:110-114. Adenoviruses, adena-associated viruses (AAV) and alphaviruses can also be used. See Kozarsky and Wilson, 1993, Current Opinion in Genetics and Development 3:499-503, Rosenfeld et al., 1991, Science 252:431-434; Rosenfeld et al., 1992, Cell 68:143-155; Mastrangeli et al., 1993, J. Clin. Invest. 91:225-234; Walsh et al., 1993, Proc. Soc. Exp. Bioi. Med. 204:289-300; and Lundstrom, 1999, J. Recept. Signal Transduct. Res. 19: 673-686. Other methods of gene delivery include the use of mammalian artificial chromosomes (Vos, 1998, Curr. Op. Genet. Dev. 8:351-359); liposomes (Tarahovsky and Ivanitsky, 1998, Biochemistry (Mosc) 63:607-618); ribozymes (Branch and Klotman, 1998, Exp. Nephrol. 6:78-83); and triplex DNA (Chan and Glazer, 1997, J. Mol. Med. 75:267-282).

Additional embodiments include sequences having 70% sequence identity; 80% sequence identity; 81% sequence identity; 82% sequence identity; 83% sequence identity; 84% sequence identity; 85% sequence identity; 86% sequence identity; 87% sequence identity; 88% sequence identity; 89% sequence identity; 90% sequence identity; 91% sequence identity; 92% sequence identity; 93% sequence identity; 94% sequence identity; 95% sequence identity; 96% sequence identity; 97% sequence identity; 98% sequence identity; or 99% sequence identity to any gene, protein or peptide sequence disclosed herein.

**"%** sequence identity" refers to a relationship between two or more sequences, as determined by comparing the sequences. In the art, "identity" also means the degree of sequence relatedness between protein sequences as determined by the match between strings of such sequences. "Identity" (often referred to as "similarity") can be readily calculated by known methods, including those described in: Computational Molecular Biology (Lesk, A. M., ed.) Oxford University Press, NY (1988); Biocomputing: Informatics and Genome Projects (Smith, D. W., ed.) Academic Press, NY (1994); Computer Analysis of Sequence Data, Part I (Griffin, A. M., and Griffin, H. G., eds.) Humana Press, NJ (1994); Sequence Analysis in Molecular Biology (Von Heijne, G., ed.) Academic Press (1987); and Sequence Analysis Primer (Gribskov, M. and Devereux, J., eds.) Oxford University Press, NY (1992). Preferred methods to determine sequence identity are designed to give the best match between the sequences tested. Methods to determine sequence identity and similarity are codified in publicly available computer programs. Sequence alignments and percent identity calculations may be performed using the Megalign program of the LASERGENE bioinformatics computing suite (DNASTAR, Inc., Madison, Wisconsin). Multiple alignment of the sequences can also be performed using the Clustal method of alignment (Higgins and Sharp CABIOS, 5, 151-153 (1989) with default parameters (GAP PENALTY=10, GAP LENGTH PENALTY=10). Relevant programs also include the GCG suite of programs (Wisconsin Package Version 9.0, Genetics Computer Group (GCG), Madison, Wisconsin); BLASTP, BLASTN, BLASTX (Altschul, et al., J. Mol. Biol. 215:403-410 (1990); DNASTAR (DNASTAR, Inc., Madison, Wisconsin); and the FASTA program incorporating the Smith-Waterman algorithm (Pearson, Comput. Methods Genome Res., [Proc. Int. Symp.] (1994), Meeting Date 1992, 111-20. Editor(s): Suhai, Sandor. Publisher: Plenum, New York, N.Y.. Within the context of this disclosure it will be understood that where sequence analysis software is used for analysis, the results of the analysis are based on the "default values" of the program referenced. "Default values" mean any set of values or parameters which originally load with the software when first initialized.

**Without** limiting the foregoing, proteins or peptides having a sequence identity to a sequence disclosed herein include variants and D-substituted analogs thereof.

"Variants" of sequences disclosed herein include sequences having one or more additions, deletions, stop positions, or substitutions, as compared to a sequence disclosed herein.

An amino acid substitution can be a conservative or a non-conservative substitution. Variants of protein or peptide sequences disclosed herein can include those having one or more conservative amino acid substitutions. A "conservative substitution" involves a substitution found in one of the following conservative substitutions groups: Group 1: alanine (Ala or A), glycine (Gly or G), Ser, Thr; Group 2: aspartic acid (Asp or D), Glu; Group 3: asparagine (Asn or N), glutamine (Gin or Q); Group 4: Arg, lysine (Lys or K), histidine (His or H); Group 5: Ile, leucine (Leu or L), methionine (Met or M), valine (Val or V); and Group 6: Phe, Tyr, Trp.

Additionally, amino acids can be grouped into conservative substitution groups by similar function, chemical structure, or composition (e.g., acidic, basic, aliphatic, aromatic, sulfur-containing). For example, an aliphatic grouping may include, for purposes of substitution, Gly, Ala, Val, Leu, and Ile. Other groups containing amino acids that are considered conservative substitutions for one another include: sulfur-containing: Met and Cys; acidic: Asp, Glu, Asn, and Gln; small aliphatic, nonpolar or slightly polar residues: Ala, Ser, Thr, Pro, and Gly; polar, negatively charged residues and their amides: Asp, Asn, Glu, and Gln; polar, positively charged residues: His, Arg, and Lys; large aliphatic, nonpolar residues: Met, Leu, Ile, Val, and Cys; and large aromatic residues: Phe, Tyr, and Trp. Additional information is found in Creighton (1984) Proteins, W.H. Freeman and Company.

"D-substituted analogs" include proteins or peptides disclosed herein having one more L-amino acids substituted with one or more D-amino acids. The D-amino acid can be the same amino acid type as that found in the reference sequence or can be a different amino acid. Accordingly, D-analogs can also be variants.

Without limiting the foregoing, and for exemplary purposes only:
In particular embodiments, a binding domain includes a sequence that has at least 80%; 81%; 82%; 83%; 84%; 85%; 86%; 87%; 88%; 89%; 90%; 91%; 92%; 93%; 94%; 95%; 96%; 97%; 98%; or 99% sequence identity to an amino acid sequence of a light chain variable region (VL) or to a heavy chain variable region (VH) disclosed herein, or both, wherein each CDR includes zero changes or at most one, two, or three changes, from a monoclonal antibody or fragment thereof that specifically binds a cellular marker of interest.

In particular embodiments, binding domains include a sequence that has at least 80%; 81%; 82%; 83%; 84%; 85%; 86%; 87%; 88%; 89%; 90%; 91%; 92%; 93%; 94%; 95%; 96%; 97%; 98%; or 99% sequence identity to an amino acid sequence of a TCR Vα, Vβ, Cα, or Cβ, wherein each CDR includes zero changes or at most one, two, or three changes, from a TCR or fragment or thereof that specifically binds to a cellular marker of interest.

In particular embodiments, the binding domain Vα, Vβ, Cα, or Cβ region can be derived from or based on a Vα, Vβ, Cα, or Cβ of a known TCR (e.g., a high-affinity TCR) and contain one or more (e.g., 2, 3, 4, 5, 6, 7, 8, 9, 10) insertions, one or more (e.g., 2, 3, 4, 5, 6, 7, 8, 9, 10) deletions, one or more (e.g., 2, 3, 4, 5, 6, 7, 8, 9, 10) amino acid substitutions (e.g., conservative amino acid substitutions or non-conservative amino acid substitutions), or a combination of the above-noted changes, when compared with the Vα, Vβ, Cα, or Cβ of a known TCR. An insertion, deletion or substitution may be anywhere in a Vα, Vβ, Cα, or Cβ region, including at the amino- or carboxy-terminus or both ends of these regions, provided that each CDR includes zero changes or at most one, two, or three changes and provided a binding domain containing a modified Vα, Vβ, Cα, or Cβ region can still specifically bind its target with an affinity similar to the wild type.

In particular embodiments, a binding domain VH or VL region can be derived from or based on a VH or VL of a known monoclonal antibody and can individually or collectively contain one or more (e.g., 2, 3, 4, 5, 6, 7, 8, 9, 10) insertions, one or more (e.g., 2, 3, 4, 5, 6, 7, 8, 9, 10) deletions, one or more (e.g., 2, 3, 4, 5, 6, 7, 8, 9, 10) amino acid substitutions (e.g., conservative amino acid substitutions or non-conservative amino acid substitutions), or a combination of the above-noted changes, when compared with the VH or VL of a known monoclonal antibody. An insertion, deletion or substitution may be anywhere in the VH or VL region, including at the amino- or carboxy-terminus or both ends of these regions, provided that each CDR includes zero changes or at most one, two, or three changes and provided a binding domain containing the modified VH or VL region can still specifically bind its target with an affinity similar to the wild type binding domain.

In particular embodiments, a binding domain includes a sequence that has at least 80%; 81%; 82%; 83%; 84%; 85%; 86%; 87%; 88%; 89%; 90%; 91%; 92%; 93%; 94%; 95%; 96%; 97%; 98%; or 99% sequence identity to that of the (i) scFv for FMC63 (ii) scFv for R12; (iii) scFv for R11; or (iv) scFv for Herceptin.

In particular embodiments, an intracellular signaling domain can have at least 80%; 81%; 82%; 83%; 84%; 85%; 86%; 87%; 88%; 89%; 90%; 91%; 92%; 93%; 94%; 95%; 96%; 97%; 98%; or 99% sequence identity a to CD3ζ having a sequence provided in FIG. 2.

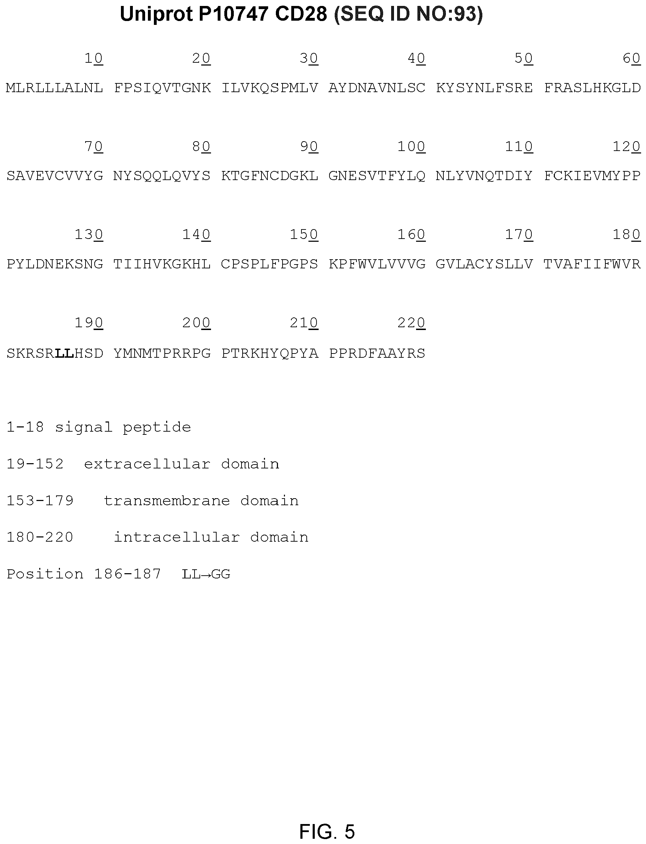

In particular embodiments, a costimulatory signaling domain can have at least 80%; 81%; 82%; 83%; 84%; 85%; 86%; 87%; 88%; 89%; 90%; 91%; 92%; 93%; 94%; 95%; 96%; 97%; 98%; or 99% sequence identity to the intracellular domain of CD28 as shown in FIG. 5 or to 4-1BB having a sequence provided in FIG. 2. In particular embodiments, a variant of the CD28 intracellular domain includes an amino acid substitution at positions 186-187, wherein LL is substituted with GG.

In particular embodiments, a transmembrane domain can be selected or modified by an amino acid substitution(s) to avoid binding of such domains to the transmembrane domains of the same or different surface membrane proteins to minimize interactions with other members of the receptor complex. In further particular embodiments, synthetic or variant transmembrane domains include predominantly hydrophobic residues such as leucine and valine. Variant transmembrane domains preferably have a hydrophobic score of at least 50 as calculated by Kyte Doolittle. In particular embodiments, a transmembrane domain can have at least 80%; 81%; 82%; 83%; 84%; 85%; 86%; 87%; 88%; 89%; 90%; 91%; 92%; 93%; 94%; 95%; 96%; 97%; 98%; or 99% sequence identity with a sequence of FIG. 2 or 6.

Proteins and peptides having the same functional capability as those expressly disclosed herein are also included.

When not expressly provided here, sequence information provided by public databases and the knowledge of those of ordinary skill in the art can be used to identify related and relevant protein and peptide sequences and gene sequences encoding such proteins and peptides.

Differentiation. In particular embodiments, modified HSPC are differentiated into modified non-T effector cells before administration to a subject. Where differentiation of modified HSPC is desired, HSPC can be exposed to one or more growth factors that promote differentiation into non-T effector cells. The growth factors and cell culture conditions that promote differentiation are known in the art (see, e.g., U.S. Patent No. 7,399,633 at Section 5.2 and Section 5.5). For example, SCF can be used in combination with GM-SCF or IL-7 to differentiate HSPC into myeloid stem/progenitor cells or lymphoid stem/progenitor cells, respectively. In particular embodiments, HSPC can be differentiated into a lymphoid stem/progenitor cell by exposing HSPC to 100 ng/ml of each of SCF and GM-SCF or IL-7. In particular embodiments, a retinoic acid receptor (RAR) agonist, or preferably all trans retinoic acid (ATRA) is used to promote the differentiation of HSPC. Differentiation into natural killer cells, for example, can be achieved by exposing cultured HSPC to RPMI media supplemented with human serum, IL-2 at 50 U/mL and IL-15 at 500ng/mL. In additional embodiments, RPMI media can also be supplemented L-glutamine.

In particular embodiments, modified HSPC can be differentiated into non-T effector cells including natural killer (NK) cells or neutrophils. NK cells perform two major functions: (i) recognizing and killing tumor cells and other virally infected cells; and (ii) regulating innate and adaptive immune responses by secreting CCL3, CCL4, CCL5, and/or XCL1 chemokines or cytokines such as granulocyte-macrophage colony-stimulating factor, tumor necrosis factor-a, or IFN-γ. Neutrophils generally circulate in the blood stream until they travel to sites of inflammation where they target and destroy aberrant cell types.

Compositions and Formulations. Cells and modified cells can be prepared as compositions and/or formulations for administration to a subject. A composition refers to a cell or modified cell prepared with a pharmaceutically acceptable carrier for administration to a subject. A formulation refers to at least two cell types within a pharmaceutically acceptable carrier (hereafter carrier) for administration to a subject.

**At** various points during preparation of a composition or formulation, it can be necessary or beneficial to cryopreserve a cell. The terms "frozen/freezing" and "cryopreserved/cryopreserving" can be used interchangeably. Freezing includes freeze drying.

**As** is understood by one of ordinary skill in the art, the freezing of cells can be destructive (see Mazur, P., 1977, Cryobiology 14:251-272) but there are numerous procedures available to prevent such damage. For example, damage can be avoided by (a) use of a cryoprotective agent, (b) control of the freezing rate, and/or (c) storage at a temperature sufficiently low to minimize degradative reactions. Exemplary cryoprotective agents include dimethyl sulfoxide (DMSO) (Lovelock and Bishop, 1959, Nature 183:1394-1395; Ashwood-Smith, 1961, Nature 190:1204-1205), glycerol, polyvinylpyrrolidine (Rinfret, 1960, Ann. N.Y. Acad. Sci. 85:576), polyethylene glycol (Sloviter and Ravdin, 1962, Nature 196:548), albumin, dextran, sucrose, ethylene glycol, i-erythritol, D-ribitol, D-mannitol (Rowe et al., 1962, Fed. Proc. 21:157), D-sorbitol, i-inositol, D-lactose, choline chloride (Bender et al.., 1960, J. Appl. Physiol. 15:520), amino acids (Phan The Tran and Bender, 1960, Exp. Cell Res. 20:651), methanol, acetamide, glycerol monoacetate (Lovelock, 1954, Biochem. J. 56:265), and inorganic salts (Phan The Tran and Bender, 1960, Proc. Soc. Exp. Biol. Med. 104:388; Phan The Tran and Bender, 1961, in Radiobiology, Proceedings of the Third Australian Conference on Radiobiology, Ilbery ed., Butterworth, London, p. 59). In particular embodiments, DMSO can be used. Addition of plasma (e.g., to a concentration of 20-25%) can augment the protective effects of DMSO. After addition of DMSO, cells can be kept at 0° C until freezing, because DMSO concentrations of 1% can be toxic at temperatures above 4° C.

In the cryopreservation of cells, slow controlled cooling rates can be critical and different cryoprotective agents (Rapatz et al., 1968, Cryobiology 5(1): 18-25) and different cell types have different optimal cooling rates (see e.g., Rowe and Rinfret, 1962, Blood 20:636; Rowe, 1966, Cryobiology 3(1):12-18; Lewis, et al., 1967, Transfusion 7(1):17-32; and Mazur, 1970, Science 168:939- 949 for effects of cooling velocity on survival of stem cells and on their transplantation potential). The heat of fusion phase where water turns to ice should be minimal. The cooling procedure can be carried out by use of, e.g., a programmable freezing device or a methanol bath procedure. Programmable freezing apparatuses allow determination of optimal cooling rates and facilitate standard reproducible cooling.

In particular embodiments, DMSO-treated cells can be pre-cooled on ice and transferred to a tray containing chilled methanol which is placed, in turn, in a mechanical refrigerator *(e.g.,* Harris or Revco) at -80° C. Thermocouple measurements of the methanol bath and the samples indicate a cooling rate of 1° to 3° C/minute can be preferred. After at least two hours, the specimens can have reached a temperature of - 80° C and can be placed directly into liquid nitrogen (-196° C).

After thorough freezing, the cells can be rapidly transferred to a long-term cryogenic storage vessel. In a preferred embodiment, samples can be cryogenically stored in liquid nitrogen (-196° C) or vapor (-1° C). Such storage is facilitated by the availability of highly efficient liquid nitrogen refrigerators.

Further considerations and procedures for the manipulation, cryopreservation, and long-term storage of cells, can be found in the following exemplary references: U.S. Patent Nos. 4,199,022; 3,753,357; and 4,559,298; Gorin, 1986, Clinics In Haematology 15(1):19-48; Bone-Marrow Conservation, Culture and Transplantation, Proceedings of a Panel, Moscow, July 22-26, 1968, International Atomic Energy Agency, Vienna, pp. 107-186; Livesey and Linner, 1987, Nature 327:255; Linner et al., 1986, J. Histochem. Cytochem. 34(9):1123-1135; Simione, 1992, J. Parenter. Sci. Technol. 46(6):226-32).

Following cryopreservation, frozen cells can be thawed for use in accordance with methods known to those of ordinary skill in the art. Frozen cells are preferably thawed quickly and chilled immediately upon thawing. In particular embodiments, the vial containing the frozen cells can be immersed up to its neck in a warm water bath; gentle rotation will ensure mixing of the cell suspension as it thaws and increase heat transfer from the warm water to the internal ice mass. As soon as the ice has completely melted, the vial can be immediately placed on ice.

In particular embodiments, methods can be used to prevent cellular clumping during thawing. Exemplary methods include: the addition before and/or after freezing of DNase (Spitzer et al., 1980, Cancer 45:3075-3085), low molecular weight dextran and citrate, hydroxyethyl starch (Stiff et al., 1983, Cryobiology 20:17-24), etc.

As is understood by one of ordinary skill in the art, if a cryoprotective agent that is toxic to humans is used, it should be removed prior to therapeutic use. DMSO has no serious toxicity.

Exemplary carriers and modes of administration of cells are described at pages 14-15 of U.S. Patent Publication No. 2010/0183564. Additional pharmaceutical carriers are described in Remington: The Science and Practice of Pharmacy, 21st Edition, David B. Troy, ed., Lippicott Williams & Wilkins (2005).

In particular embodiments, cells can be harvested from a culture medium, and washed and concentrated into a carrier in a therapeutically-effective amount. Exemplary carriers include saline, buffered saline, physiological saline, water, Hanks' solution, Ringer's solution, Nonnosol-R (Abbott Labs), Plasma-Lyte A^{®} (Baxter Laboratories, Inc., Morton Grove, IL), glycerol, ethanol, and combinations thereof.

In particular embodiments, carriers can be supplemented with human serum albumin (HSA) or other human serum components or fetal bovine serum. In particular embodiments, a carrier for infusion includes buffered saline with 5% HAS or dextrose. Additional isotonic agents include polyhydric sugar alcohols including trihydric or higher sugar alcohols, such as glycerin, erythritol, arabitol, xylitol, sorbitol, or mannitol.

Carriers can include buffering agents, such as citrate buffers, succinate buffers, tartrate buffers, fumarate buffers, gluconate buffers, oxalate buffers, lactate buffers, acetate buffers, phosphate buffers, histidine buffers, and/or trimethylamine salts.

Stabilizers refer to a broad category of excipients which can range in function from a bulking agent to an additive which helps to prevent cell adherence to container walls. Typical stabilizers can include polyhydric sugar alcohols; amino acids, such as arginine, lysine, glycine, glutamine, asparagine, histidine, alanine, ornithine, L-leucine, 2-phenylalanine, glutamic acid, and threonine; organic sugars or sugar alcohols, such as lactose, trehalose, stachyose, mannitol, sorbitol, xylitol, ribitol, myoinisitol, galactitol, glycerol, and cyclitols, such as inositol; PEG; amino acid polymers; sulfur-containing reducing agents, such as urea, glutathione, thioctic acid, sodium thioglycolate, thioglycerol, alpha-monothioglycerol, and sodium thiosulfate; low molecular weight polypeptides (i.e., <10 residues); proteins such as HSA, bovine serum albumin, gelatin or immunoglobulins; hydrophilic polymers such as polyvinylpyrrolidone; monosaccharides such as xylose, mannose, fructose and glucose; disaccharides such as lactose, maltose and sucrose; trisaccharides such as raffinose, and polysaccharides such as dextran.

Where necessary or beneficial, compositions or formulations can include a local anesthetic such as lidocaine to ease pain at a site of injection.

Exemplary preservatives include phenol, benzyl alcohol, meta-cresol, methyl paraben, propyl paraben, octadecyldimethylbenzyl ammonium chloride, benzalkonium halides, hexamethonium chloride, alkyl parabens such as methyl or propyl paraben, catechol, resorcinol, cyclohexanol, and 3-pentanol.

Therapeutically effective amounts of cells within compositions or formulations can be greater than 10² cells, greater than 10³ cells, greater than 10⁴ cells, greater than 10⁵ cells, greater than 10⁶ cells, greater than 10⁷ cells, greater than 10⁸ cells, greater than 10⁹ cells, greater than 10¹⁰ cells, or greater than 10¹¹.

In compositions and formulations disclosed herein, cells are generally in a volume of a liter or less, 500 mls or less, 250 mls or less or 100 mls or less. Hence the density of administered cells is typically greater than 10⁴ cells/ml, 10⁷ cells/ml or 10⁸ cells/ml.

As indicated, compositions include one cell type (e.g., modified HSPC or modified effectors). Formulations can include HSPC, modified-HSPC and/or modified-effectors (such as modified-NK cells) in combination. In particular embodiments, combinations of modified-HSPC and modified-effectors with the same binding domain are combined. In other embodiments, modified-HSPC and modified-effectors of different binding domains are combined. Similarly, all other aspects of an expressed molecule (e.g., effector domain components, spacer regions, etc.) can be the same or different in various combinations between modified HSPC and modified effectors within a formulation. Additionally, modified HSPC expressing different molecules or components thereof can be included together within a formulation and modified effectors expressing different molecules or components thereof can be included together within a formulation. In particular embodiments, a formulation can include at least two modified HSPC expressing different molecules and at least two modified effector cells expressing different molecules.

HSPC, modified-HSPC and modified-effectors can be combined in different ratios for example, a 1:1:1 ratio, 2:1:1 ratio, 1:2:1 ratio, 1:1:2 ratio, 5:1:1 ratio, 1:5:1 ratio, 1:1:5 ratio, 10:1:1 ratio, 1:10:1 ratio, 1:1:10 ratio, 2:2:1 ratio, 1:2:2 ratio, 2:1:2 ratio, 5:5:1 ratio, 1:5:5 ratio, 5:1:5 ratio, 10:10:1 ratio, 1:10:10 ratio, 10:1:10 ratio, etc. These ratios can also apply to numbers of cells expressing the same or different molecule components. If only two of the cell types are combined or only 2 combinations of expressed molecule components are included within a formulation, the ratio can include any 2 number combination that can be created from the 3 number combinations provided above. In embodiments, the combined cell populations are tested for efficacy and/or cell proliferation in vitro and/or in vivo, and the ratio of cells that provides for efficacy and/or proliferation of cells is selected.

**The** compositions and formulations disclosed herein can be prepared for administration by, for example, injection, infusion, perfusion, or lavage. The compositions and formulations can further be formulated for bone marrow, intravenous, intradermal, intraarterial, intranodal, intralymphatic, intraperitoneal, intralesional, intraprostatic, intravaginal, intrarectal, topical, intrathecal, intratumoral, intramuscular, intravesicular, and/or subcutaneous injection.

Kits. Kits can include one or more containers including one or more of the cells, compositions or formulations described herein. In particular embodiments, the kits can include one or more containers containing one or more cells, compositions or formulations and/or compositions to be used in combination with other cells, compositions or formulations. Associated with such container(s) can be a notice in the form prescribed by a governmental agency regulating the manufacture, use, or sale of pharmaceuticals or biological products, which notice reflects approval by the agency of manufacture, use, or sale for human administration. The notice may state that the provided cells, compositions or formulations can be administered to a subject without immunological matching. The kits can include further instructions for using the kit, for example, instructions regarding preparation of cells, compositions and/or formulations for administration; proper disposal of related waste; and the like. The instructions can be in the form of printed instructions provided within the kit or the instructions can be printed on a portion of the kit itself. Instructions may be in the form of a sheet, pamphlet, brochure, CD-Rom, or computer-readable device, or can provide directions to instructions at a remote location, such as a website. In particular embodiments, kits can also include some or all of the necessary medical supplies needed to use the kit effectively, such as syringes, ampules, tubing, facemask, a needleless fluid transfer device, an injection cap, sponges, sterile adhesive strips, Chloraprep, gloves, and the like. Variations in contents of any of the kits described herein can be made.

Methods of Use. Methods disclosed herein include treating subjects (humans, veterinary animals (dogs, cats, reptiles, birds, etc.), livestock (horses, cattle, goats, pigs, chickens, etc.), and research animals (monkeys, rats, mice, fish, etc.) with cells disclosed herein. Treating subjects includes delivering therapeutically effective amounts. Therapeutically effective amounts include those that provide effective amounts, prophylactic treatments, and/or therapeutic treatments.

An "effective amount" is the number of cells necessary to result in a desired physiological change in a subject. Effective amounts are often administered for research purposes. Effective amounts disclosed herein do one or more of: (i) provide blood support by reducing immunodeficiency, pancytopenia, neutropenia and/or leukopenia (e.g., repopulating cells of the immune system and (ii) have an anti-cancer effect.

A "prophylactic treatment" includes a treatment administered to a subject who does not display signs or symptoms of a condition to be treated or displays only early signs or symptoms of the condition to be treated such that treatment is administered for the purpose of diminishing, preventing, or decreasing the risk of developing the condition. Thus, a prophylactic treatment functions as a preventative treatment against a condition.

A "therapeutic treatment" includes a treatment administered to a subject who displays symptoms or signs of a condition and is administered to the subject for the purpose of reducing the severity or progression of the condition.

The actual dose amount administered to a particular subject can be determined by a physician, veterinarian, or researcher taking into account parameters such as physical and physiological factors including target; body weight; type of condition; severity of condition; upcoming relevant events, when known; previous or concurrent therapeutic interventions; idiopathy of the subject; and route of administration, for example. In addition, in vitro and in vivo assays can optionally be employed to help identify optimal dosage ranges.

Therapeutically effective amounts to administer can include greater than 10² cells, greater than 10³ cells, greater than 10⁴ cells, greater than 10⁵ cells, greater than 10⁶ cells, greater than 10⁷ cells, greater than 10⁸ cells, greater than 10⁹ cells, greater than 10¹⁰ cells, or greater than 10¹¹.

As indicated, the compositions and formulations disclosed herein can be administered by, for example, injection, infusion, perfusion, or lavage and can more particularly include administration through one or more bone marrow, intravenous, intradermal, intraarterial, intranodal, intralymphatic, intraperitoneal, intralesional, intraprostatic, intravaginal, intrarectal, topical, intrathecal, intratumoral, intramuscular, intravesicular, and/or subcutaneous infusions and/or bolus injections.

Uses of non-modified HSPC are described in sec. 5.6.1 of U.S. Patent No. 7,399,633 and WO 2013/086436. HSPC and modified HSPC can be administered for the same purposes or different purposes. Common purposes include to provide hematopoietic function to a subject in need thereof; and/or to treat one or more of immunodeficiency, pancytopenia, neutropenia and/or leukopenia (including cyclic neutropenia and idiopathic neutropenia) (collectively, "the purposes"). HSPC and modified HSPC can be administered to subjects who have a decreased blood cell level, or are at risk of developing a decreased blood cell level as compared to a control blood cell level. In particular embodiments, the subject has anemia or is at risk for developing anemia.

Treatment for the purposes can be needed based on exposure to an intensive chemotherapy regimen including exposure to one or more of alkylating agents, Ara-C, azathioprine, carboplatin, cisplatin, chlorambucil, clofarabine, cyclophosphamide, ifosfamide, mechlorethamine, mercaptopurine, oxaliplatin, taxanes, and vinca alkaloids (e.g., vincristine, vinblastine, vinorelbine, and vindesine).

Treatment for the purposes can also be needed based on exposure to a myeloablative regimen for hematopoietic cell transplantation (HCT). In particular embodiments, HSPC and/or modified-HSPC are administered to a bone marrow donor, at risk of depleted bone marrow, or at risk for depleted or limited blood cell levels. Administration can occur prior to and/or after harvesting of the bone marrow. HSPC and/or modified-HSPC can also be administered to a recipient of a bone marrow transplant.

Treatment for the purposes can also be needed based on exposure to acute ionizing radiation and/or exposure to other drugs that can cause bone marrow suppression or hematopoietic deficiencies including antibiotics, penicillin, gancyclovir, daunomycin, sulfa drugs, phenothiazones, tranquilizers, meprobamate, analgesics, aminopyrine, dipyrone, anticonvulsants, phenytoin, carbamazepine, antithyroids, propylthiouracil, methimazole, and diuretics.

Treatment for the purposes can also be needed based on viral (e.g., HIVI, HIVII, HTLVI, HTLVII, HTLVIII), microbial or parasitic infections and/or as a result of treatment for renal disease or renal failure, e.g., dialysis. Various immunodeficiencies, e.g., in T and/or B lymphocytes, or immune disorders, e.g., rheumatoid arthritis, may also be beneficially affected by treatment with HSPC and/or modified-HSPC. Immunodeficiencies may also be the result of other medical treatments.

HSPC and modified-HSPC can also be used to treat aplastic anemia, Chediak-Higashi syndrome, systemic lupus erythematosus (SLE), leukemia, myelodysplastic syndrome, myelofibrosis or thrombocytopenia. Severe thrombocytopenia may result from genetic defects such as Fanconi's Anemia, Wiscott- Aldrich, or May-Hegglin syndromes. Acquired thrombocytopenia may result from auto- or allo-antibodies as in Immune Thrombocytopenia Purpura, Systemic Lupus Erythromatosis, hemolytic anemia, or fetal maternal incompatibility. In addition, splenomegaly, disseminated intravascular coagulation, thrombotic thrombocytopenic purpura, infection, and/or prosthetic heart valves may result in thrombocytopenia. Thrombocytopenia may also result from marrow invasion by carcinoma, lymphoma, leukemia or fibrosis.

In particular embodiments, the subject has blood loss due to, e.g., trauma, or is at risk for blood loss. In particular embodiments, the subject has depleted bone marrow related to, e.g., congenital, genetic or acquired syndrome characterized by bone marrow loss or depleted bone marrow. In particular embodiments, the subject is in need of hematopoiesis.

As indicated in relation to bone marrow donors, administration of HSPC or modified-HSPC to a subject can occur at any time within a treatment regimen deemed helpful by an administering professional. As non-limiting examples, HSPC and/or modified-HSPC can be administered to a subject, e.g., before, at the same time, or after chemotherapy, radiation therapy or a bone marrow transplant. HSPC and/or modified - HSPC can be effective to provide engraftment when assayed at 1, 2, 3, 4, 5, 6, 7, 8, 9, 10 days (or more or less than 1, 2, 3, 4, 5, 6, 7, 8, 9, 10 days); 1, 2, 3, 4, 5, 6, 7, 8, 9, 10 weeks (or more or less than 1, 2, 3, 4, 5, 6, 7, 8, 9, 10 weeks); 1; 2, 3, 4, 5, 6, 7, 8, 9, 10, 11, 12 months (or more or less than 1, 2, 3, 4, 5, 6, 7, 8, 9, 10, 11, 12 months); or 1, 2, 3, 4, 5 years (or more or less than 1, 2, 3, 4, 5 years) after administration of the HSPC and/or modified-HSPC to a subject. In particular embodiments, the HSPC and/or modified-HSPC are effective to provide engraftment when assayed within 10 days, 2 weeks, 3 weeks, 4 weeks, 6 weeks, or 13 weeks after administration of the HSPC and/or CAR-HSPC to a subject.

HSPC, Modified-HSPC and Modified Effectors. HSPC, modified-HSPC and modified-effectors can be administered for different purposes within a treatment regimen. The use of HSPC and modified HSPC to provide blood support, and modified HSPC and modified effectors to provide a graft vs. leukemia effect in the treatment of ALL is described above. Similar approaches can be used to provide blood support and/or to target unwanted cancer cells and as an adjunct treatment to chemotherapy or radiation.

Exemplary cancers that can be treated with modified HSPC and modified effectors include adrenal cancers, bladder cancers, blood cancers, bone cancers, brain cancers, breast cancers, carcinoma, cervical cancers, colon cancers, colorectal cancers, corpus uterine cancers, ear, nose and throat (ENT) cancers, endometrial cancers, esophageal cancers, gastrointestinal cancers, head and neck cancers, Hodgkin's disease, intestinal cancers, kidney cancers, larynx cancers, leukemias, liver cancers, lymph node cancers, lymphomas, lung cancers, melanomas, mesothelioma, myelomas, nasopharynx cancers, neuroblastomas, non-Hodgkin's lymphoma, oral cancers, ovarian cancers, pancreatic cancers, penile cancers, pharynx cancers, prostate cancers, rectal cancers, sarcoma, seminomas, skin cancers, stomach cancers, teratomas, testicular cancers, thyroid cancers, uterine cancers, vaginal cancers, vascular tumors, and metastases thereof.

In the context of cancers, therapeutically effective amounts have an anti-cancer effect. An anti-cancer effect can be quantified by observing a decrease in the number of tumor cells, a decrease in the number of metastases, a decrease in tumor volume, an increase in life expectancy, induction of apoptosis of cancer cells, induction of cancer cell death, inhibition of cancer cell proliferation, inhibition of tumor growth, prevention of metastasis, prolongation of a subject's life, and/or reduction of relapse or re-occurrence of the cancer following treatment.

In the context of blood support, therapeutically effective amounts treat immunodeficiency, pancytopenia, neutropenia and/or leukopenia by increasing the number of desired cells in a subject's circulation. Increasing the desired number of cells in a subject's circulation can re-populate the subject's immune system by increasing the number of immune system cells and/or immune system cell progenitors.

In particular embodiments utilizing modified-HSPC and modified-effectors, a subject's cancer cells can be characterized for presence of cellular markers. The binding domain expressed by a modified-HSPC or modified-effector can be selected based on the characterization of the cellular marker. In particular embodiments, modified-HSPC and modified-effectors previously generated are selected for a subject's treatment based on their ability to bind a cellular marker preferentially expressed on a particular subject's cancer cells.

When formulated to treat cancer, the disclosed compositions and formulations can also include plasmid DNA carrying one or more anticancer genes selected from p53, RB, BRCA1, E1A, bcl-2, MDR-1, p21, p16, bax, bcl-xs, E2F, IGF-I VEGF, angiostatin, oncostatin, endostatin, GM-CSF, IL-12, IL-2, IL-4, IL-7, IFN-γ, TNF-α and/or HSV-tk. Compositions and formulations can also include or be administered in combination with one or more antineoplastic drugs including adriamycin, angiostatin, azathioprine, bleomycin, busulfane, camptothecin, carboplatin, carmustine, chlorambucile, chlormethamine, chloroquinoxaline sulfonamide, cisplatin, cyclophosphamide, cycloplatam, cytarabine, dacarbazine, dactinomycin, daunorubicin, didox, doxorubicin, endostatin, enloplatin, estramustine, etoposide, extramustinephosphat, flucytosine, fluorodeoxyuridine, fluorouracil, gallium nitrate, hydroxyurea, idoxuridine, interferons, interleukins, leuprolide, lobaplatin, lomustine, mannomustine, mechlorethamine, mechlorethaminoxide, melphalan, mercaptopurine, methotrexate, mithramycin, mitobronitole, mitomycin, mycophenolic acid, nocodazole, oncostatin, oxaliplatin, paclitaxel, pentamustine, platinum-triamine complex, plicamycin, prednisolone, prednisone, procarbazine, protein kinase C inhibitors, puromycine, semustine, signal transduction inhibitors, spiroplatin, streptozotocine, stromelysin inhibitors, taxol, tegafur, telomerase inhibitors, teniposide, thalidomide, thiamiprine, thioguanine, thiotepa, tiamiprine, tretamine, triaziquone, trifosfamide, tyrosine kinase inhibitors, uramustine, vidarabine, vinblastine, vinca alcaloids, vincristine, vindesine, vorozole, zeniplatin, zeniplatin or zinostatin.

Modified-HSPC and Modified Effectors. Modified-HSPC and/or modified-effectors can be used without HSPC when a treatment to provide hematopoietic function or to treat immunodeficiency; pancytopenia; neutropenia and/or leukopenia is not desired or needed.

As is understood by one of ordinary skill in the art, animal models of different blood disorders and cancers are well known and can be used to assess effectiveness of particular treatment paradigms, as necessary or beneficial.

**The** Examples and Exemplary Embodiments below are included to demonstrate particular embodiments of the disclosure. Those of ordinary skill in the art should recognize in light of the present disclosure that many changes can be made to the specific embodiments disclosed herein and still obtain a like or similar result without departing from the spirit and scope of the disclosure.

### Exemplary Embodiments.

1. A CD34+ hematopoietic stem progenitor cell (HSPC) genetically modified to express (i) an extracellular component including a ligand binding domain that binds CD19; (ii) an intracellular component including an effector domain including a cytoplasmic domain of CD28 or 4-1BB; (iii) a spacer region including a hinge region of human IgG4; and (iv) a human CD4 or CD28 transmembrane domain.
2. A HSPC of embodiment 1 wherein the ligand binding domain is a single chain Fv fragment (scFv) including a CDRL1 sequence of RASQDISKYLN (SEQ ID NO. 108), a CDRL2 sequence of SRLHSGV (SEQ ID NO. 111), a CDRL3 sequence of GNTLPYTFG (SEQ ID NO. 104), a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).
3. A HSPC of embodiments 1 or 2 wherein the spacer region is 12 amino acids or less.
4. A HSPC of any one of embodiments 1-3 wherein the spacer region includes SEQ ID NO: 47.
5. A non-T effector cell genetically modified to express (i) an extracellular component including a ligand binding domain that binds CD19; (ii) an intracellular component including an effector domain including a cytoplasmic domain of CD28 or 4-1BB; (iii) a spacer region including a hinge region of human IgG4; and (iv) a human CD4 or CD28 transmembrane domain.
6. A non-T effector cell of embodiment 5 wherein the ligand binding domain is a single chain Fv fragment (scFv) including a CDRL1 sequence of RASQDISKYLN (SEQ ID NO. 108), a CDRL2 sequence of SRLHSGV (SEQ ID NO. 111), a CDRL3 sequence of GNTLPYTFG (SEQ ID NO. 104), a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).
7. A non-T effector cell of embodiments 5 or 6 wherein the spacer region is 12 amino acids or less.
8. A non-T effector cell of any one of embodiments 5-7 wherein the spacer region includes SEQ ID NO: 47.
9. A non-T effector cell of any one of embodiments 5-8 wherein the non-T effector cell is a natural killer cell.
10. A hematopoietic stem progenitor cell (HSPC) genetically modified to express a chimeric antigen receptor (CAR) of SEQ ID NO: 34, 53, 54, 55, 56, 57, or 58.
11.A HSPC of embodiment 10 wherein the HSPC is CD34+.
12. A non-T effector cell genetically modified to express a CAR of SEQ ID NO: 34, 53, 54, 55, 56, 57, or 58.
13. A non-T effector cell of embodiment 12 wherein the non-T effector cell is a natural killer cell.
14. A HSPC genetically modified to express (i) an extracellular component including a ligand binding domain that binds a cellular marker that is preferentially expressed on an unwanted cell; and (ii) an intracellular component including an effector domain.
15.A HSPC of embodiment 14 wherein the ligand binding domain is an antibody fragment.
16. A HSPC of embodiments 14 or 15 wherein the ligand binding domain is single chain variable fragment of an antibody.
17. A HSPC of any one of embodiments 14-16 wherein the ligand binding domain binds CD19.
18.A HSPC of any one of embodiments 14-17 wherein the ligand binding domain is a scFv including a CDRL1 sequence of RASQDISKYLN (SEQ ID NO. 108), a CDRL2 sequence of SRLHSGV (SEQ ID NO. 111), a CDRL3 sequence of GNTLPYTFG (SEQ ID NO. 104), a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).
19. A HSPC of embodiment 18 wherein the HSPC is also genetically modified to express a spacer region of 12 amino acids or less.
20.A HSPC of embodiment 19 wherein the spacer region includes SEQ ID NO: 47.
21.A HSPC of any one of embodiments 14-16 wherein the ligand binding domain binds ROR1.
22. A HSPC of any one of embodiments 14-16 or 21 wherein the ligand binding domain is a scFv including a CDRL1 sequence of ASGFDFSAYYM (SEQ ID NO. 101), a CDRL2 sequence of TIYPSSG (SEQ ID NO. 112), a CDRL3 sequence of ADRATYFCA (SEQ ID NO. 100), a CDRH1 sequence of DTIDWY (SEQ ID NO. 102), a CDRH2 sequence of VQSDGSYTKRPGVPDR (SEQ ID NO. 113), and a CDRH3 sequence of YIGGYVFG (SEQ ID NO. 117).
23. A HSPC of any one of embodiments 14-16 or 21 wherein the ligand binding domain is a scFv including a CDRL1 sequence of SGSDINDYPIS (SEQ ID NO. 109), a CDRL2 sequence of INSGGST (SEQ ID NO. 105), a CDRL3 sequence of YFCARGYS (SEQ ID NO. 116), a CDRH1 sequence of SNLAW (SEQ ID NO. 110), a CDRH2 sequence of RASNLASGVPSRFSGS (SEQ ID NO. 107), and a CDRH3 sequence of NVSYRTSF (SEQ ID NO. 106).
24. A HSPC of embodiment 23 wherein the HSPC is also genetically modified to express a spacer region of 229 amino acids or less.
25. A HSPC of embodiment 24 wherein the spacer region includes SEQ ID NO: 61.
26. A HSPC of any one of embodiments 14-16 wherein the ligand binding domain binds PSMA, PSCA, mesothelin, CD20, WT1, or Her2.
27. A HSPC of any one of embodiments 14-26 wherein the intracellular component includes an effector domain including one or more signaling and/or stimulatory domains selected from: 4-1BB, CARD11, CD3 γ, CD3δ, CD3ε, CD3ζ, CD27, CD28, CD79A, CD79B, DAP10, FcRa, FcRβ, FcRy, Fyn, HVEM, ICOS, LAG3, LAT, Lck, LRP, NKG2D, NOTCH1, pTα, PTCH2, OX40, ROR2, Ryk, SLAMF1, Slp76, TCRα, TCRβ, TRIM, Wnt, and Zap70 signaling and/or stimulatory domains.
28. A HSPC of any one of embodiments 14-27 wherein the intracellular component includes an effector domain including an intracellular signaling domain of CD3ζ, 0028ζ, or 4-1BB.
29.A HSPC of any one of embodiments 14-28 wherein the intracellular component includes an effector domain including one or more costimulatory domains selected from: CD27, CD28, 4-1BB, OX40, CD30, CD40, lymphocyte function-associated antigen-1 (LFA-1), CD2, CD7, LIGHT, NKG2C, or B7-H3 costimulatory domains.
30.A HSPC of any one of embodiments 14-29 wherein the intracellular component includes an effector domain including an intracellular signaling domain including (i) all or a portion of the signaling domain of CD3ζ, (ii) all or a portion of the signaling domain of CD28, (iii) all or a portion of the signaling domain of 4-1BB, or (iv) all or a portion of the signaling domain of CD3ζ, CD28, and/or 4-1BB.
31.A HSPC of any one of embodiments 14-30 wherein the intracellular component includes an effector domain including a variant of CD3ζ and/or a portion of the 4-1BB intracellular signaling domain.
32. A HSPC of any one of embodiments 14-18, 21-23, or 26-31 wherein the HSPC is also genetically modified to express a spacer region.
33. A HSPC of embodiment 32 wherein the spacer region includes a portion of a hinge region of a human antibody.
34. A HSPC of embodiment 32 or 33 wherein the spacer region includes a hinge region and at least one other portion of an Fc domain of a human antibody selected from CH1, CH2, CH3 or combinations thereof.
35. A HSPC of embodiment 32 or 33 wherein the spacer region includes a Fc domain and a human IgG4 heavy chain hinge.
36. A HSPC of embodiment 32 wherein the spacer region is of a length selected from 12 amino acids or less, 119 amino acids or less, or 229 amino acids or less.
37.A HSPC of embodiment 32 wherein the spacer region is SEQ ID NO:47, SEQ ID NO:52, or SEQ ID NO:61.
38. A HSPC of any one of embodiments 14-37 wherein the HSPC is also genetically modified to express a transmembrane domain.
39.A HSPC of embodiment 38 wherein the transmembrane domain is a CD28 transmembrane domain or a CD4 transmembrane domain.
40. A HSPC of any one of embodiments 14-39 wherein the extracellular component further includes a tag sequence.
41. A HSPC of embodiment 40 wherein the tag sequence is EGFR lacking an intracellular signaling domain.
42.A HSPC of any one of embodiments 14-41 wherein the HSPC is CD34+.
43. A non-T effector cell genetically modified to express (i) an extracellular component including a ligand binding domain that binds a cellular marker on an unwanted cell; and (ii) an intracellular component including an effector domain.
44. A non-T effector cell of embodiment 43 wherein the ligand binding domain is an antibody fragment.
45. A non-T effector cell of embodiment 43 or 44 wherein the ligand binding domain is single chain variable fragment of an antibody.
46. A non-T effector cell of any one of embodiments 43-45 wherein the ligand binding domain binds CD19.
47. A non-T effector cell of any one of embodiments 43-46 wherein the ligand binding domain is a scFv including a CDRL1 sequence of RASQDISKYLN (SEQ ID NO. 108), a CDRL2 sequence of SRLHSGV (SEQ ID NO. 111), a CDRL3 sequence of GNTLPYTFG (SEQ ID NO. 104), a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).
48.A non-T effector cell of embodiment 47 wherein the non-T effector cell is also genetically modified to express a spacer region of 12 amino acids or less.
49. A non-T effector cell of embodiment 48 wherein the spacer region includes SEQ ID NO: 47.
50. A non-T effector cell of any one of embodiments 43-45 wherein the ligand binding domain binds ROR1.
51. A non-T effector cell of any one of embodiments 43-45 or 50 wherein the ligand binding domain is a scFv including a CDRL1 sequence of ASGFDFSAYYM (SEQ ID NO. 101), a CDRL2 sequence of TIYPSSG (SEQ ID NO. 112), a CDRL3 sequence of ADRATYFCA (SEQ ID NO. 100), a CDRH1 sequence of DTIDWY (SEQ ID NO. 102), a CDRH2 sequence of VQSDGSYTKRPGVPDR (SEQ ID NO. 113), and a CDRH3 sequence of YIGGYVFG (SEQ ID NO. 117).
52.A non-T effector cell of any one of embodiments 43-45 or 50 wherein the ligand binding domain is a single chain Fv fragment (scFv) including a CDRL1 sequence of SGSDINDYPIS (SEQ ID NO. 109), a CDRL2 sequence of INSGGST (SEQ ID NO. 105), a CDRL3 sequence of YFCARGYS (SEQ ID NO. 116), a CDRH1 sequence of SNLAW (SEQ ID NO. 110), a CDRH2 sequence of RASNLASGVPSRFSGS (SEQ ID NO. 107), and a CDRH3 sequence of NVSYRTSF (SEQ ID NO. 106).
53.A non-T effector cell of embodiment 52 wherein the non-T effector cell is also genetically modified to express a spacer region that is 229 amino acids or less.
54. A non-T effector cell of embodiment 53 wherein the spacer region includes SEQ ID NO: 61.
55. A non-T effector cell of any one of embodiments 43-45 wherein the ligand binding domain binds PSMA, PSCA, mesothelin, CD20, WT1, or Her2.
56. A non-T effector cell of any one of embodiments 43-55 wherein the intracellular component includes an effector domain including one or more signaling and/or stimulatory domains selected from: 4-1BB, CARD11, CD3γ, CD3δ, CD3ε, CD3ζ, CD27, CD28, CD79A, CD79B, DAP10, FcRa, FcRβ, FcRy, Fyn, HVEM, ICOS, LAG3, LAT, Lck, LRP, NKG2D, NOTCH1, pTα, PTCH2, OX40, ROR2, Ryk, SLAMF1, Slp76, TCRα, TCRβ, TRIM, Wnt, and Zap70 signaling and/or stimulatory domains.
57.A non-T effector cell of any one of embodiments 43-56 wherein the intracellular component includes an effector domain including an intracellular signaling domain of CD3ζ, CD28ζ, or 4-1BB.
58. A non-T effector cell of any one of embodiments 43-57 wherein the intracellular component includes an effector domain including one or more costimulatory domains selected from: CD27, CD28, 4-1BB, OX40, CD30, CD40, LFA-1, CD2, CD7, LIGHT, NKG2C, or B7-H3 costimulatory domains.
59. A non-T effector cell of any one of embodiments 43-58 wherein the intracellular component includes an effector domain including an intracellular signaling domain including (i) all or a portion of the signaling domain of CD3ζ, (ii) all or a portion of the signaling domain of CD28, (iii) all or a portion of the signaling domain of 4-1BB, or (iv) all or a portion of the signaling domain of CD3ζ, CD28, and/or 4-1BB.
60. A non-T effector cell of any one of embodiments 43-59 wherein the intracellular component includes an effector domain including a variant of CD3ζ and/or a portion of the 4-1BB intracellular signaling domain.
61.A non-T effector cell of any one of embodiments 43-47, 50-52, or 55-60 genetically modified to express a spacer region.
62. A non-T effector cell of embodiment 61 wherein the spacer region includes a portion of a hinge region of a human antibody.
63. A non-T effector cell of embodiment 61 or 62 wherein the spacer region includes a hinge region and at least one other portion of an Fc domain of a human antibody selected from CH1, CH2, CH3 or combinations thereof.
64. A non-T effector cell of embodiment 61 or 62 wherein the spacer region includes a Fc domain and a human IgG4 heavy chain hinge.
65. A non-T effector cell of embodiment 61 wherein the spacer region is of a length selected from 12 amino acids or less, 119 amino acids or less, or 229 amino acids or less.
66. A non-T effector cell of embodiment 61 wherein the spacer region is SEQ ID NO:47, SEQ ID NO:52, or SEQ ID NO:61.
67. A non-T effector cell of any one of embodiments 43-66 wherein the non-T effector cell is also genetically modified to express a transmembrane domain.
68. A non-T effector cell of embodiment 67 wherein the transmembrane domain is a CD28 transmembrane domain or a CD4 transmembrane domain.
69. A non-T effector cell of any one of embodiments 43-68 wherein the extracellular component further includes a tag sequence.
70. A non-T effector cell of embodiment 69 wherein the tag sequence is EGFR lacking an intracellular signaling domain.
71. A non-T effector cell of any one of embodiments 43-70 wherein the non-T effector cell is a natural killer cell.
72.A composition including a genetically modified HSPC of any one of embodiments 1-4, 10, 11, or 14-42.
73. A composition including a non-T effector cell of any one of embodiments 5-9, 12, 13, or 43-71.
74. A composition of embodiment 72 or 73 formulated for infusion or injection.
75.A formulation including HSPC and a genetically modified HSPC of any one of embodiments 1-4, 10, 11, or 14-42.
76. A formulation including HSPC and a genetically modified non-T effector cell of any one of embodiments 5-9, 12, 13, or 43-71.
77. A formulation including a genetically modified HSPC of any one of embodiments 1-4, 10, 11, or 14-42, and a non-T effector cell of any one of embodiments 5-9, 12, 13, or 43-71.
78. A formulation of embodiment 77 further including HSPC.
79. A formulation of any one of embodiments 75-78 formulated for infusion or injection.
80. A kit including the compositions of any one of embodiments 72-74 wherein the kit includes instructions advising that the compositions or formulations can be administered to a subject without immunological matching.
81. A kit including the formulations of any one of embodiments 75-79 wherein the kit includes instructions advising that the compositions or formulations can be administered to a subject without immunological matching.
82. A kit including the compositions of any one of embodiments 72-74 and the formulations of any one of embodiments 75-79 wherein the kit includes instructions advising that the compositions or formulations can be administered to a subject without immunological matching.
83. A method of repopulating an immune system in a subject in need thereof and targeting unwanted cancer cells in the subject including administering a therapeutically-effective amount of genetically modified HSPC wherein the genetically modified HSPC express (i) an extracellular component including a ligand binding domain that binds a cellular marker that is preferentially expressed on the unwanted cancer cells, and (ii) an intracellular component including an effector domain thereby repopulating the subject's immune system and targeting the unwanted cancer cells.
84. A method of embodiment 83 further including administering genetically modified non-T effector cells wherein the genetically modified non-T effector cells express (i) an extracellular component including a ligand binding domain that binds a cellular marker that is preferentially expressed on the unwanted cancer cells, and (ii) an intracellular component including an effector domain.
85. A method of embodiment 83 or 84 further including administering HSPC.
86. A method of any one of embodiments 83-85 wherein immunological matching to the subject is not required before the administering.
87. A method of any one of embodiments 83-86 wherein the cellular marker is CD19, ROR1, PSMA, PSCA, mesothelin, CD20, WT1, or Her2.
88. A method of any one of embodiments 83-87 wherein repopulation is needed based on exposure to a myeloablative regimen for hematopoietic cell transplantation (HCT) and the unwanted cancer cells are acute lymphoblastic leukemia cells expressing CD19.
89. A method of any one of embodiments 83-88 wherein the subject is a relapsed pediatric acute lymphoblastic leukemia patient.
90. A method of targeting unwanted cancer cells in a subject including identifying at least one cellular marker preferentially expressed on a cancer cell from the subject; administering to the subject a therapeutically effective amount of genetically modified non-T effector cells wherein the genetically modified non-T effector cells express (i) an extracellular component including a ligand binding domain that binds the preferentially expressed cellular marker, and (ii) an intracellular component including an effector domain.
91. A method of embodiment 90 further including administering to the subject a genetically modified HSPC wherein the genetically modified HSPC express (i) an extracellular component including a ligand binding domain that binds the preferentially expressed cellular marker, and (ii) an intracellular component including an effector domain.
92. A method of targeting unwanted cancer cells in a subject including identifying at least one cellular marker preferentially expressed on a cancer cell from the subject; administering to the subject a genetically modified HSPC wherein the genetically modified HSPC express (i) an extracellular component including a ligand binding domain that binds the preferentially expressed cellular marker, and (ii) an intracellular component including an effector domain.
93. A method of any one of embodiments 90-92 further including treating immunodeficiency, pancytopenia, neutropenia, and/or leukopenia in the subject by administering a therapeutically effective amount of HSPC to the subject.
94.A method of embodiment 93 wherein the immunodeficiency, pancytopenia, neutropenia, and/or leukopenia is due to chemotherapy, radiation therapy, and/or a myeloablative regimen for HCT.
95. A method of any one of embodiments 90-94 wherein the cellular marker is CD19, ROR1, PSMA, PSCA, mesothelin, CD20, WT1, or Her2.
96. A method of any one of embodiments 90-95 wherein immunological matching to the subject is not required before the administering.
97. A method of any one of embodiments 90-96 wherein the unwanted cancer cells are acute lymphoblastic leukemia cells expressing CD19.
98. A method of any one of embodiments 90-97 wherein the subject is a relapsed pediatric acute lymphoblastic leukemia patient.
99. A method of repopulating an immune system in a subject in need thereof including administering a therapeutically effective amount of HSPC and/or genetically modified HSPC to the subject, thereby repopulating the immune system of the subject.
100. A method of embodiment 99 wherein the repopulating is needed based on one or more of immunodeficiency, pancytopenia, neutropenia, or leukopenia.
101. A method of embodiment 99 or 100 wherein the repopulating is needed based on one or more of viral infection, microbial infection, parasitic infections, renal disease, and/or renal failure.
102. A method of any one of embodiments 99-101 wherein the repopulating is needed based on exposure to a chemotherapy regimen, a myeloablative regimen for HCT, and/or acute ionizing radiation.
103. A method of any one of embodiments 99-102 wherein the repopulating is needed based on exposure to drugs that cause bone marrow suppression or hematopoietic deficiencies.
104. A method of any one of embodiments 99-103 wherein the repopulating is needed based on exposure to penicillin, gancyclovir, daunomycin, meprobamate, aminopyrine, dipyrone, phenytoin, carbamazepine, propylthiouracil, and/or methimazole.
105. A method of any one of embodiments 99-104 wherein the repopulating is needed based on exposure to dialysis.
106. A method of any one of embodiments 99-105 further including targeting unwanted cancer cells in the subject by administering genetically modified HSPC and/or genetically modified non-T effector cells wherein the genetically modified HSPC and/or genetically modified non-T effector cells express (i) an extracellular component including a ligand binding domain that binds to a cellular marker known to be preferentially expressed on cancer cells within the subject, and (ii) an intracellular component including an effector domain.
107. A method of embodiment 106 wherein the cancer cells are from an adrenal cancer, a bladder cancer, a blood cancer, a bone cancer, a brain cancer, a breast cancer, a carcinoma, a cervical cancer, a colon cancer, a colorectal cancer, a corpus uterine cancer, an ear, nose and throat (ENT) cancer, an endometrial cancer, an esophageal cancer, a gastrointestinal cancer, a head and neck cancer, a Hodgkin's disease, an intestinal cancer, a kidney cancer, a larynx cancer, a leukemia, a liver cancer, a lymph node cancer, a lymphoma, a lung cancer, a melanoma, a mesothelioma, a myeloma, a nasopharynx cancer, a neuroblastoma, a non-Hodgkin's lymphoma, an oral cancer, an ovarian cancer, a pancreatic cancer, a penile cancer, a pharynx cancer, a prostate cancer, a rectal cancer, a sarcoma, a seminoma, a skin cancer, a stomach cancer, a teratoma, a testicular cancer, a thyroid cancer, a uterine cancer, a vaginal cancer, a vascular tumor, and/or a metastasis thereof.
108. A method of embodiment 106 or 107 wherein the cellular marker(s) are selected from A33; BAGE; Bcl-2; β-catenin; B7H4; BTLA; CA125; CA19-9; CD5; CD19; CD20; CD21; CD22; CD33; CD37; CD44v6; CD45; CD123; CEA; CEACAM6; c-Met; CS-1; cyclin B1; DAGE; EBNA; EGFR; ephrinB2; ErbB2; ErbB3; ErbB4; EphA2; estrogen receptor; FAP; ferritin; α-fetoprotein (AFP); FLT1; FLT4; folate-binding protein; Frizzled; GAGE; G250; GD-2; GHRHR; GHR; GM2; gp75; gp100 (Pmel 17); gp130; HLA; HER-2/neu; HPV E6; HPV E7; hTERT; HVEM; IGF1R; IL6R; KDR; Ki-67; LIFRβ; LRP; LRP5; LTβR; mesothelin; OSMRβ; p53; PD1; PD-L1; PD-L2; PRAME; progesterone receptor; PSA; PSMA; PTCH1; MAGE; MART; mesothelin; MUC; MUC1; MUM-1-B; myc; NYESO-1; RANK; ras; Robo1; RORI; survivin; TCRα; TCRβ; tenascin; TGFBR1; TGFBR2;TLR7; TLR9; TNFR1; TNFR2; TNFRSF4; TWEAK-R; TSTA tyrosinase; VEGF; and WT1.
109. A method of any of embodiments 106-108 wherein the cancer is leukemia/lymphoma and the cellular marker(s) are one or more of CD19, CD20, CD22, ROR1, CD33, and WT-1; wherein the cancer is multiple myeloma and the cellular marker is BCMA; wherein the cancer is prostate cancer and the cellular marker(s) are one or more of PSMA, WT1, PSCA, and SV40 T; wherein the cancer is breast cancer and the cellular marker(s) are one or more of HER2, ERBB2, and ROR1; wherein the cancer is stem cell cancer and the cellular marker is CD133; wherein the cancer is ovarian cancer and the cellular marker(s) are one or more of L1-CAM, MUC-CD, folate receptor, Lewis Y, ROR1, mesothelin, and WT-1; wherein the cancer is mesothelioma and the cellular marker is mesothelin; wherein the cancer is renal cell carcinoma and the cellular marker is CAIX; wherein the cancer is melanoma and the cellular marker is GD2; wherein the cancer is pancreatic cancer and the cellular marker(s) are one or more of mesothelin, CEA, CD24, and ROR1; or wherein the cancer is lung cancer and the cellular marker is ROR1.
110. A method of any one of embodiments 106-109 wherein the cancer is acute lymphoblastic leukemia and the subject is a pediatric patient.
111. A method of any one of embodiments 106-110 wherein immunological matching to the subject is not required before the administering.
112. A method of targeting cells preferentially expressing CD19 for destruction including administering to a subject in need thereof a therapeutically effective amount of genetically modified HSPC and/or genetically modified non-T effector cells wherein the genetically modified cells express (i) an extracellular component including a CD19 ligand binding domain, and (ii) an intracellular component including an effector domain thereby targeting and destroying cells preferentially expressing CD19.
113. A method of embodiment 112 further including treating immunodeficiency, pancytopenia, neutropenia, and/or leukopenia in the subject by administering a therapeutically effective amount of HSPC to the subject.
114. A method of embodiment 113 wherein the immunodeficiency, pancytopenia, neutropenia, and/or leukopenia is due to chemotherapy, radiation therapy, and/or a myeloablative regimen for HCT.
115. A method of any one of embodiments 112-114 wherein immunological matching to the subject is not required before the administering.
116. A method of any one of embodiments 112-115 wherein the cells preferentially expressing CD19 are acute lymphoblastic leukemia cells.
117. A method of any one of embodiments 112-116 wherein the subject is a relapsed pediatric acute lymphoblastic leukemia patient.

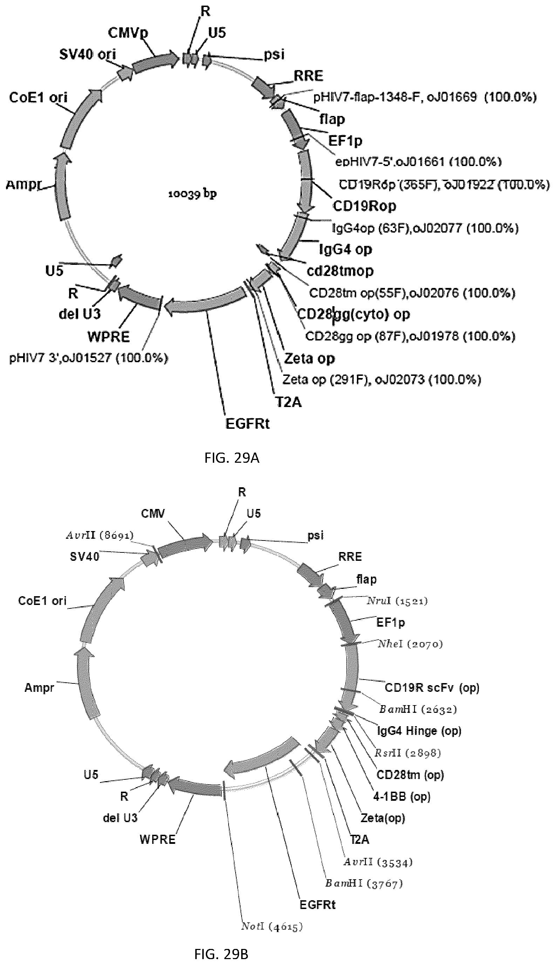

Example 1. Design and cGMP production of two third generation lentiviral vectors for the coordinate expression of the CD19-CAR and a huEGFRt selection/suicide construct have been created. For both a SIN vesicular stomatitis virus G (VSV-G) pseudotyped lentiviral vector under cGMP conditions that encodes for a CD19 specific CAR and huEGFRt, which is a truncated human EGFR protein that does not contain an intracellular signaling domain was developed. The CD19 specific scFvFc-CD3ζCD28 CAR and huEGFRt vector contains a hybrid 5'LTR in which the U3 region is replaced with the CMV promoter, and a 3' LTR in which the cis-acting regulatory sequences are completely removed from the U3 region. As a result, both the 5' and 3' LTRs are inactivated when the provirus is produced and integrated into the chromosome. The CD19 CAR includes the human GMCSFRα chain leader sequence, the VL and VH sequences derived from the CD19 specific murine IgG1mAb (FMC63), the Fc and hinge regions of human IgG4 heavy chain, the human CD28 transmembrane region, and the cytoplasmic domain of CD3ζ and CD28. This construct has been cloned into a modified pHIV7 in which the CMV promoter was swapped for the human EF-1 alpha promoter (Figure 29A). The vector allows approximately 1:1 expression of the CD19 CAR and huEGFRt through the use of a T2A element. The second, is the CD19-specific scFv-4-1BB/CD3ζ CAR fragment encodes an N-terminal leader peptide of the human GMCSF receptor alpha chain signal sequence to direct surface expression, CD19-specific scFv derived from the IgG1 murine monoclonal antibody (FMC63), human IgG4 hinge and human CD28 transmembrane region and 4-1BB costimulatory element with the cytoplasmic tail of human CD3ζ (Figure 29B). Again the vector allows approximately 1:1 expression of the CD19 CAR and huEGFRt through the use of a T2A element.

The expression of huEGFRt provides for a second cell surface marker that allows easy examination of transduction efficiency. Biotinylated Erbitux binds to the huEGFRt expressed on the cell surface and can be labeled with flurochrome for analysis with flow cytometry. Additionally it can be used as a suicide gene in the clinical setting with the treatment of Erbitux. A similar vector with eGFP in place of the CAR has also been generated.

Example 2. Notch-mediated ex vivo expansion of CB HSPC is a clinically validated cell therapy product that is well tolerated, can be given off the shelf without HLA matching, and provides transient myeloid engraftment in both the HCT and intensive chemotherapy setting. Off the shelf expanded units have been infused into >85 subjects and no serious adverse events have been noted except for one allergic reaction attributed to DMSO. Additionally, there has been no persistent engraftment beyond day 180 in the HCT setting and 14 days post infusion in the chemotherapy setting.

Methods. Umbilical cord blood/placental blood unit(s) were collected from human(s) at birth. The collected blood was mixed with an anti-coagulant to prevent clotting and stored. Prior to planned initiation of expansion cultures, tissue culture vessels were first coated overnight at 4°C or a minimum of 2 hours at 37°C with Delta1^{ext-IgG} at 2.5 µg/ml and RetroNectin^{®} (a recombinant human fibronectin fragment) (Clontech Laboratories, Inc., Madison, WI) at 5 µg/ml in phosphate buffered saline (PBS). The flasks were then washed with PBS and then blocked with PBS-2% Human Serum Albumin (HSA). The fresh cord blood unit is red cell lysed and processed to select for CD34⁺ cells using the autoMACS^{®} Cell Separation System (Miltenyi Biotec GmbH, Gladbach, Germany). After enrichment, the percentage of CD34⁺ cells in the sample is increased relative to the percentage of CD34⁺ cells in the sample prior to enrichment. The enriched CD34⁺ cell fraction was resuspended in final culture media, which consists of STEMSPAN^{™} Serum Free Expansion Medium (StemCell Technologies, Vancouver, British Columbia) supplemented with rhlL-3 (10 ng/ml), rhlL-6 (50 ng/ml), rhTPO (50 ng/ml), rhFIt-3L (50 ng/ml), rhSCF (50 ng/ml).

A SIN lentiviral vector that directs the co-expression of a CD19-specific scFvFc:CD28:ζ chimeric antigen receptor and a huEGFRt selection suicide construct was transduced into the Notch expanded CB stem cells on day 3 or 4 via centrifugation at 800 × g for 45 minutes at 32°C with lentiviral supernatant (MOI 3) and 4 µg/ml of protamine sulfate. Alternatively, the SIN lentiviral vector encoded for 4-1BB costimulation (see Brief Description of the Figures). Due to concerns of expression of the CAR on HSPC with potential signaling capacity, irradiated LCL was added on day 7 of culture at a 1:1 ratio to provide antigen stimulation.

At the end of the expansion culture, NK cells and neutrophils are still immature. In order to fully assess lytic capabilities, culture methods were devised to increase maturity. For the NK cells, the culture was replated in RPMI media supplemented with human serum, IL-2 at 50 U/mL and IL-15 at 500 ng/mL or RPMI media supplemented with human serum, L-glutamine, IL-2 at 50 U/mL and IL-15 at 500 ng/mL for an additional week of culture.

A NOD/SCID IL2R null (NOG) mouse model was used to assess engraftment of expanded CB cells. After undergoing sub-lethal irradiation, mice are able to reliably engraft expanded CB cells. In order to look at engraftment with transduced expanded CB cells, NOG mice were irradiated at a dose of 325cGy by linear accelerator and infused via tail vein injection with the progeny generated from 10,000 - 30,000 CD34⁺ CB cells cultured on Delta-1^{ext-IgG}.

Results. Transduction efficiency ranged from 10 to > 50% and there was generally equal transduction between CD34+ and CD34- cells. Copy number analysis demonstrated between 1-4 copies/cell as determined by validated real time, quantitative PCR analysis, which is in line with the FDA requirements for clinical gene therapy cell products.

CD34+ CB cells cultured on Notch ligand contain a variety of cell types, which can be identified based on immunophenotyping. Cultures transduced with the CD19 CAR lentivirus have been compared with an untransduced culture from the same cord blood unit and no significant differences have been detected in regards to the final immunophenotyping at the time of harvest, or the overall growth of the cells in culture including the CD34 fold expansion and the TNC fold expansion.

Expression of the transgene did not affect the final culture phenotype at 14 days and transgene expression is seen in all cell subsets and appears relatively stable over the culture period.

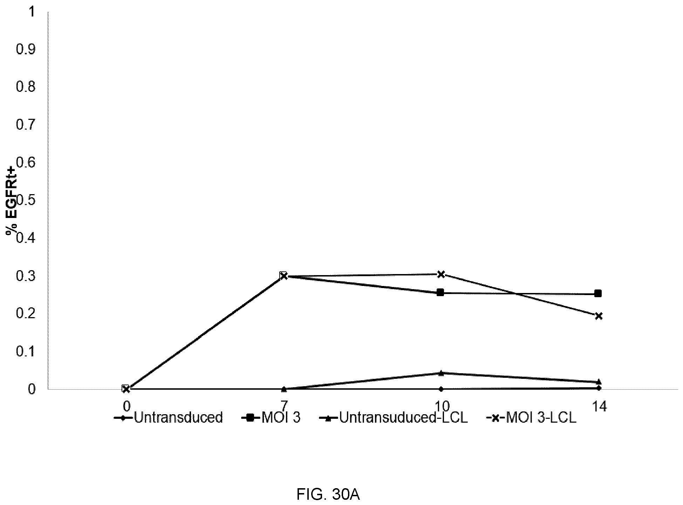

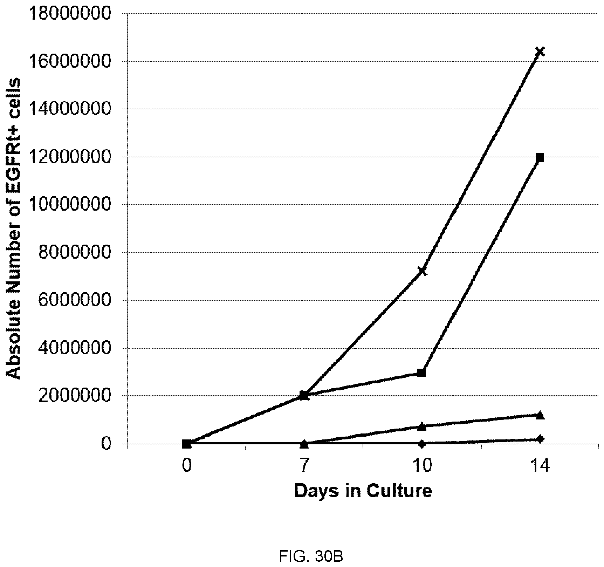

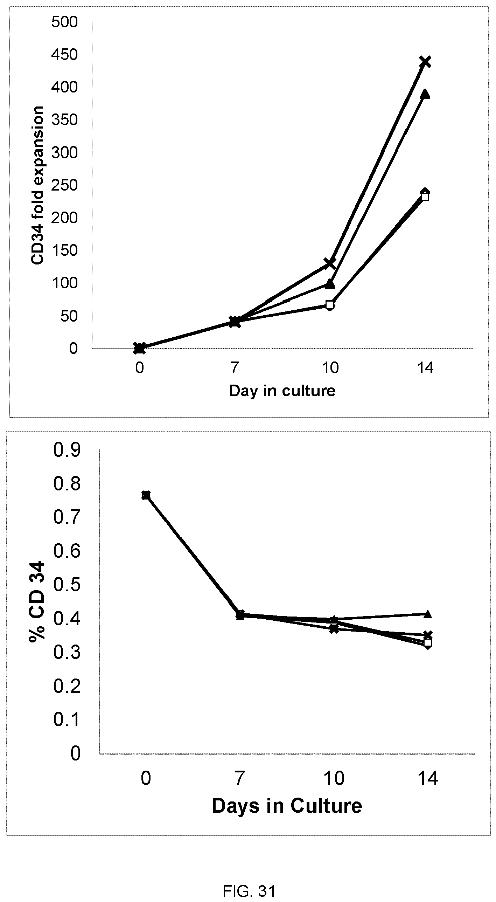

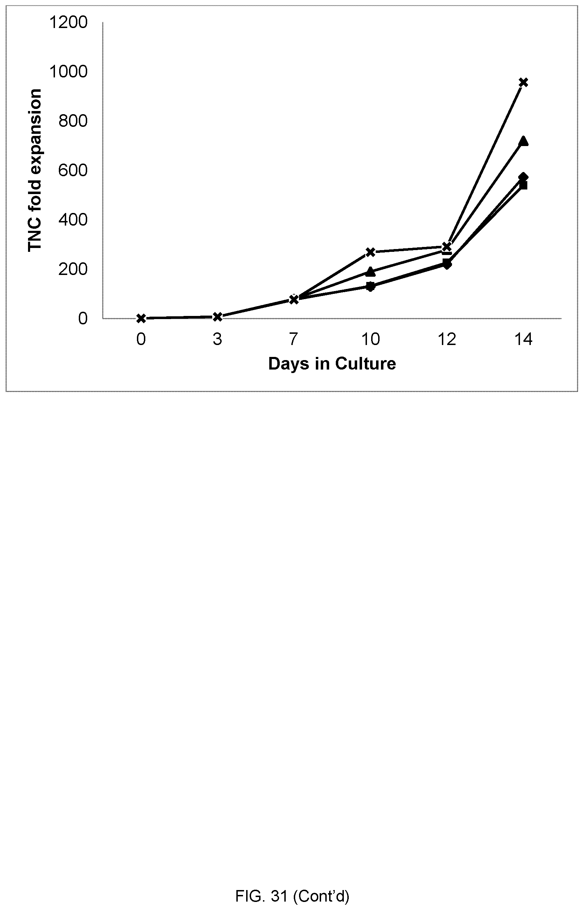

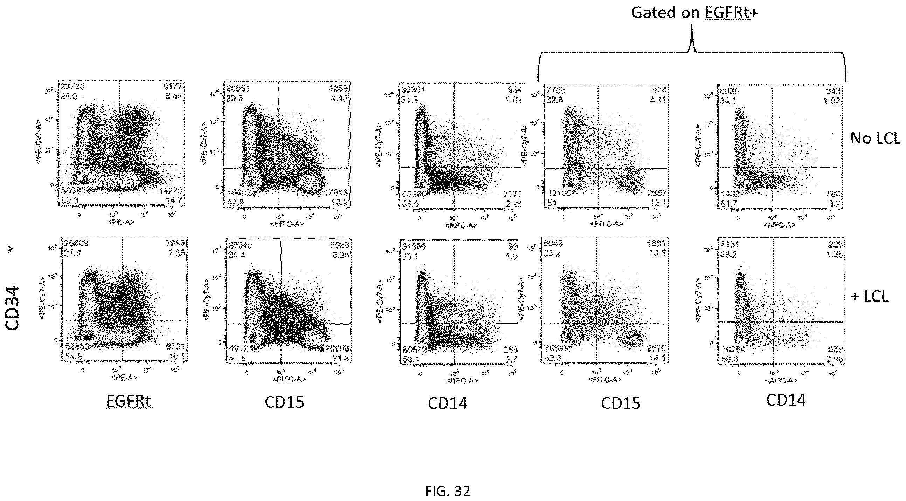

Additional experiments were carried out exposing the cell cultures to CD19+ LCL to determine if exposure to antigen causes untoward effects on the culture. Adding irradiated LCL to the culture on day 7 at a 1:1 ratio did not have untoward outcomes, and in fact enhanced the growth and viability in both the transduced and untransduced cultures. The LCL did not appear to increase the CAR+ population, suggesting that antigen does not enhance the proliferation of CAR expressing immature cells. Additionally, the transgene has been detected equivalently in all phenotypic cell subsets of the final product. For a graphical depiction of these results, see FIGs. 30A, 30B, 31, 32 and 33.

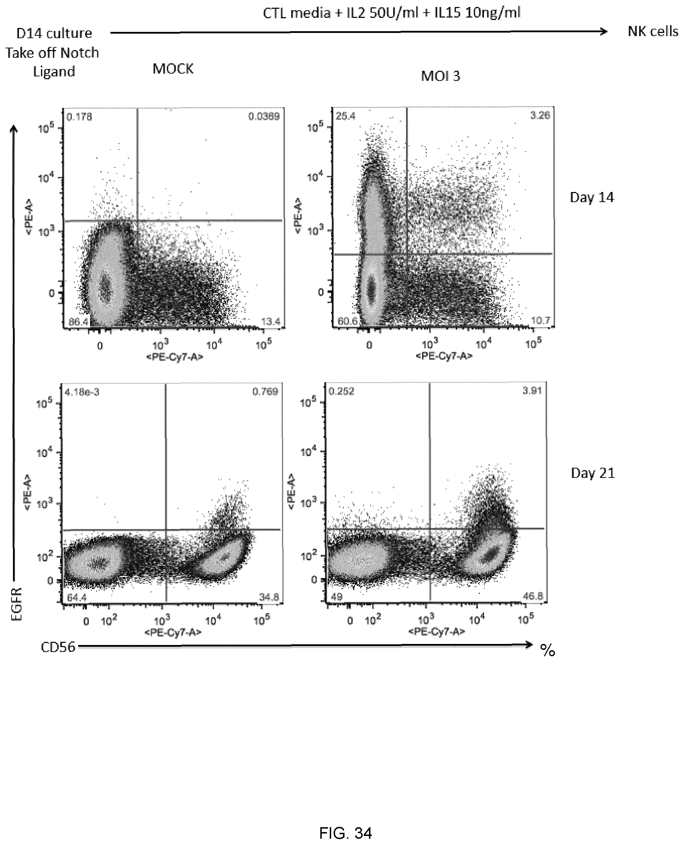

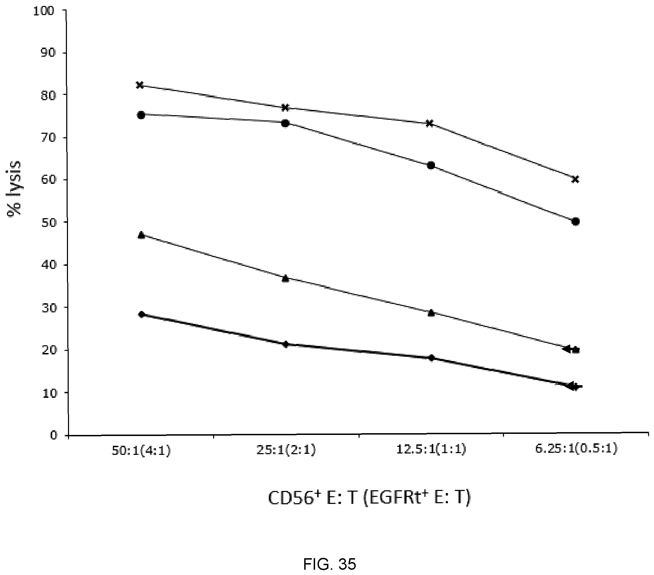

**The** transfer of effector function upon encountering CD19 through the expression of the CD19 CAR is important for the ultimate anti-cancer (e.g., anti-leukemic) activity of the modified CB HSPC cells. Differentiating culture conditions resulted in an increase of NK cells (FIG. 34). The CD56+ cell fraction was sorted and used in a CRA with target cells of K562 and LCL. As expected, both untransduced and transduced cells were able to kill K562, and although the LCL was also killed by both, the lysis of the LCL was significantly enhanced through the expression of the CAR. More particularly, the CD19-CAR expressing NK cells had enhanced cytotoxic activity compared with non-transduced NK cells (50 v 30%) whereas both killed K562 targets equally (75 v 80%). See FIG. 35.

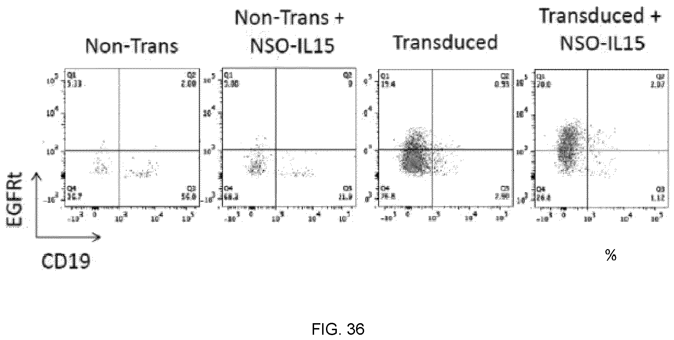

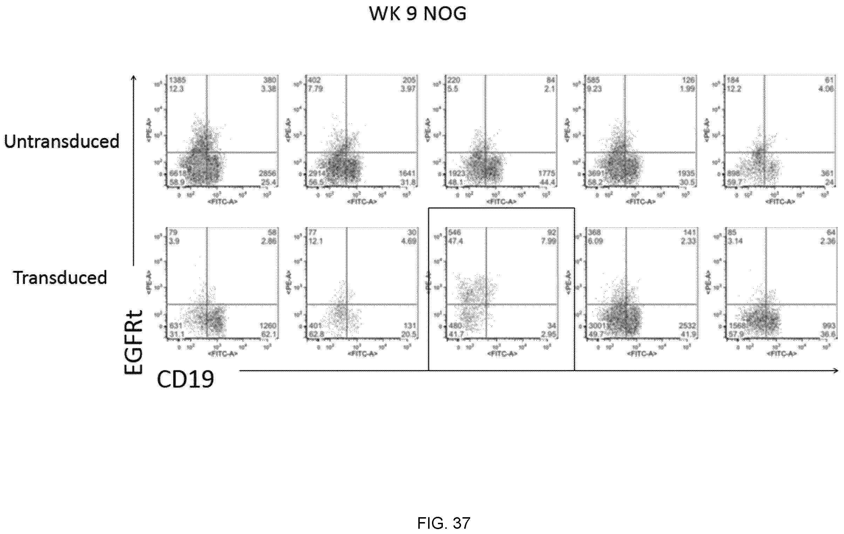

**The** NOG model when transplanted with expanded CB cells led to the development of a large population of CD19+ cells, beginning around week 4-5 post transplant. There was no effect on early engraftment of transduced cells, however there was a substantial reduction in CD19 engraftment in the mice transplanted with CD19 CAR expressing cells compared with untransduced cells, in which the CD19 population was >20% of the engrafted cells, indicating anti-CD19 activity. NK cell populations were increased using NSO-IL15 secreting cells, irradiated and injected subcutaneously three times per week starting at week 3 to provide enhanced effector function. This effect enhances the amount of CD56+ cells in vivo. See FIGs. 36 and 37.

**The** data show that transduction of expanded CB cells during culture in the presence of immobilized Delta1^{ext-IgG} to express a CD19 specific CAR does not have detectable effects of the quality or quantity of the expansion, nor on its repopulating abilities in the mouse model. These results are promising as a way to engineer a graft versus cancer (e.g., leukemia) effect into cord blood transplant. Furthermore, transduction of a CD19 CAR into universal donor expanded CB HSPC allows for infusion of an anti-CD19 cell product to be given immediately (e.g., immunological matching not required before administration) following identification of a subject with clinical need for therapy, for example one in relapse or with persistent MRD. Reliable transduction of CD34+ cord blood cells expanded on Notch ligand without affecting the overall culture nor in vivo engraftment capacity while at the same time engineering anti-CD19 activity has been demonstrated. Because expanded cord blood cells are already being used clinically as an off the shelf, non-HLA matched cellular therapy, the described Examples show additional use as an off the shelf cellular therapy, enabling patients to receive immunotherapy even if unable to obtain and engineer an autologous T cell product.

**As** indicated, the practice of the present disclosure can employ, unless otherwise indicated, conventional methods of virology, microbiology, molecular biology and recombinant DNA techniques within the ordinary skill of the art. Such techniques are explained fully in the literature; see, e.g., Sambrook, et al. Molecular Cloning: A Laboratory Manual (Current Edition); DNA Cloning: A Practical Approach, vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition); CRC Handbook of Parvoviruses, vol. I & II (P. Tijessen, ed.); Fundamental Virology, 2nd Edition, vol. I & II (B. N. Fields and D. M. Knipe, eds.) each of which is incorporated by reference herein for its teachings regarding the same.

**As** will be understood by one of ordinary skill in the art, each embodiment disclosed herein can comprise, consist essentially of or consist of its particular stated element, step, ingredient or component. "Includes" or "including" means "comprises, consists essentially of or consists of." The transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment. A material effect would result in (i) a statistically significant reduction in the effectiveness of a cell administration to create an anti-cancer effect in a subject and/or (ii) a statistically significant reduction in the effectiveness of a cell administration to re-populate a subject's immune system.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Particular embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to books, journal articles, treatises, patents, printed publications, etc. (collectively "references") throughout this specification. Each of the above-cited references are individually incorporated by reference herein for their cited teachings.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

Definitions and explanations used in the present disclosure are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3rd Edition or a dictionary known to those of ordinary skill in the art, such as the Oxford Dictionary of Biochemistry and Molecular Biology (Ed. Anthony Smith, Oxford University Press, Oxford, 2004).

The present invention will now be described by way of reference to the following clauses:
1. A CD34+ hematopoietic stem progenitor cell (HSPC) genetically modified to express (i) an extracellular component comprising a ligand binding domain that binds CD19; (ii) an intracellular component comprising an effector domain comprising a cytoplasmic domain of CD28 or 4-1BB; (iii) a spacer region comprising a hinge region of human IgG4; and (iv) a human CD4 or CD28 transmembrane domain.
2. A HSPC of clause 1 wherein the ligand binding domain is a single chain Fv fragment (scFv) comprising a CDRL1 sequence of RASQDISKYLN (SEQ ID NO. 108), a CDRL2 sequence of SRLHSGV (SEQ ID NO. 111), a CDRL3 sequence of GNTLPYTFG (SEQ ID NO. 104), a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).
3. A HSPC of clause 2 wherein the spacer region is 12 amino acids or less.
4. A HSPC of clause 2 wherein the spacer region comprises SEQ ID NO: 47.
5. A non-T effector cell genetically modified to express (i) an extracellular component comprising a ligand binding domain that binds CD19; (ii) an intracellular component comprising an effector domain comprising a cytoplasmic domain of CD28 or 4-1BB; (iii) a spacer region comprising a hinge region of human IgG4; and (iv) a human CD4 or CD28 transmembrane domain.
6. A non-T effector cell of clause 5 wherein the ligand binding domain is a scFv comprising a CDRL1 sequence of RASQDISKYLN (SEQ ID NO. 108), a CDRL2 sequence of SRLHSGV (SEQ ID NO. 111), a CDRL3 sequence of GNTLPYTFG (SEQ ID NO. 104), a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).
7. A non-T effector cell of clause 6 wherein the spacer region is 12 amino acids or less.
8. A non-T effector cell of clause 6 wherein the spacer region comprises SEQ ID NO: 47.
9. A non-T effector cell of clause 5 wherein the non-T effector cell is a natural killer cell.
10. A HSPC genetically modified to express a chimeric antigen receptor (CAR) of SEQ ID NO: 34, 53, 54, 55, 56, 57, or 58.
11. A HSPC of clause 10 wherein the HSPC is CD34+.
12. A non-T effector cell genetically modified to express a CAR of SEQ ID NO: 34, 53, 54, 55, 56, 57, or 58.
13. A non-T effector cell of clause 12 wherein the non-T effector cell is a natural killer cell.
14. A HSPC genetically modified to express (i) an extracellular component comprising a ligand binding domain that binds a cellular marker that is preferentially expressed on an unwanted cell; and (ii) an intracellular component comprising an effector domain.
15. A HSPC of clause 14 wherein the ligand binding domain is an antibody fragment.
16. A HSPC of clause 14 wherein the ligand binding domain is single chain variable fragment of an antibody.
17. A HSPC of clause 14 wherein the ligand binding domain binds CD19.
18. A HSPC of clause 17 wherein the ligand binding domain is a scFv comprising a CDRL1 sequence of RASQDISKYLN (SEQ ID NO. 108), a CDRL2 sequence of SRLHSGV (SEQ ID NO. 111), a CDRL3 sequence of GNTLPYTFG (SEQ ID NO. 104), a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).
19. A HSPC of clause 18 wherein the HSPC is also genetically modified to express a spacer region of 12 amino acids or less.
20. A HSPC of clause 19 wherein the spacer region comprises SEQ ID NO: 47.
21. A HSPC of clause 14 wherein the ligand binding domain binds ROR1.
22. A HSPC of clause 21 wherein the ligand binding domain is a scFv comprising a CDRL1 sequence of ASGFDFSAYYM (SEQ ID NO. 101), a CDRL2 sequence of TIYPSSG (SEQ ID NO. 112), a CDRL3 sequence of ADRATYFCA (SEQ ID NO. 100), a CDRH1 sequence of DTIDWY (SEQ ID NO. 102), a CDRH2 sequence of VQSDGSYTKRPGVPDR (SEQ ID NO. 113), and a CDRH3 sequence of YIGGYVFG (SEQ ID NO. 117).
23. A HSPC of clause 21 wherein the ligand binding domain is a scFv comprising a CDRL1 sequence of SGSDINDYPIS (SEQ ID NO. 109), a CDRL2 sequence of INSGGST (SEQ ID NO. 105), a CDRL3 sequence of YFCARGYS (SEQ ID NO. 116), a CDRH1 sequence of SNLAW (SEQ ID NO. 110, a CDRH2 sequence of RASNLASGVPSRFSGS (SEQ ID NO. 107), and a CDRH3 sequence of NVSYRTSF (SEQ ID NO. 106).
24. A HSPC of clause 23 wherein the HSPC is also genetically modified to express a spacer region of 229 amino acids or less.
25. A HSPC of clause 24 wherein the spacer region comprises SEQ ID NO: 61.
26.A HSPC of clause 14 wherein the ligand binding domain binds PSMA, PSCA, mesothelin, CD20, WT1, or Her2.
27. A HSPC of clause 14 wherein the intracellular component comprises an effector domain comprising one or more signaling and/or stimulatory domains selected from: 4-1BB, CARD11, CD3γ, CD3δ, CD3ε, CD3ζ, CD27, CD28, CD79A, CD79B, DAP10, FcRα, FcRβ, FcRγ, Fyn, HVEM, ICOS, LAG3, LAT, Lck, LRP, NKG2D, NOTCH1, pTα, PTCH2, OX40, ROR2, Ryk, SLAMF1, Slp76, TCRα, TCRβ, TRIM, Wnt, and Zap70 signaling and/or stimulatory domains.
28. A HSPC of clause 14 wherein the intracellular component comprises an effector domain comprising an intracellular signaling domain of CD3ζ, CD28ζ, or 4-1BB.
29. A HSPC of clause 14 wherein the intracellular component comprises an effector domain comprising one or more costimulatory domains selected from: CD27, CD28, 4-1BB, OX40, CD30, CD40, lymphocyte function-associated antigen-1 (LFA-1), CD2, CD7, LIGHT, NKG2C, or B7-H3 costimulatory domains.
30. A HSPC of clause 14 wherein the intracellular component comprises an effector domain comprising an intracellular signaling domain comprising (i) all or a portion of the signaling domain of CD3ζ, (ii) all or a portion of the signaling domain of CD28, (iii) all or a portion of the signaling domain of 4-1BB, or (iv) all or a portion of the signaling domain of CD3ζ, CD28, and/or 4-1BB.
31.A HSPC of clause 14 wherein the intracellular component comprises an effector domain comprising a variant of CD3ζ and/or a portion of the 4-1BB intracellular signaling domain.
32. A HSPC of clause 14 wherein the HSPC is also genetically modified to express a spacer region.
33. A HSPC of clause 32 wherein the spacer region comprises a portion of a hinge region of a human antibody.
34. A HSPC of clause 32 wherein the spacer region comprises a hinge region and at least one other portion of an Fc domain of a human antibody selected from CH1, CH2, CH3, or combinations thereof.
35. A HSPC of clause 32 wherein the spacer region comprises a Fc domain and a human IgG4 heavy chain hinge.
36. A HSPC of clause 32 wherein the spacer region is of a length selected from 12 amino acids or less, 119 amino acids or less, or 229 amino acids or less.
37.A HSPC of clause 32 wherein the spacer region is SEQ ID NO:47, SEQ ID NO:52, or SEQ ID NO:61.
38. A HSPC of clause 14 wherein the HSPC is also genetically modified to express a transmembrane domain.
39.A HSPC of clause 38 wherein the transmembrane domain is a CD28 transmembrane domain or a CD4 transmembrane domain.
40. A HSPC of clause 14 wherein the extracellular component further includes a tag sequence.
41. A HSPC of clause 40 wherein the tag sequence is EGFR lacking an intracellular signaling domain.
42. A HSPC of clause 14 wherein the HSPC is CD34+.
43. A non-T effector cell genetically modified to express (i) an extracellular component comprising a ligand binding domain that binds a cellular marker on an unwanted cell; and (ii) an intracellular component comprising an effector domain.
44. A non-T effector cell of clause 43 wherein the ligand binding domain is an antibody fragment.
45. A non-T effector cell of clause 43 wherein the ligand binding domain is single chain variable fragment of an antibody.
46. A non-T effector cell of clause 43 wherein the ligand binding domain binds CD19.
47. A non-T effector cell of clause 46 wherein the ligand binding domain is a scFv comprising a CDRL1 sequence of RASQDISKYLN (SEQ ID NO. 108), a CDRL2 sequence of SRLHSGV (SEQ ID NO. 111), a CDRL3 sequence of GNTLPYTFG (SEQ ID NO. 104), a CDRH1 sequence of DYGVS (SEQ ID NO. 103), a CDRH2 sequence of VTWGSETTYYNSALKS (SEQ ID NO. 114), and a CDRH3 sequence of YAMDYWG (SEQ ID NO. 115).
48. A non-T effector cell of clause 47 wherein the non-T effector cell is also genetically modified to express a spacer region of 12 amino acids or less.
49.A non-T effector cell of clause 48 wherein the spacer region comprises SEQ ID NO: 47.
50. A non-T effector cell of clause 43 wherein the ligand binding domain binds ROR1.
51. A non-T effector cell of clause 50 wherein the ligand binding domain is a scFv comprising a CDRL1 sequence of ASGFDFSAYYM (SEQ ID NO. 101), a CDRL2 sequence of TIYPSSG (SEQ ID NO. 112), a CDRL3 sequence of ADRATYFCA (SEQ ID NO. 100), a CDRH1 sequence of DTIDWY (SEQ ID NO. 102), a CDRH2 sequence of VQSDGSYTKRPGVPDR (SEQ ID NO. 113), and a CDRH3 sequence of YIGGYVFG (SEQ ID NO. 117).
52. A non-T effector cell of clause 50 wherein the ligand binding domain is a scFv comprising a CDRL1 sequence of SGSDINDYPIS (SEQ ID NO. 109), a CDRL2 sequence of INSGGST (SEQ ID NO. 105), a CDRL3 sequence of YFCARGYS (SEQ ID NO. 116), a CDRH1 sequence of SNLAW (SEQ ID NO. 110), a CDRH2 sequence of RASNLASGVPSRFSGS (SEQ ID NO. 107), and a CDRH3 sequence of NVSYRTSF (SEQ ID NO. 106).
53. A non-T effector cell of clause 52 wherein the non-T effector cell is also genetically modified to express a spacer region that is 229 amino acids or less.
54. A non-T effector cell of clause 53 wherein the spacer region comprises SEQ ID NO: 61.
55. A non-T effector cell of clause 43 wherein the ligand binding domain binds PSMA, PSCA, mesothelin, CD20, WT1, or Her2.
56. A non-T effector cell of clause 43 wherein the intracellular component comprises an effector domain comprising one or more signaling and/or stimulatory domains selected from: 4-1BB, CARD11, CD3γ, CD3δ, CD3ε, CD3ζ, CD27, CD28, CD79A, CD79B, DAP10, FcRα, FcRβ, FcRγ, Fyn, HVEM, ICOS, LAG3, LAT, Lck, LRP, NKG2D, NOTCH1, pTα, PTCH2, OX40, ROR2, Ryk, SLAMF1, Slp76, TCRα, TCRβ, TRIM, Wnt, and Zap70 signaling and/or stimulatory domains.
57. A non-T effector cell of clause 43 wherein the intracellular component comprises an effector domain comprising an intracellular signaling domain of CD3ζ, CD28ζ or 4-1BB.
58. A non-T effector cell of clause 43 wherein the intracellular component comprises an effector domain comprising one or more costimulatory domains selected from: CD27, CD28, 4-1BB, OX40, CD30, CD40, LFA-1, CD2, CD7, LIGHT, NKG2C, or B7-H3 costimulatory domains.
59. A non-T effector cell of clause 43 wherein the intracellular component comprises an effector domain comprising an intracellular signaling domain comprising (i) all or a portion of the signaling domain of CD3ζ, (ii) all or a portion of the signaling domain of CD28, (iii) all or a portion of the signaling domain of 4-1BB, or (iv) all or a portion of the signaling domain of CD3ζ, CD28, and/or 4-1BB.
60. A non-T effector cell of clause 43 wherein the intracellular component comprises an effector domain comprising a variant of CD3ζ and/or a portion of the 4-1BB intracellular signaling domain.
61. A non-T effector cell of clause 43 genetically modified to express a spacer region.
62. A non-T effector cell of clause 61 wherein the spacer region comprises a portion of a hinge region of a human antibody.
63. A non-T effector cell of clause 61 wherein the spacer region comprises a hinge region and at least one other portion of an Fc domain of a human antibody selected from CH1, CH2, CH3, or combinations thereof.
64. A non-T effector cell of clause 61 wherein the spacer region comprises a Fc domain and a human IgG4 heavy chain hinge.
65. A non-T effector cell of clause 61 wherein the spacer region is of a length selected from 12 amino acids or less, 119 amino acids or less, or 229 amino acids or less.
66.A non-T effector cell of clause 61 wherein the spacer region is SEQ ID NO:47, SEQ ID NO:52, or SEQ ID NO:61.
67. A non-T effector cell of clause 43 wherein the non-T effector cell is also genetically modified to express a transmembrane domain.
68. A non-T effector cell of clause 67 wherein the transmembrane domain is a CD28 transmembrane domain or a CD4 transmembrane domain.
69. A non-T effector cell of clause 43 wherein the extracellular component further includes a tag sequence.
70. A non-T effector cell of clause 69 wherein the tag sequence is EGFR lacking an intracellular signaling domain.
71. A non-T effector cell of clause 43 wherein the non-T effector cell is a natural killer cell.
72. A composition comprising a genetically modified HSPC of clause 1-4, 10, 11, or 14-42.
73. A composition comprising a non-T effector cell of clause 5-9, 12, 13, or 43-71.
74. A composition of clause 72 formulated for infusion or injection.
75. A formulation comprising HSPC and a genetically modified HSPC of clause 1-4, 10, 11, or 14-42.
76. A formulation comprising HSPC and a genetically modified non-T effector cell of clause 5-9, 12, 13, or 43-71.
77. A formulation comprising a genetically modified HSPC of clause 1-4, 10, 11, or 14-42 and a non-T effector cell of clause 5-9, 12, 13, or 43-71.
78. A formulation of clause 77 further comprising HSPC.
79. A formulation of clause 75 formulated for infusion or injection.
80. A kit comprising the compositions of clauses 72-74 wherein the kit comprises instructions advising that the compositions or formulations can be administered to a subject without immunological matching.
81. A kit comprising the formulations of clauses 75-79 wherein the kit comprises instructions advising that the compositions or formulations can be administered to a subject without immunological matching.
82. A kit comprising the compositions of clauses 72-74 and the formulations of clause 75-79 wherein the kit comprises instructions advising that the compositions or formulations can be administered to a subject without immunological matching.
83. A method of repopulating an immune system in a subject in need thereof and targeting unwanted cancer cells in the subject comprising administering a therapeutically-effective amount of genetically modified HSPC wherein the genetically modified HSPC express (i) an extracellular component comprising a ligand binding domain that binds a cellular marker that is preferentially expressed on the unwanted cancer cells, and (ii) an intracellular component comprising an effector domain thereby repopulating the subject's immune system and targeting the unwanted cancer cells.
84. A method of clause 83 further comprising administering genetically modified non-T effector cells wherein the genetically modified non-T effector cells express (i) an extracellular component comprising a ligand binding domain that binds a cellular marker that is preferentially expressed on the unwanted cancer cells, and (ii) an intracellular component comprising an effector domain.
85. A method of clause 83 or 84 further comprising administering HSPC.
86. A method of clause 85 wherein immunological matching to the subject is not required before the administering.
87. A method of clause 86 wherein the cellular marker is CD19, ROR1, PSMA, PSCA, mesothelin, CD20, WT1, or Her2.
88. A method of clause 85 wherein repopulation is needed based on exposure to a myeloablative regimen for hematopoietic cell transplantation (HCT) and the unwanted cancer cells are acute lymphoblastic leukemia cells expressing CD19.
89.A method of clause 85 wherein the subject is a relapsed pediatric acute lymphoblastic leukemia patient.
90. A method of targeting unwanted cancer cells in a subject comprising identifying at least one cellular marker preferentially expressed on a cancer cell from the subject; administering to the subject a therapeutically effective amount of genetically modified non-T effector cells, wherein the genetically modified non-T effector cells express (i) an extracellular component comprising a ligand binding domain that binds the preferentially expressed cellular marker and (ii) an intracellular component comprising an effector domain.
91. A method of clause 90 further comprising administering to the subject a genetically modified HSPC wherein the genetically modified HSPC express (i) an extracellular component comprising a ligand binding domain that binds the preferentially expressed cellular marker, and (ii) an intracellular component comprising an effector domain.
92. A method of targeting unwanted cancer cells in a subject comprising identifying at least one cellular marker preferentially expressed on a cancer cell from the subject; administering to the subject a genetically modified HSPC wherein the genetically modified HSPC express (i) an extracellular component comprising a ligand binding domain that binds the preferentially expressed cellular marker and (ii) an intracellular component comprising an effector domain.
93.A method of clauses 90-92 further comprising treating immunodeficiency, pancytopenia, neutropenia, and/or leukopenia in the subject by administering a therapeutically effective amount of HSPC to the subject.
94. A method of clause 93 wherein the immunodeficiency, pancytopenia, neutropenia, and/or leukopenia is due to chemotherapy, radiation therapy, and/or a myeloablative regimen for HCT.
95. A method of clause 93 wherein the cellular marker is CD19, ROR1, PSMA, PSCA, mesothelin, CD20, WT1, or Her2.
96. A method of clause 93 wherein immunological matching to the subject is not required before the administering.
97. A method of clause 93 wherein the unwanted cancer cells are acute lymphoblastic leukemia cells expressing CD19.
98. A method of clause 93 wherein the subject is a relapsed pediatric acute lymphoblastic leukemia patient.
99.A method of repopulating an immune system in a subject in need thereof comprising administering a therapeutically effective amount of HSPC and/or genetically modified HSPC to the subject, thereby repopulating the immune system of the subject.
100. A method of clause 99 wherein the repopulating is needed based on one or more of immunodeficiency, pancytopenia, neutropenia, or leukopenia.
101. A method of clause 99 wherein the repopulating is needed based on one or more of viral infection, microbial infection, parasitic infections, renal disease, and/or renal failure.
102. A method of clause 99 wherein the repopulating is needed based on exposure to a chemotherapy regimen, a myeloablative regimen for HCT, and/or acute ionizing radiation.
103. A method of clause 99 wherein the repopulating is needed based on exposure to drugs that cause bone marrow suppression or hematopoietic deficiencies.
104. A method of clause 99 wherein the repopulating is needed based on exposure to penicillin, gancyclovir, daunomycin, meprobamate, aminopyrine, dipyrone, phenytoin, carbamazepine, propylthiouracil, and/or methimazole.
105. A method of clause 99 wherein the repopulating is needed based on exposure to dialysis.
106. A method of clause 99 further comprising targeting unwanted cancer cells in the subject by administering genetically modified HSPC and/or genetically modified non-T effector cells, wherein the genetically modified HSPC and/or genetically modified non-T effector cells express (i) an extracellular component comprising a ligand binding domain that binds to a cellular marker known to be preferentially expressed on cancer cells within the subject and (ii) an intracellular component comprising an effector domain.
107. A method of clause 106 wherein the cancer cells are from an adrenal cancer, a bladder cancer, a blood cancer, a bone cancer, a brain cancer, a breast cancer, a carcinoma, a cervical cancer, a colon cancer, a colorectal cancer, a corpus uterine cancer, an ear, nose and throat (ENT) cancer, an endometrial cancer, an esophageal cancer, a gastrointestinal cancer, a head and neck cancer, a Hodgkin's disease, an intestinal cancer, a kidney cancer, a larynx cancer, a leukemia, a liver cancer, a lymph node cancer, a lymphoma, a lung cancer, a melanoma, a mesothelioma, a myeloma, a nasopharynx cancer, a neuroblastoma, a non-Hodgkin's lymphoma, an oral cancer, an ovarian cancer, a pancreatic cancer, a penile cancer, a pharynx cancer, a prostate cancer, a rectal cancer, a sarcoma, a seminoma, a skin cancer, a stomach cancer, a teratoma, a testicular cancer, a thyroid cancer, a uterine cancer, a vaginal cancer, a vascular tumor, and/or a metastasis thereof.
108. A method of clause 106 wherein the cellular marker(s) are selected from A33; BAGE; Bcl-2; β-catenin; B7H4; BTLA; CA125; CA19-9; CD5; CD19; CD20; CD21; CD22; CD33; CD37; CD44v6; CD45; CD123; CEA; CEACAM6; c-Met; CS-1; cyclin B1; DAGE; EBNA; EGFR; ephrinB2; ErbB2; ErbB3; ErbB4; EphA2; estrogen receptor; FAP; ferritin; α-fetoprotein (AFP); FLT1; FLT4; folate-binding protein; Frizzled; GAGE; G250; GD-2; GHRHR; GHR; GM2; gp75; gp100 (Pmel 17); gp130; HLA; HER-2/neu; HPV E6; HPV E7; hTERT; HVEM; IGF1R; IL6R; KDR; Ki-67; LIFRβ; LRP; LRP5; LTβR; mesothelin; OSMRβ; p53; PD1; PD-L1; PD-L2; PRAME; progesterone receptor; PSA; PSMA; PTCH1; MAGE; MART; mesothelin; MUC; MUC1; MUM-1-B; myc; NYESO-1; RANK; ras; Robo1; RORI; survivin; TCRα; TCRβ; tenascin; TGFBR1; TGFBR2;TLR7; TLR9; TNFR1; TNFR2; TNFRSF4; TWEAK-R; TSTA tyrosinase; VEGF; and WT1.
109. A method of clause 106 wherein the cancer is leukemia/lymphoma and the cellular marker(s) are one or more of CD19, CD20, CD22, ROR1, CD33, and WT-1; wherein the cancer is multiple myeloma and the cellular marker is BCMA; wherein the cancer is prostate cancer and the cellular marker(s) are one or more of PSMA, WT1, PSCA, and SV40 T; wherein the cancer is breast cancer and the cellular marker(s) are one or more of HER2, ERBB2, and ROR1; wherein the cancer is stem cell cancer and the cellular marker is CD133; wherein the cancer is ovarian cancer and the cellular marker(s) are one or more of L1-CAM, MUC-CD, folate receptor, Lewis Y, ROR1, mesothelin, and WT-1; wherein the cancer is mesothelioma and the cellular marker is mesothelin; wherein the cancer is renal cell carcinoma and the cellular marker is CAIX; wherein the cancer is melanoma and the cellular marker is GD2; wherein the cancer is pancreatic cancer and the cellular marker(s) are one or more of mesothelin, CEA, CD24, and ROR1; or wherein the cancer is lung cancer and the cellular marker is ROR1.
110. A method of clause 106 wherein the cancer is acute lymphoblastic leukemia and the subject is a pediatric patient.
111. A method of clause 106 wherein immunological matching to the subject is not required before the administering.
112. A composition of clause 73 formulated for infusion or injection.
113. A formulation of clause 76 formulated for infusion or injection.
114. A formulation of clause 77 formulated for infusion or injection.
115. A formulation of clause 78 formulated for infusion or injection.
116. A method of targeting cells preferentially expressing CD19 for destruction comprising administering to a subject in need thereof a therapeutically effective amount of genetically modified HSPC and/or genetically modified non-T effector cells wherein the genetically modified cells express (i) an extracellular component including a CD19 ligand binding domain, and (ii) an intracellular component including an effector domain thereby targeting and destroying cells preferentially expressing CD19.
117. A method of clause 116 further including treating immunodeficiency, pancytopenia, neutropenia, and/or leukopenia in the subject by administering a therapeutically effective amount of HSPC to the subject.
118. A method of clause 117 wherein the immunodeficiency, pancytopenia, neutropenia, and/or leukopenia is due to chemotherapy, radiation therapy, and/or a myeloablative regimen for HCT.
119. A method of clause 116 or 117 wherein immunological matching to the subject is not required before the administering.
120. A method of clause 116 wherein the cells preferentially expressing CD19 are acute lymphoblastic leukemia cells.
121. A method of clause 116 or 117 wherein the subject is a relapsed pediatric acute lymphoblastic leukemia patient.

## Claims

1. Notch agonist-expanded genetically modified hematopoietic stem and progenitor cells (HSPCs) and/orgenetically modified natural killer cells differentiated from the Notch agonist expanded genetically modified HSPCs,
wherein the genetically modified cells express molecules comprising an extracellular component linked to an intracellular component, and
wherein the extracellular component comprises a ligand binding domain that binds CD19, ROR1, PSMA, PSCA, mesothelin, CD20, WT1 or Her2; and
wherein the intracellular component comprises an effector domain comprising (a) all or a portion of the signaling domain of CD3ζ, (b) all or a portion of the signaling domain of CD28, and/or (c) all or a portion of the signaling domain of 4-1 BB,
for use in a method of repopulating an immune system in a human subject in need thereof and/or targeting unwanted cancer cells in the human subject comprising administering a therapeutically effective amount of the genetically modified cells,
wherein the method further comprises administering a therapeutically effective amount of Notch agonist-expanded non-genetically modified HSPCs, and
wherein the administering is without immunologically matching the human subject to the genetically modified cells and the non-genetically modified cells.

2. A CD34+ hematopoietic stem progenitor cell (HSPC) genetically modified to express (i) an extracellular component comprising a ligand binding domain that binds CD19; (ii) an intracellular component comprising an effector domain comprising a cytoplasmic domain of CD28 or 4-1BB; (iii) a spacer region comprising a hinge region of human IgG4; and (iv) a human CD4 or CD28 transmembrane domain.

3. A non-T effector cell genetically modified to express (i) an extracellular component comprising a ligand binding domain that binds CD19; (ii) an intracellular component comprising an effector domain comprising a cytoplasmic domain of CD28 or 4-1BB; (iii) a spacer region comprising a hinge region of human IgG4; and (iv) a human CD4 or CD28 transmembrane domain.

4. A HSPC genetically modified to express a chimeric antigen receptor (CAR) of SEQ ID NO: 34, 53, 54, 55, 56, 57, or 58.

5. A non-T effector cell genetically modified to express a CAR of SEQ ID NO: 34, 53, 54, 55, 56, 57, or 58.

6. A HSPC genetically modified to express (i) an extracellular component comprising a ligand binding domain that binds a cellular marker that is preferentially expressed on an unwanted cell; and (ii) an intracellular component comprising an effector domain.

7. A non-T effector cell genetically modified to express (i) an extracellular component comprising a ligand binding domain that binds a cellular marker on an unwanted cell; and (ii) an intracellular component comprising an effector domain.

8. A method of repopulating an immune system in a subject in need thereof and targeting unwanted cancer cells in the subject comprising administering a therapeutically-effective amount of genetically modified HSPC wherein the genetically modified HSPC express (i) an extracellular component comprising a ligand binding domain that binds a cellular marker that is preferentially expressed on the unwanted cancer cells, and (ii) an intracellular component comprising an effector domain thereby repopulating the subject's immune system and targeting the unwanted cancer cells.

9. A method of targeting unwanted cancer cells in a subject comprising identifying at least one cellular marker preferentially expressed on a cancer cell from the subject; administering to the subject a therapeutically effective amount of genetically modified non-T effector cells, wherein the genetically modified non-T effector cells express (i) an extracellular component comprising a ligand binding domain that binds the preferentially expressed cellular marker and (ii) an intracellular component comprising an effector domain.

10. A method of targeting unwanted cancer cells in a subject comprising identifying at least one cellular marker preferentially expressed on a cancer cell from the subject; administering to the subject a genetically modified HSPC wherein the genetically modified HSPC express (i) an extracellular component comprising a ligand binding domain that binds the preferentially expressed cellular marker and (ii) an intracellular component comprising an effector domain.

11. A method of repopulating an immune system in a subject in need thereof comprising administering a therapeutically effective amount of HSPC and/or genetically modified HSPC to the subject, thereby repopulating the immune system of the subject.

12. A method of targeting cells preferentially expressing CD19 for destruction comprising administering to a subject in need thereof a therapeutically effective amount of genetically modified HSPC and/or genetically modified non-T effector cells wherein the genetically modified cells express (i) an extracellular component including a CD19 ligand binding domain, and (ii) an intracellular component including an effector domain thereby targeting and destroying cells preferentially expressing CD19.
